# EUROPEAN PATENT SPECIFICATION

(11) **EP 2 492 355 B1**
(45) Date of publication and mention of the grant of the patent: **08.04.2015**
(21) Application number: 11004689.3
(22) Date of filing: 28.11.2008
(51) Int. Cl.: C12Q 1/68, G01N 33/50, G01N 33/74, G01N 33/574

(54) **Tissue protective erythropoietin receptor (nepor) and methods of use**
Erythropoietin-Rezeptor zum Gewebeschutz und Verwendungsverfahren
Récepteur d'érythropoietine protecteur de tissu (nepor) et procédés d'utilisation

(30) Priority: 29.11.2007 US 991042 P
(43) Date of publication of application: 29.08.2012
(62) Divisional of application: 08854346.7
(73) Proprietor: Molecular Health GmbH, 69115 Heidelberg (DE)
(72) Inventor: Jackson, David B., 69117 Heidelberg (DE); Stein, Martin, 68307 Mannheim (DE); Voss, Hartmut, 69198 Schriesheim (DE); Brock, Stephan, 69469 Weinheim (DE)
(74) Representative: Lahrtz, Fritz

(56) References cited:
- WO-A-2004/024773
- WO-A-2004/096148
- WO-A2-2004/080418
- WO-A2-2006/034456
- XIA GUANGBIN ET AL: "Up-regulation of EphB4 in mesothelioma and its biological significance.", CLINICAL CANCER RESEARCH : AN OFFICIAL JOURNAL OF THE AMERICAN ASSOCIATION FOR CANCER RESEARCH 15 JUN 2005 LNKD- PUBMED:15958611, vol. 11, no. 12, 15 June 2005 (2005-06-15) , pages 4305-4315, XP55033638, ISSN: 1078-0432
- OGAWA K ET AL: "THE EPHRIN-A1 LIGAND AND ITS RECEPTOR, EPHA2, ARE EXPRESSED DURING TUMOR NEOVASCULARIZATION", ONCOGENE, NATURE PUBLISHING GROUP, GB, vol. 19, no. 52, 1 January 2000 (2000-01-01), pages 6043-6052, XP001024674, ISSN: 0950-9232, DOI: 10.1038/SJ.ONC.1204004
- WOHLFAHRT JAN G ET AL: "Ephrin-A1 suppresses Th2 cell activation and provides a regulatory link to lung epithelial cells.", JOURNAL OF IMMUNOLOGY (BALTIMORE, MD. : 1950) 15 JAN 2004 LNKD- PUBMED:14707054, vol. 172, no. 2, 15 January 2004 (2004-01-15), pages 843-850, XP055033644, ISSN: 0022-1767
- BRINES MICHAEL ET AL: "Erythropoietin mediates tissue protection through an erythropoietin and common beta-subunit heteroreceptor", PROCEEDINGS OF THE NATIONAL ACADEMY OF SCIENCES OF THE UNITED STATES OF AMERICA, vol. 101, no. 41, 12 October 2004 (2004-10-12), pages 14907-14912, XP002517811, ISSN: 0027-8424
- JUBINSKY PAUL T ET AL: "The beta chain of the interleukin-3 receptor functionally associates with the erythropoietin receptor", BLOOD, vol. 90, no. 5, 1997, pages 1867-1873, XP002517812, ISSN: 0006-4971
- KONSTANTINOPOULOS ET AL: "Selective modulation of the erythropoietic and tissue-protective effects of erythropoietin: Time to reach the full therapeutic potential of erythropoietin", BBA - REVIEWS ON CANCER, ELSEVIER SCIENCE BV, AMSTERDAM, NL, vol. 1776, no. 1, 29 August 2007 (2007-08-29), pages 1-9, XP022219014, ISSN: 0304-419X
- SUENOBU SOUICHI ET AL: "A role of EphB4 receptor and its ligand, ephrin-B2, in erythropoiesis", BIOCHEMICAL AND BIOPHYSICAL RESEARCH COMMUNICATIONS, vol. 293, no. 3, 10 May 2002 (2002-05-10), pages 1124-1131, XP002517813, ISSN: 0006-291X
- HEROULT M ET AL: "Eph receptor and ephrin ligand-mediated interactions during angiogenesis and tumor progression", EXPERIMENTAL CELL RESEARCH, ACADEMIC PRESS, US, vol. 312, no. 5, 10 March 2006 (2006-03-10), pages 642-650, XP024944931, ISSN: 0014-4827 [retrieved on 2006-03-10]
- FOUBERT PHILIPPE ET AL: "PSGL-1-mediated activation of EphB4 increases the proangiogenic potential of endothelial progenitor cells", JOURNAL OF CLINICAL INVESTIGATION, vol. 117, no. 6, June 2007 (2007-06), pages 1527-1537, XP002517814, ISSN: 0021-9738
- ACS GEZA ET AL: "Prognostic significance of erythropoietin expression in human endometrial carcinoma", CANCER, vol. 100, no. 11, 1 June 2004 (2004-06-01) , pages 2376-2386, XP002517815, ISSN: 0008-543X

## Description

### Technical Field

The present disclosure provides a novel tissue protective erythropoietin (EPO) binding receptor protein complex, here termed NEPOR. NEPOR is proposed to mediate EPO's tissue protective properties. Thus, presence of NEPOR on a tumour allows EPO to impinge on the survival of associated cells thereby enhancing tumour progression and negatively effecting patient survival. In this circumstance, presence of NEPOR represents a prognostic biomarker for poorer patient outcome, while also representing a unique clinically tractable anti-cancer target protein, Likewise, NEPOR mediates EPO's neuroprotective activity and thus represents a unique target for treatment of diseases associated with neuronal insults, such as stroke.

In the first instance, the present disclosure provides a method for assessing a tumour for expression of this protein. The present disclosure further provides a method to stratify patients having a tumour as suitable (*i.e.* NEPOR not present) or non-suitable (*i.e*., NEPOR present) for EPO treatment. The method disclosed comprises: (a) isolating a tissue sample from an individual who is receiving or is a candidate for receiving erythropoietin, (b) determining the level of expression of the NEPOR gene(s) (mRNA) and/or the presence of the NEPOR gene product (protein) from the isolated tissue, and (c) correlating the presence of an NEPOR gene expression product or the presence of NEPOR protein to a physiological response to the treatment with erythropoietin. In a second instance, the present disclosure provides a method for treating patients with NEPOR positive tumors. Furthermore, the present disclosure provides a method for treating stroke. Finally, the present disclosure provides a method for screening for NEPOR directed therapeutics (both antagonistic therapeutics for cancer, and agonistic therapeutics for treatment of hypoxia associated tissue damage, such as stroke).

### Background

Approved by the FDA in 1993 for treatment of anemia, Erythropoietin (EPO) is a 193 amino acid glycoprotein hormone, produced by the kidneys to regulate red blood cell (RBC) production; a process commonly termed erythropoiesis. EPO was originally identified as a cytokine that promotes erythrocyte progenitor survival and differentiation, but has also been shown to possess neuroprotective functions, particularly in response to ischemic injury in the central nervous system, (CNS). Clinical use of EPO has been prevalent in the treatment of anemic cancer patients, while ongoing studies are exploring EPO's potential in the treatment of neurological diseases (*e.g.* stroke). Notwithstanding, recent clinical studies in cancer patients have begun to uncover highly worrying adverse events, suggesting that administration of recombinant human EPO (rHuEPO) can adversely effect overall patient survival. An urgent need thus exists in medical oncology to better understand and predict the prevalence or susceptibility to this effect, so that administration of rHuEPO can be contra-indicated, continued or stopped.

### EPO: Biological function

Erythropoietin (EPO) is a 193 amino acid type I cytokine, produced by cells of the renal cortex to regulate red blood cell (RBC) production in a process termed erythropoiesis. Erythropoiesis is multistage in nature, involving the differentiation of pluripotent hematopoietic stem cells through the lineage-committed burst-forming unit-erythroid (BFU-E) and colony-forming unit-erythroid (CFU-E) progenitor cells, which give rise to a series of early and late erythroblasts, eventually leading to the formation of reticulocytes and mature erythrocytes. During this process, the sequential formation of pro-erythroblasts, basophilic, polychromatophilic, and orthochromatic erythroblasts is positively regulated by EPO. EPO induces multiple positive effects on early erythroblasts, including increased proliferation, progression through maturation, and protection from programmed cell death.

In terms of molecular mechanism, EPO binds to two identical receptors (EpoR), an event which activates several intracellular signaling pathways. These include Janus kinase 2-signal transducer and activator of transcription 5 (JAK2-STAT5), phosphatidylinositol 3-kinase (PI3K), protein kinase C (PKC), and Ras-Raf-MEK (mitogen-activated or extracellular signal-regulated protein kinase kinase)-ERK (extracellular signal-regulated protein kinase). The JAK2-STAT5 and RAS-RAF-MEK-ERK pathways are thought to be associated with Epo's mitogenic action, while the PI3K pathway, acting through Akt (PI3K-Akt), is viewed as a mediator of EPO's anti-apoptotic activities.

### EPO: Clinical use

Anemia (AmE) or anæmia/anaemia (BrE), from the Greek ('vατµíα)(an-haîma) meaning "without blood", is a deficiency of red blood cells (RBCs) and/or hemoglobin. The condition is commonly observed in patients with chronic diseases, and is particularly common in cancer where about 50% of patients are anaemic at presentation and some 70-90% developing the condition during the course of treatment (typically termed chemotherapy induced anemia (CIA)). In a recent review of the European Cancer Anemia Survey (ECAS), Ludwig et al. cited a 50% baseline anemia rate (hemoglobin [Hb] < 12 g/dL) among 3010 patients with hematological malignancies and a 41 % baseline anemia rate among 11,453 patients with solid tumours (*Blood*, 2002;100:234a-235a. Abstract 884). Further longitudinal analysis revealed that 72% of 2780 patients with haematologic malignancies and 66% of 10,067 patients with solid tumours succumbed to CIA. Other published studies have reported varying high rates in patients at different phases and with different types of treatment (Table 1). Notwithstanding, all studies demonstrate the extremely high prevalence of anemia amongst cancer patients.

**Table 1. Prevalence of Anemia in Cancer Patients Undergoing Treatmen**

| **Type of Cancer** | **Prevalence of Anemia (Hb < 12 g/dL)** |
|---|---|
| Cervical cancer^{[3]} | 82% |
| Solid tumors^{[1]} | 66% |
| Colorectal cancer^{[3]} | 67% |
| Lung cancer^{[3]} | 63% |
| Haematological malignancies | 72% |

A number of factors contribute to the high incidence of anemia among cancer patients, including not only chemotherapy and radiation-induced myelosuppression, but also cytokine-mediated anemia of chronic disease, bleeding, marrow infiltration by tumour, hemolysis, and nutritional deficiencies. Whatever the source, anemia results in a reduced ability of blood to transfer oxygen to the tissues, leading to tissue hypoxia and an associated range of clinical consequences, affecting all realms of patient health: physiologic status, psychosocial well-being and quality of life. Not surprising, anemia can negatively affect a patient's response to cancer therapy, a fact which highlights the important supportive role of rHuEPO in restoring normal RBC counts.

### EPO: Clinical Safety signals

ESA's were for many years considered to be extremely safe in their labelled indications of chronic kidney disease and chemotherapy-induced anemia. The first hints of safety issues came in 2003 when results from a pair of studies examining EPO's potentiation of radiation and chemotherapy prompted an FDA meeting in May 2004. This first study (the ENHANCE study: Lancet 2003;362:1255-1260) suggested the relative risk of progression-free survival was worse for patients who received radiotherapy plus NeoRecormon epoetin beta from Roche than for patients receiving placebo plus radiotherapy. A randomized, double-blind, multi-institutional trial that included a study population of 351 patients who were receiving radiotherapy was performed. The patients were treated 3 times per week with either placebo or EPO in the form of epoetin beta starting 10 to 14 days before and continuing through radiation therapy. Although haemoglobin levels increased in 82% of patients receiving EPO, compared with 15% in patients receiving placebo, the rate of loco-regional progression-free survival was significantly lower. In addition, the EPO group had a higher relative risk for loco-regional progression and death.

In the second trial involving 939 breast cancer patients receiving chemotherapy (the BEST study: J. Clin. Oncol. 2005;23:5960-5972; see table 2), those given Eprex epoetin alfa from Johnson & Johnson had a higher 4-month mortality rate and a lower 12-month survival rate than those on placebo. Both studies attempted to push the limits of hemoglobin levels beyond that permitted for marketing by the FDA - the recommended haemoglobin target for Aranesp was at the time up to 12 g/dL, while the labels for Epogen and Procrit recommended 10-12 g/dL. Henke treated men to target levels of at least 15 g/dL, while women were treated to at least 14 g/dL. The target level in the BEST study was 12- 14 g/dL.

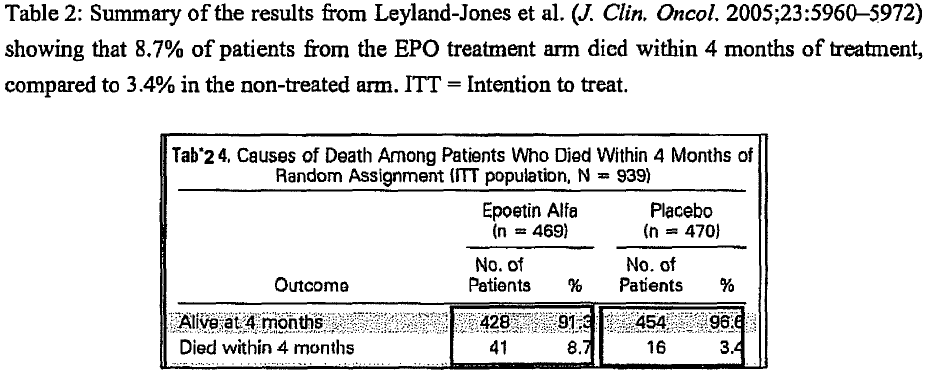

Johnson & Johnson (JNJ, New Brunswick, N.J.) have since reported data from the Phase IV CHOIR trial (N. Engl. J. Med. 2006 Nov 16;355(20):2085-98.) that tested whether using Procrit epoetin alfa to get hemoglobin levels to 13.5 g/dL would improve outcomes vs. treating to 11.3 g/dL (within the 10-12 g/ dL range on the drug's label). Patients in the higher haemoglobin group had a significantly increased incidence of mortality and cardiovascular events. While this study was carried out in the renal disease space, the safety implications were further emphasized in a more recent study - DAHANCA10. In February 2007, Amgen disclosed that this independent study had been halted three months earlier after interim data showed that Aranesp plus radiation missed the primary endpoint of 3-year loco-regional control vs. radiation alone. The study also showed a non-significant increase in death in the Aranesp arm. DAHANCA10 explored whether the use of Aranesp to maintain a hemoglobin level of 14-15.5 g/dL during radiotherapy could improve loco-regional disease control in patients with primary head and neck squamous cell carcinoma (HNSCC).

Safety signals also emerged from the use of Aranesp in the AoC space (study 103). In January 2007, Amgen reported that the risk/benefit profile of Aranesp was "at best neutral" in a Phase III trial in patients who had AoC and who were not receiving chemo- or radio-therapy. Here the data revealed significantly more deaths in Aranesp patients than in placebo patients. The trial, which treated patients to a haemoglobin level of 12-13 g/dL, also missed its primary endpoint of a significant reduction in transfusion frequency at 16 weeks. Study 103 enrolled patients with various cancers, including non-small cell lung cancer (NSCLC), breast cancer and prostate cancer. Canadian researchers have published similar findings (J. Clin. Oncol. 2007 Mar 20; 25(9):1027-32). Here the authors showed that of the 70 advanced NSCLC patients with AoC, those receiving Procrit, had a significantly higher mortality rate than those receiving placebo. A synopsis of each of these studies is provided in Table 3 below:

**Table 3: Summary of results from EPO safety studies highlighting survival issues.**

| STUDY | EPO type | POPULATION | DESIGN | STATUS |
|---|---|---|---|---|
| DAHANCA (SE20029001) | *Aranesp* | HNSCC; Baseline Hb <= 14.5 | Multicenter, open-label trial of radiotherapy +/- Aranesp | Terminated early by DMC (after 522 of 600 planned patients enrolled) based on lower LRC rates and Increased deaths in the ESA arm at planned interim analysis; 522 of 600 planned pts; summary results 12/06; CSR anticipated 9/08 |
| EPO-CAN-20 | *Eprex*/*Procrit* | NSCLC not receiving chemo; baseline Hb <=12 | Double-blind, placebo controlled, randomized (1:1) +/- Eprex | Terminated early by DSMB for Increased deaths in ESA arm; 70 of 300 patients enrolled; results published in abstract in 2004 and In the journal of clinical oncology 3/07 |
| BEST (EPO-INT-76) | *Eprex*/*Procrit* | Metastatic breast cancer | Randomised, double blind, placebo controlled | Terminated in April 2002, after review of data In the first 938 pts by the DMC, due to evidence of excess mortality In the Eprex arm |
| RTOG 9903 | *Eprex*/*Procrit* | HNSCC; baseline Hb 9-12.5 (female), 9-13.5 (male) | Open-label, randomized (1:1), chemo/radiation +/- procrit | Terminated early by DSMB for trend to poorer LRC and OS In EPO arm, 14B of 372 patients enrolled. Results published in abstract 2004 |
| Study 103 (Amgen) | *Aranesp* | NSCLC, prostate, breast cancer | | |

### EPO clinical safety; a role for EPOR?

These clinical findings have 1ed many investigators to suggest a possible role for ESA's in promoting tumour growth through stimulation of EPO receptor survival signalling in tumour cells, and via the stimulation of angiogenesis. Implicit in these proposed activities is the notion that the EPO receptor can somehow confer survival advantage to cancer cells, a negative side effect. This, in turn, suggests that EPO receptor is both present and activated by EPO binding in such cells. Using real-time, quantitative RT-PCR, the EPOR gene has not only been shown to be strongly expressed in bone marrow (containing the EPO-responsive erythroid progenitors), but also at significant levels in normal tissues (*e.g.* kidney, heart, brain, endothelium, and smooth muscle). Moreover, EPOR transcript levels in breast, colon, ovary, prostate, lung, lymphoma, ileum, stomach, and kidney tumour tissues and tumour cell lines were no higher than those levels observed in normal tissue counterparts. These findings are in concordance other reports which demonstrated that EPOR transcript levels are basically equivalent in matched tumour and non-tumour samples from patients with lung, colon and prostate cancer. From the perspective of these data, it is questionable whether the EPOR gene might somehow provide selective advantage to tumour cells, at least via abnormal expression levels.

Therefore, there is a possible role for EPOR in mediating tumour cell survival in response to EPO. From a molecular perspective, the ability of cancer cells to subvert the EPO/EPOR system would not be surprising. A number of preclinical studies have demonstrated EPO-mediated activation of the mitogen-activated protein kinase (MAPK), phosphatidylinositol 3-kinase (PI₃K)-Akt, JAK-STAT (Janus kinase-Signal Transducer and Activator of Transcription), and nuclear factor-kappa B (NFκB) signalling pathways in a variety of human cancers. Each of these signalling cascades has been associated with cellular functions that promote tumour progression. EPO stimulated not only chemotaxis of endothelial cells, together with migration and invasion of breast cancer and HNSCC cells, but also appears to induce cancer cell proliferation and inhibit apoptosis. Moreover, pretreatment with rHuEPO protects some cancer cell lines from the cytotoxic effects of the chemotherapeutic agent, cisplatin. Thus, EPO/EPOR signalling appears to contribute to a wide variety of tumour-promoting functions in different cancer types.

Despite this evidence, the possible contribution of EPO/EPOR signalling to cancer progression is anything but straightforward. The influence of EPO/EPOR on different cancer types appears to be quite variable and remains incompletely understood. Studies have shown that EPO does not influence the proliferation of cancer cell lines. Rosti et al. (Haematologica 1993 Jul-Aug; 78(4):208-12.), for example, investigated the proliferative potential of rHuEPO by testing the effects of this factor on clonogenic growth and DNA synthesis in 10 different cell lines derived from haematologic malignancies and solid tumours. The cell lines K-562 and HEL were included in this study, both of which express EPO receptors. Results showed that rHuEPO had no effect on either colony growth or DNA synthesis (see Table 4).

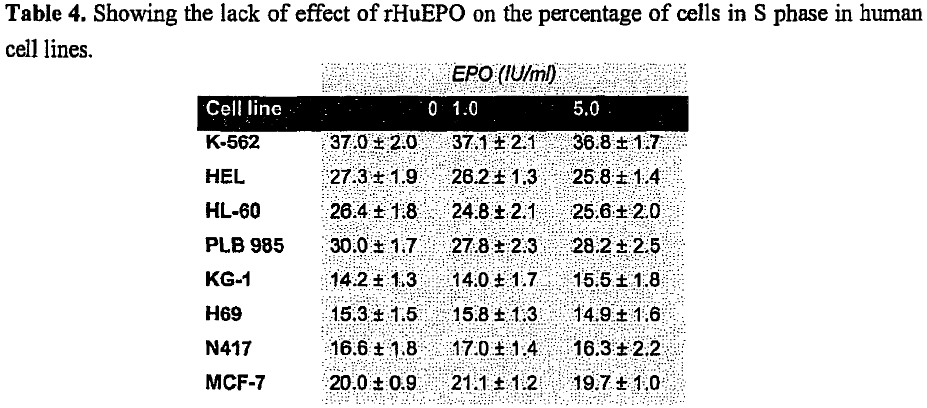

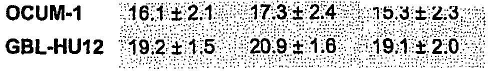

In a similar study, Westphal et al. (Tumori 2002 Mar-Apr; 88(2):150-9.) investigated the effects of EPO on more than 25 different benign and malignant human cell lines. Expression of EPO receptor mRNA and protein was analyzed with RT-PCR, Western blot, and immunocytochemistry. Cellular responses to various concentrations of EPO were evaluated using tritiated thymidine uptake, Northern blot analysis of c-fos expression, and tyrosine-kinase activity assay. EPO receptor mRNA and protein were identified in the majority of the tumour cell lines evaluated. Despite these findings, treatment with rHuEPO did not significantly influence the proliferation rate of EPO-receptor-positive tumour cell lines. Moreover, treatment with EPO neither affected the gene c-*fos* mRNA of those cell lines nor stimulated tyrosine-kinase activation. Based on their findings, the authors concluded that expression of the EPO receptor in tumour cells does not appear to be essential for growth and therefore should not have a deleterious effect in cancer patients.

### Might a novel EPO receptor explain these clinical and molecular findings?

Results by Lu et al. (J. Biol. Chem., Vol. 281, Issue 11, 7002-7011, 2006) establish that receptor activation is not simply accomplished by bringing two receptors into close proximity through disulfide linkages in the transmembrane or extracellular domains. Instead, the relative orientation of the two transmembrane domains of an EpoR dimer, rather than their proximity, determines the extent of receptor activation. More specifically, these authors propose that Epo binding to the inactive, symmetric EpoR dimer causes the repositioning of the two fibronectinIII domains to an asymmetric 120° relative orientation, which in turn changes the orientation of the transmembrane domains and intracellular domains, and juxtaposes the appended JAK2s to initiate the phosphorylation cascade. EPO mutants would not necessarily be expected to be capable of initiating EPOR-signalling, due to their inability to induce the correct relative conformation of the fibronectinIII domains. Interestingly, it appears that certain aspects of EPO function can be decoupled from EPOR activity. Leist et al. (Science 305, 239-242.) have shown that the haematopoietic and tissue-protective activities of Epo are distinct and separate, demonstrating for example that carbamylated Epo (CEpo) does not stimulate erythropoiesis, yet prevents tissue injury in a wide variety of *in vivo* and *in vitro* models.

### EPO and neuronal protection:

EPO's efficacy in treating nervous system disease has been demonstrated in several experimental models of brain and spinal cord injury. As such, EPO has become a candidate for therapeutic neuro-protection. Notwithstanding, the use of EPO as a neuro-protectant raises several safety issues. Although recombinant EPO seems to be potentially safe at neuroprotective proven doses, cardiovascular or cerebrovascular events can occur as a result of its bone marrow stimulating activities. Interestingly, as highlighted above, EPO's neuronal protective function appears molecularly separable from the haematopoietic activity, as carbamylated EPO and certain EPO mutants are neuroprotective but fail to induce haematopoiesis. Such mutants fail to bind EPOR (Leist et al. Science 305, 239-242).

### Synopsis:

EPO was for a long time considered to act solely on haematopoietic cells, a fact which led to its emergence as a leading treatment for chemotherapy-induced anemia. However, emerging evidence has shown that EPO is expressed in a variety of tissue and cell types, including cancer, vascular endothelial, and neuronal cells. Expression of EPO is induced in response to hypoxia, an event mediated by the HIF-1 transcription factor. EPO is prototypically thought to exert its biological effects via binding to its cell surface receptor EPOR, resulting in tyrosine phosphorylation of the receptor and other intracellular proteins, including JAK2 and STAT5. The JAK/STAT pathway is utilized both in haematopoietic and non-haematopoietic cells (including brain cells) following binding of EPO to the EPO receptor. The recent findings of EPO-receptor expression in human breast and renal cancer cells, as well as in several tumour cell lines, have raised important questions in the oncology setting about a possible tumour-growth-promoting effect of rHuEPO on EPO-receptor-bearing tumours. This possibility has been borne out in several clinical trials. Interestingly, other studies have shown that certain EPO mutants which are cytoprotective but not longer able to induce haematopoiesis, function independently of EPOR. This suggests that another EPO receptor may exist which lacks EPOR's strict binding conformation requirements.

In view of the disclosure provided herein and its related discovery, a brief description of ephrin receptors and ephrin biology is provided as follows.

### Ephrin and Ephrin Receptor Biology

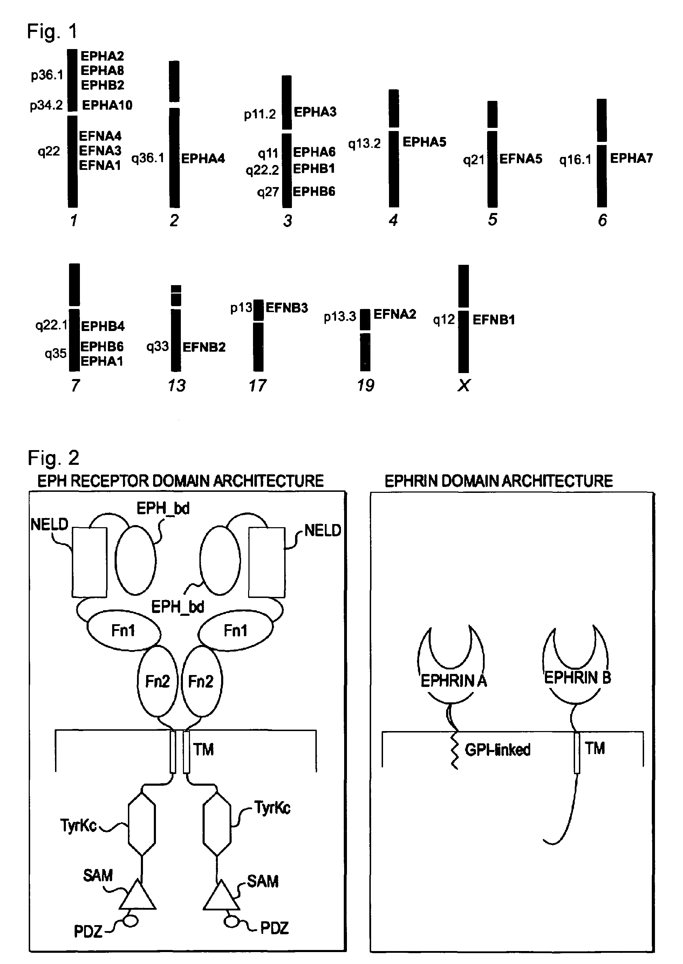

Erythropoietin-producing hepatocellular carcinoma (Eph) receptors form the largest family of receptor tyrosine kinases. Eph receptors are divided into two groups (Eph-A's and Eph-B's) based on the similarity of their extracellular domain sequences and the distinct structural properties of the ephrin ligands (Eph Nomenclature Committee, 1997). About 16 ephrin receptor genes (EphA1-10, EphB1-6) have been identified in the vertebrate genome (Pasquale, Nat. Rev., Mol. Cell Biol. 6 (2005), pp. 462-475.), 14 of which are present in humans (Figure 1) and other mammals (EphA1-8, EphA10, EphB1-4, EphB6).

Eph receptors are single-pass transmembrane proteins with highly conserved extracellular and intracellular domains. The former domains consists of an N-terminal ligand binding domain, a cysteine-rich EGF-like region and two fibronectin type III repeats (Yamaguchi and Pasquale, Curr. Opin. Neurobiol. 14 (2004), pp. 288-296.). Intracellularly, the juxtamembrane region is followed by a tyrosine kinase domain, followed by a sterile-α-motif (SAM), and a type-II PSD-95/Disc large/ZO-1 (PDZ) binding motif at the carboxyl terminus (Kullander and Klein, Nat. Rev., Mol. Cell Biol. 3 (2002), pp. 475-486.). The tyrosine kinase domain of one receptor from each class (EphA10 and EphB6) lacks residues that are essential for catalytic activity. Eph receptor variants are generated by alternative splicing and their structures differ from the prototypical domain structure. The domain architecture of Eph receptors and Ephrins (A and B subclasses) are shown in Figure 2.

Eph receptors can undergo *cis*-oriented homo- as well as heterodimerization (Freywald et al., J. Biol. Chem. 277 (2002), pp. 3823-3828.), which is mediated directly by the extracellular cysteine-rich region, the fibronectin type III repeats (Lackmann et al., J. Biol. Chem. 273 (1998), pp. 20228-20237.) and the SAM motif (Stapleton et al., Nat. Struct. Biol. 6 (1999), pp. 44-49. and Thanos et al., Science 283 (1999), pp. 833-836.) or indirectly through PDZ protein interactions (Fanning and Anderson, J. Clin. Invest. 103 (1999), pp. 767-772). *Trans*-oriented interactions typically occur with select ephrin molecules on opposing cells. In common with their receptors, the ephrins (named derived from Eph family receptor interacting proteins or *ephoros*) are divided into two distinct subclasses A and B. Ephrin-A ligands are GPI-anchored peripheral membrane molecules. In contrast, ephrin-B ligands are transmembrane molecules whose short cytoplasmic domain is capable of participating in various signalling events. The ephrin-A and ephrin-B molecules were initially described as selectively interacting with EphA and EphB receptors, respectively. However, there may be crosstalk between A and B family members. For example, ephrin-A5 is capable of binding EphB2, while EphA4 binds to ephrin-A and ephrin-B family members. Although interactions across classes are limited, within a class they are promiscuous, with multiple EphA receptors binding to a given ephrinA and vice versa.

While neither class of ephrins possesses a catalytic activity, both can activate signal transduction pathways after interaction with Eph receptors (reverse signalling). Reverse signalling activated by transmembrane ephrins includes tyrosine phosphorylation of their cytoplasmic tail and interaction with various signalling molecules. The mechanism by which GPI-linked ephrins stimulate downstream signalling is still unclear.

Signalling sometimes involves formation of signalling assemblies, a process that begins with a monovalent interaction (nanomolar affinity) between an Eph receptor and an ephrin on a juxtaposed cell. Crystallographic work has shown that the globular ephrin-binding domain of EphB2 contains a cavity that accommodates a hydrophobic protrusion from the ephrins. Structural changes occur upon binding. For example, EphB2 undergoes different structural rearrangements upon binding to ephrin-B2 or ephrin-A5.

A lower affinity binding interface is also present on the opposite side of the EphB2 ligand binding domain (Eph_1b), with complementary interfaces also present in the Eph-receptor-binding domain of ephrin-B2. While only of micromolar binding affinity, the second interface can mediate the dimerization of two Eph-ephrin dimers into a tetramer that comprises two receptor and two ephrin molecules extending from adjacent cell surfaces. The lower-affinity interface contains important determinants of subclass specificity and is not engaged in the EphB2-ephrin-A5 complex.

Signalling is initiated upon transphosphorylation via correctly orientated kinase domains. Eph receptors become extensively phosphorylated upon activation by ephrins and via src-kinase association. Phosphorylation promotes conformational order on the activation segment of the kinase domain that favours substrate binding and also disrupts intra-molecular inhibitory interactions that occur between the juxtamembrane segment and the kinase domain. Src-family mediated phosphorylation of Eph receptors has also been shown to act in a similar manner.

### Summary

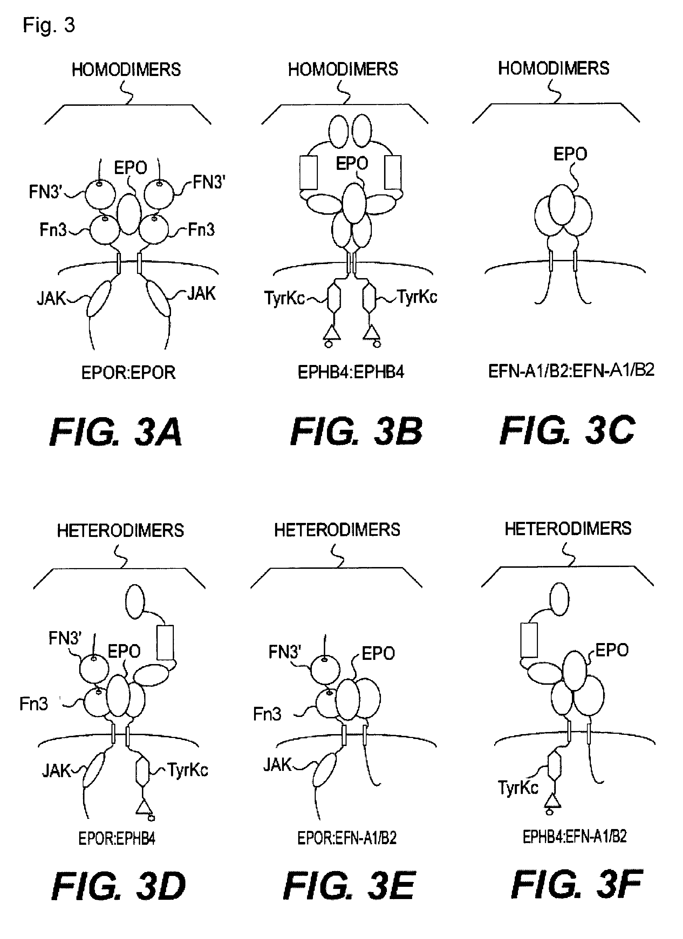

The object of the present invention is defined by the appended claims. Members of the ephrin family (ephrinA1 and EPH-B4) are disclosed as mediators of cytoprotective EPO signalling, either as homodimers and/or as heterodimeric partners of EPOR and/or each other. Our data emphasize the importance of EPH-B4 and EphrinA1 in mediating this function. As such, NEPOR represents a novel EPO receptor derived from a unique combination (*i.e.* via homo- and/or hetero-dimerization) of components derived from ephrin biology and possibly the EPO receptor. See figure 3 for summary.

The present disclosure is based upon the data that EPH-B4 and EphrinA1 are the components of a novel EPO receptor (NEPOR). We are able to show that EPO stimulates enhanced tumor growth in a mouse tumor model system. EPO stimulates the Akt signalling pathway in cell lines lacking EPO receptor expression. These cells express EPH-B4 which is a receptor that stimulates signalling via the Akt pathway. Furthermore in a mouse tumor model it can be shown that EPO is capable of stimulating significant tumor growth. Such activity is inhibited via kno-down of the EPH-B4 receptor highlighting the EPH-B4 dependant nature of a EPO mediated tumor genesis. As such, NEPOR is primarily composed of EPH-B4 as a homodimer and/or in heterodimeric association with EPOR or an Ephrin. Furthermore, *in silico* analyses points to structural complementarity between EPO and Ephrin molecules, particularly Ephrin A1. Thus, NEPOR may also be composed of EphrinA1 as a homodimer and/or in heterodimeric association EPH-B4. A summary of these putative NEPOR species is provided in Figure 3 and Table 5.

The present disclosure provides a method for assessing a tissue for expression of the tissue protective NEPOR receptor complex and/or EPH-B4 and/or Ephrin A1. In so doing, the present disclosure provides a prognostic method to stratify patients having a tumour as suitable (NEPOR not present on the tumour, NEPOR-) or non-suitable (NEPOR present on the tumour, NEPOR+) for EPO treatment. Specifically, the method for assessing tumour tissue NEPOR and/or gene expression components comprises:
(a) isolating a tissue sample from an individual who is receiving or shall receive erythropoietin,
(b) determining the level of expression of the NEPOR gene transcript(s) (*i.e.* EPH-B4, and/or Ephrin A1 mRNA) and/or the presence of the NEPOR gene products (*i.e.* EPH-B4, and/or Ephrin A1 proteins) from the isolated tissue, and
(c) correlating the presence of these NEPOR component gene expression products to a negative physiological response to the treatment with erythropoietin.

Preferably, the expression of the NEPOR component genes (*i.e.* EPH-B4, and/or Ephrin A1 mRNA) is determined by a molecular biological technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF) and combinations thereof.

Preferably, the determination of the presence of the NEPOR gene products is done by detecting the respective proteins with an immunoassay procedure, where the immunoassay procedure is selected from the group of immunoprecipitation, enzyme immunoassay (EIA), radioimmunoassay (RIA) or fluorescent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a luciferase-assay. The immunoassay procedure is most preferably based on ELISA.

Preferably, the method for detection of NEPOR and/or EPH-B4, and/or Ephrin A1 on tumour tissue can also be an *in situ* imaging method, comprising administering an anti-NEPOR antibody or NEPOR binding peptide linked to a radio-ligand or other imaging agent, and measuring for tissue distribution and location of the radio-ligand or other imaging agent. Preferably, the tissue sample is selected from the cancerous tissue or circulating cells derived from same, or from a group of biological tissues and fluids such as blood, lymph, urine, cerebral fluid. Specifically, the individual is a cancer patient who is to be treated with erythropoietin or is being treated with erythropoietin. Preferably, the negative physiological effect is increased tumor progression and/or poorer patient survival. Preferably, the presence of NEPOR gene products and/or EPH-B4, and/or Ephrin A1 is indicative of increased tumor progression and/or poorer patient survival upon treatment with erythropoietin. Preferably the cancer is one of head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer. It is particularly preferred that the cancer is selected from the group of squamous cell carcinoma of the head and neck (HNSCC), prostate cancer, colorectal cancer, lung cancer, kidney cancer, brain cancer, bladder cancer and breast cancer.

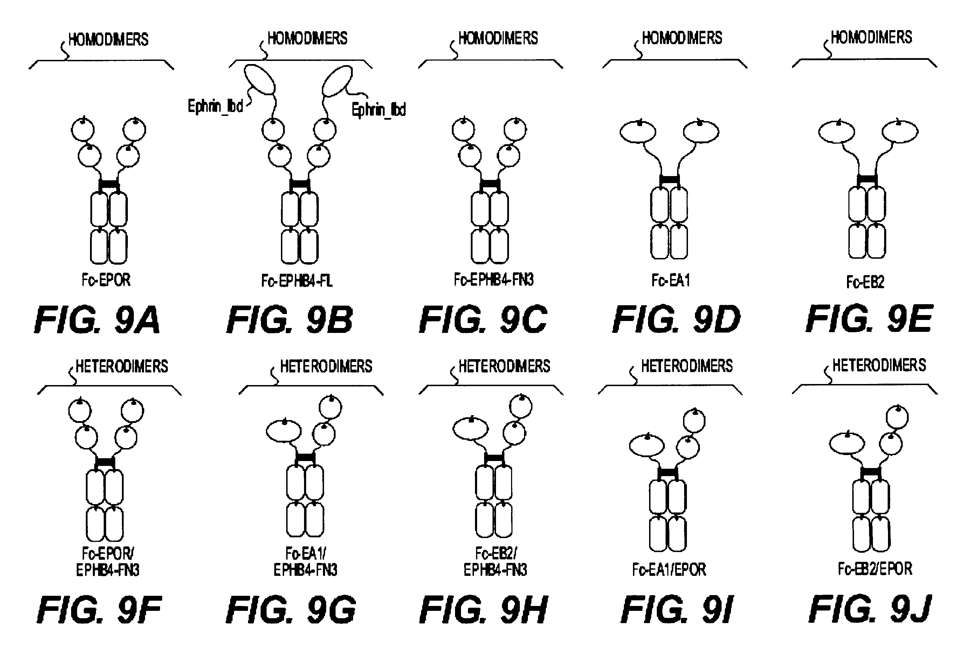

The present disclosure further provides a method for designing a therapy which modulates the activity of NEPOR and/or EPH-B4, and/or Ephrin A1, comprising:
1) performing an *in vitro* screening assay for NEPOR and/or EPH-B4, and/or Ephrin A1 specific therapies; by measuring the binding of test compounds to a tissue protective NEPOR receptor complex and/or EPH-B4, and/or Ephrin A1 (also in comparison to EPOR homodimer complexes), wherein the test compound is labelled (binding of the labelled test compound to the receptor complexes detailed in figure 9) and is measured by detecting the label attached to the test compound;
2) performing a label-free screening approach such as surface plasmon resonance. In this case the test compound is not labelled and its binding to NEPOR receptor complexes (as detailed in figure 9) is measured by a label independent (optical) method.
3) testing NEPOR and/or EPH-B4, and/or Ephrin A1 activity by (a) contacting a test compound with a tissue protective NEPOR receptor complex (N) or tissue protective NEPOR receptor complex-expressing cell; measuring the level of the activity of (N) in the cell; identifying a test compound that increases or decreases the level of activity of (N) as compared to the level of activity of (N) measured in the absence of the test compound; and assaying the identified test compound for tissue protective activity;
4) testing the modulation of NEPOR/ligand binding and/or EPH-B4, and/or Ephrin A1 ligand binding by (a) contacting (N) with a tissue protective NEPOR receptor complex ligand and/or EPH-B4, and/or Ephrin A1 ligand attached to a first label, and an equivalent amount of a test compound attached to a second label under conditions conducive to binding, removing unbound material from (N), and detecting the level of the first and second labels, where if the second label is present the compound binds (N) and if the level of the first label decreases relative to the level of the first label when the labelled ligand is contacted with (N) under conditions conducive to binding in the absence of a test compound after removal of unbound material, then a compound that binds to (N) is identified.
5) identifying a compound that modulates a tissue protective activity in a mammal, comprising: (a) administering the compound to a first animal immediately following infliction of an injury, wherein the first animal endogenously expresses a tissue protective NEPOR receptor complex; and (b) administering the compound to a second animal immediately following infliction of the same injury as in step (a), wherein the second animal is deficient in expression of a tissue protective NEPOR receptor complex and/or EPH-B4, and/or Ephrin A1 or components thereof; such that if recovery from the injury differs in the animal of step (a) as compared to the animal of step (b), a compound that modulates a tissue protective activity is identified.

The present disclosure further provides methods for treating or preventing a disease or disorder in a human comprising administering a therapeutically effective amount of a compound that modulates the activity of a tissue protective NEPOR receptor complex to a human in need of such treatment or prevention, with the proviso that the compound is not EPO. The compound is selected from the group consisting of an antibody specific for the tissue protective NEPOR receptor complex, an antibody is specific for a tissue protective NEPOR receptor complex ligand, a small molecule, a peptide, an EPO mutant, an EPO:Ephrin_ligand_binding domain chimera, a member of a library, and a combination thereof. Preferably, such compounds negatively modulate the tissue protective function of the NEPOR receptor complex in the aforementioned mentioned cancers. Preferably such compounds positively modulate the tissue protective function of the NEPOR receptor complex wherein the disease or disorder is caused by hypoxia, seizure disorders, neurodegenerative diseases, neurotoxin poisoning, multiple sclerosis, hypotension, cardiac arrest, radiation, or hypoglycemia.

The present disclosure further provides a method for identifying compounds that modulate NEPOR's tissue protective signalling activity, comprising (a) contacting a test compound with the NEPOR receptor complex expressing cell; (b) measuring the level of tissue protective activity initiated by NEPOR activation in the cell; (c) identifying a test compound which increases or decreases the level of tissue protective NEPOR complex activity in a cell; (d) assaying the identified compounds for tissue protective activity mediated via NEPOR; and (e) assaying the identified therapeutics for NEPOR inhibitory activity. Preferably, the assay in step (d) is a tissue protective NEPOR receptor complex activity is measured by a cell proliferation/differentiation assay. More preferably, the cells in the cell proliferentiation/differentiation assay are recombinantly engineered to express EPH-B4, and/or EPOR, and/or Ephrin A1. More preferably, the cells endogenously express an EPO receptor and are transformed with a nucleic acid comprising a nucleotide sequence that (i) is operably linked to a promoter, and (ii) encodes either EPH-B4 and/or Ephrin A1. Most preferably, the cells endogenously express EPH-B4 and/or Ephrin A1 and are transformed with a nucleic acid comprising a nucleotide sequence that (i) is operably linked to a promoter, and (ii) encodes an EPO receptor polypeptide.

The present disclosure further provides a method for identifying a compound that modulates the interaction between a tissue protective NEPOR receptor complex and a tissue protective NEPOR receptor complex ligand, comprising: (a) contacting a tissue protective NEPOR receptor complex with one or more test compounds; and (b) measuring the tissue protective NEPOR receptor complex activity, whereby if the activity measured in (b) differs from the tissue protective NEPOR receptor complex activity in the absence of the one or more test compounds, then a compound that modulates the interaction between the tissue protective NEPOR receptor complex and the tissue protective NEPOR receptor complex ligand is identified. Preferably, the tissue protective NEPOR receptor complex activity is measured by cell proliferation or cell differentiation. Preferably, the tissue protective NEPOR receptor complex activity measured is the ability of the tissue protective NEPOR receptor complex to interact with a tissue protective NEPOR receptor complex ligand. Preferably, the step of assaying the identified compound for tissue protective activity comprises detecting the presence of nucleolin in the cell. Preferably, the step of assaying the identified compound for tissue protective activity comprises detecting or measuring an increased level of activity of neuroglobin or cytoglobin in a cell. Preferably, the tissue protective NEPOR receptor complex is in solution. Preferably, the tissue protective NEPOR receptor complex is in a cell. Preferably, the compound inhibits the binding of a tissue protective NEPOR receptor complex ligand to a tissue protective NEPOR receptor complex. Preferably, the compound enhances the binding of a tissue protective NEPOR receptor complex ligand to a tissue protective NEPOR receptor complex. Preferably, the tissue protective NEPOR receptor complex contacted in step (a) is on a cell surface. Preferably, the tissue protective NEPOR receptor complex is on an isolated cell membrane. Preferably, the tissue protective NEPOR receptor complex activity is compared to EPOR receptor activation to identify NEPOR specific compounds. Preferably, the tissue protective NEPOR receptor complex is immobilized to a solid surface and more preferably, the solid surface is a microtiter dish or a chip.

The present disclosure further provides a method for identifying a compound that binds a tissue protective NEPOR receptor complex, comprising: (a) contacting a test compound with a ligand-binding tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support; and (b) contacting a test compound with a ligand-binding EPOR receptor complex fragment comprising at least two EPO receptor extracellular domains fused to an Fc fragment attached to a solid support (c) removing unbound test compounds from the solid supports; (d) identifying the compound attached to the tissue protective NEPOR receptor complex fragment, but not the EPOR receptor complex (and vice versa), whereby a compound bound to the solid support is identified as a compound that binds specifically to a tissue protective NEPOR receptor complex or a compound that binds specifically to an EPOR receptor complex.

The present disclosure further provides a method for identifying a compound that binds a tissue protective NEPOR receptor complex, comprising: (a) contacting a test compound with a ligand-binding tissue protective NEPOR receptor complex fragment comprising at least one EPO receptor or EPH-B4 receptor or Ephrin A1 receptor, extracellular domain fused to an Fc fragment attached to a solid support; (b) removing unbound test compounds from the solid supports; (c) identifying the compound attached to the tissue protective NEPOR receptor complex fragment, whereby a compound bound to the solid support is identified as a compound that binds specifically to a tissue protective NEPOR receptor complex.

The present disclosure further provides a method for identifying a compound that binds to a tissue protective NEPOR receptor complex, comprising: (a) contacting a tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support with (i) a tissue protective NEPOR receptor complex ligand attached to a first label and (ii) an equivalent amount of a test compound attached to a second label under conditions conducive to binding; (b) removing unbound material from the tissue protective NEPOR receptor complex; and (c) detecting the level of the first and second labels wherein if the second label is present the compound binds the complex and if the level of the first label decreases relative to the level of the first label where the labelled ligand is contacted with a tissue protective NEPOR receptor complex under conditions conducive to binding in the absence of a test compound after removal of unbound material, then a compound that binds to a tissue protective NEPOR receptor complex is identified.

The present disclosure further provides a method for identifying a compound that modulates the binding of a tissue protective NEPOR receptor complex ligand to a tissue protective NEPOR receptor complex, comprising: (a) contacting a tissue protective NEPOR receptor complex ligand with a tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support; in the presence of one or more test compounds under conditions conducive to binding; and (b) measuring the amount of tissue protective NEPOR receptor complex ligated bound to the tissue protective NEPOR receptor complex; whereby if the amount of bound tissue protective NEPOR receptor complex ligand measured in (b) differs from the amount of bound tissue protective NEPOR receptor complex ligand measured in the absence of the one or more test compounds, then a compound that modulates the binding of a tissue protective NEPOR receptor complex ligand to the tissue protective NEPOR receptor complex is identified.

Preferably, the amount of bound tissue protective NEPOR receptor complex ligand is measured using a tissue protective NEPOR receptor complex ligand-specific antibody. Preferably, the tissue protective NEPOR receptor complex ligand is labelled and binding of the tissue protective NEPOR receptor complex ligand to the tissue protective NEPOR receptor complex is measured by detecting the label attached to the tissue protective NEPOR receptor complex ligand. Preferably, the tissue protective NEPOR receptor complex ligand is labelled and binding of the labelled ligand to the tissue protective NEPOR receptor complex is measured by detecting the label attached to the tissue protective NEPOR receptor complex ligand. Preferably, the label is fluorescent. Preferably, the test compound is an antibody specific for the tissue protective NEPOR receptor complex. Preferably, the test compound is a small molecule. Preferably, the test compound is a peptide or a member of a library. Preferably, the tissue protective NEPOR receptor complex ligand is EPO, or derivatives thereof. Preferably, the compound binds the tissue protective NEPOR receptor complex or ligand thereof. Preferably, the tissue protective NEPOR receptor complex activity is compared to EPOR receptor activation to identify NEPOR specific compounds.

The present disclosure further provides a method for identifying a compound that modulates a tissue protective activity in a mammal, comprising: (a) administering the compound to a first animal immediately following infliction of an injury, wherein the first animal endogenously expresses a tissue protective NEPOR receptor complex; and (b) administering the compound to a second animal immediately following infliction of the same injury as in step (a), wherein the second animal is deficient in expression of a tissue protective NEPOR receptor complex or components thereof; such that if recovery from the injury differs in the animal of step (a) as compared to the animal of step (b), a compound that modulates a tissue protective activity is identified.

The present disclosure further provides a method for designing a compound which interferes with NEPOR's survival promoting activity, comprising:
(a) providing the molecular makeup of the NEPOR species and providing amino acid sequences of a component NEPOR polypeptides;
(b) using software comprised by the digital computer to design a chemical compound/protein construct which is predicted to bind to NEPOR; and
(c) optionally designing protein constructs which mimic NEPOR in its dimerised/multimerised state (*e.g.* Fc constructs).

The present disclosure further provides a method for identifying compounds that modulate NEPOR's tissue protective signalling activity, comprising (a) contacting a test compound with the NEPOR receptor complex; (b) measuring the level of tissue protective activity initiated by NEPOR activation; (c) identifying a test compound which increases or decreases the level of tissue protective NEPOR complex activity; (d) assaying the identified therapeutics for tissue protective activity mediated via NEPOR; and (e) assaying the identified therapeutics for NEPOR inhibitory activity. Preferably, the tissue protective NEPOR receptor complex activity is measured by measuring the binding of the test compound to the NEPOR receptor complex. More preferably, the test compound is labelled and binding of the labelled test compound to the tissue protective NEPOR receptor complex is measured by detecting the label attached to the test compound. Most preferably, the tissue protective NEPOR receptor complex activity is measured by measuring the binding of the test compound to the tissue protective NEPOR receptor complex.

The present disclosure further provides a method for imaging tumour tissue that is susceptible to enhanced survival in response to EPO treatment, comprising administering an anti-NEPOR antibody or NEPOR binding peptide linked to a radio-ligand or other imaging agent, and measuring for tissue distribution and location of the radio-ligand or other imaging agent. Preferably, the anti-NEPOR antibody is a monoclonal or polyclonal antibody selected from the group of antibodies listed in Table 6.

The present disclosure further provides a method for modulating cell survival in NEPOR positive tissue comprising administering an EPO mutants and peptides selected from the group consisting of peptides from SEQ ID NO. 17 through SEQ ID NO. 212.

The present disclosure further provides a method for modulating cell survival in NEPOR positive tissue comprising administering an effective amount of an EPO chimera, comprising an ephrin receptor ligand binding domain selected from the group consisting of SEQ ID NO. 215, and SEQ ID NO. 216.

### Brief Description of the Figures

Figure 1 shows the genomic localization of human Eph receptor (EPH) and ephrin (EFN) genes on human chromosomes.
Figure 2 shows the domain architecture of Eph receptors and Ephrins (A and B subclasses).
Figure 3 shows a process for identifying putative EPO binding transmembrane receptors. All proteins containing two membrane proximal FN3 domains were extracted (84 in all) and assessed for evidence of response to hypoxia. EPH-B4 was amongst one of four possible proteins extracted. Moreover, it is the only member of the Ephrin receptor family which is embryonic lethal, with death in embryo's preceding that of EPOR knock-outs.

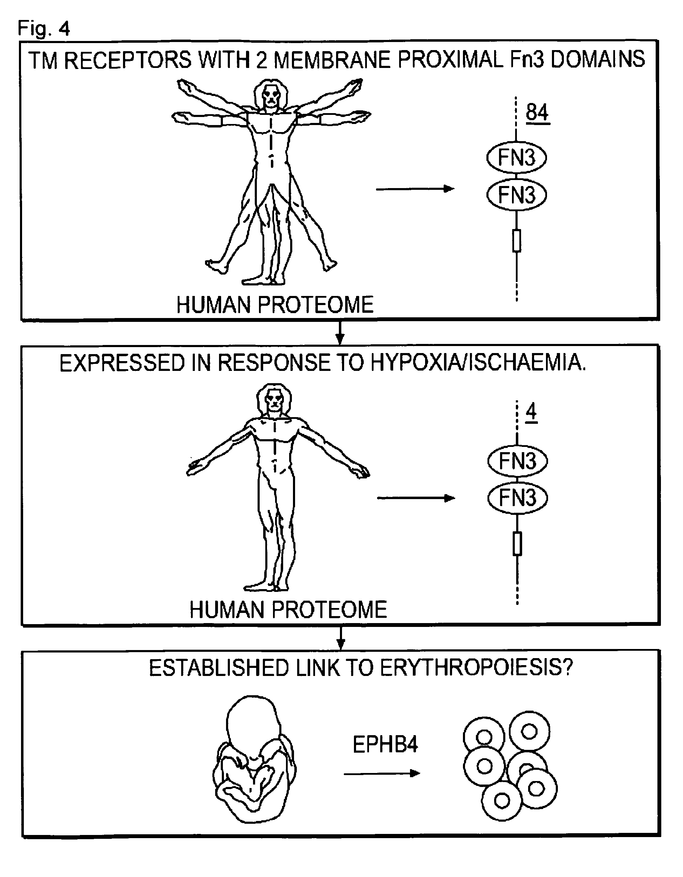

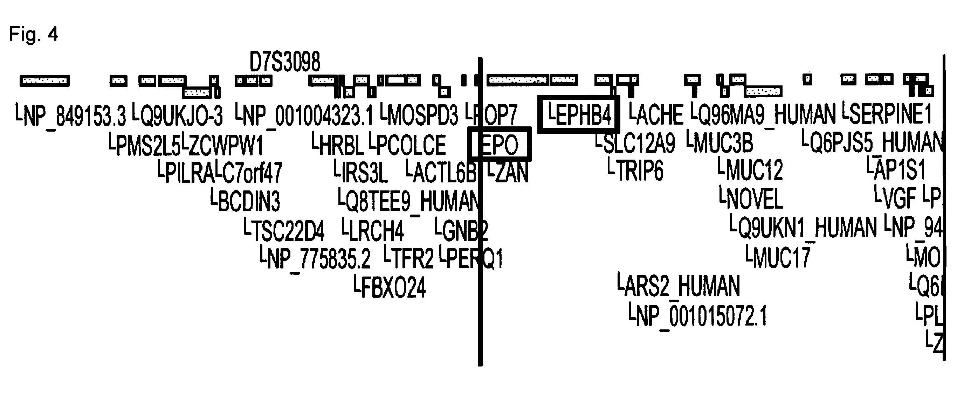

Figure 4 shows the human EPO locus showing the neighbouring EPH-B4 gene.

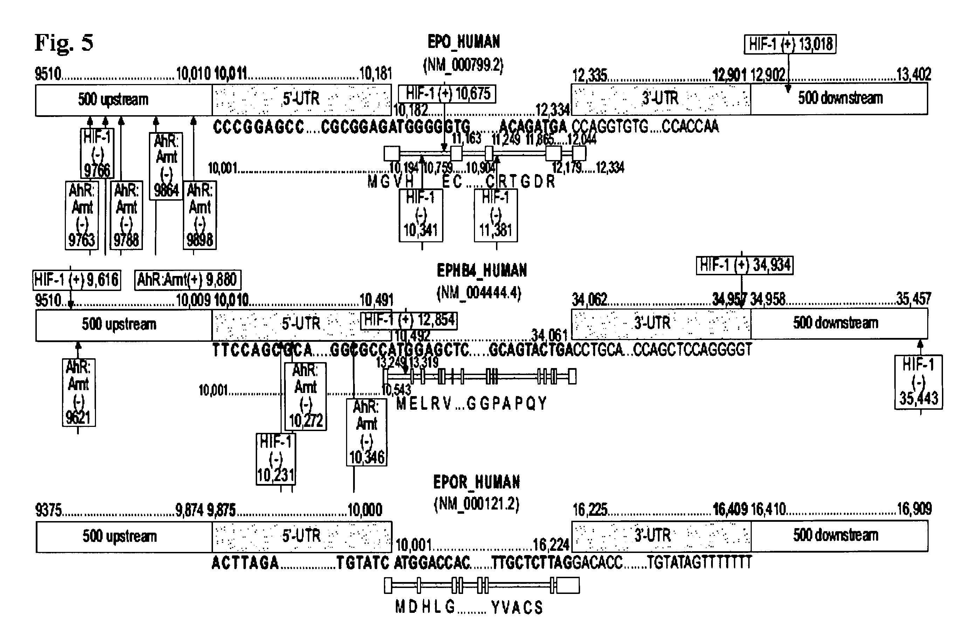

Figure 5 shows a schematic of the results from analysis of the 5' and 3' UTR's (and an additional 500bp on either side) of the EPO, EPOR and EPH-B4 genes for the presence of hypoxia inducible transcription factor binding sites. This study was performed employing the "Match" algorithm from TRANSFAC (Nucleic Acids Res. 2003 Jan 1, 31(1):374-8) to analyse the composition of HIF1 binding sites. Strikingly, only the EPO and EPH-B4 genes were found to contain such sites, supporting the hypothesis that EPH-B4 is indeed hypoxia inducible.

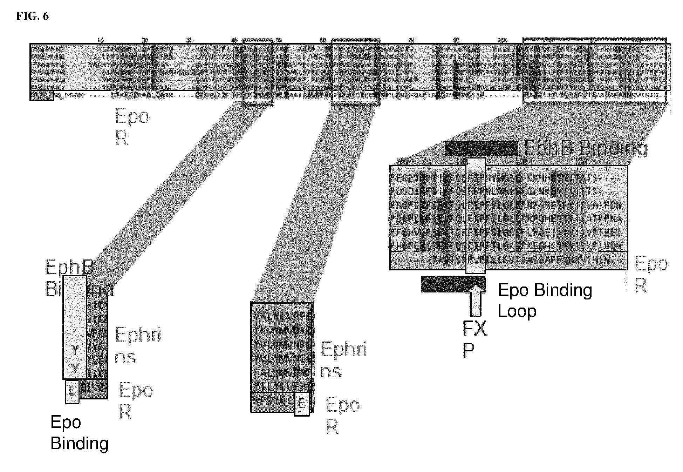

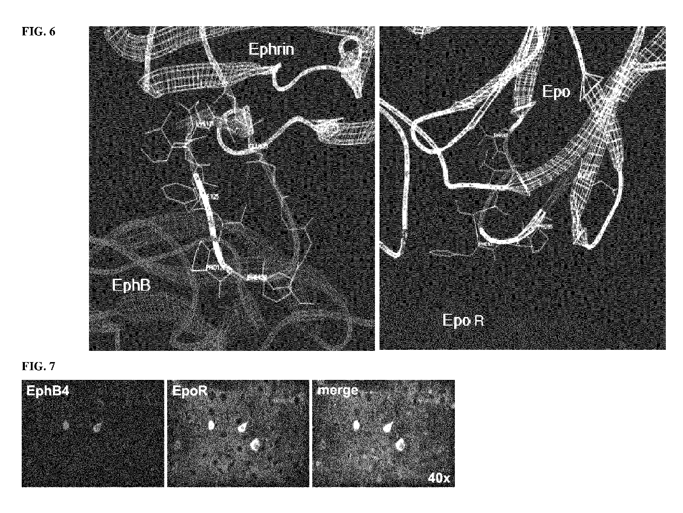

Figure 6 shows a structural analysis of EphrinA5:EphB2 association in comparison with that of EPO:EPOR. This structural analysis reveals several commonalities consistent with a propensity for Ephrin A1 to bind EPO. The top panel shows homology of the EPO binding region of EPOR to the human Ephrin A molecules. The two lower panels compare the structural aspects of Ephrin A with EPOR.
Figure 7 shows staining of hippocampus with anti-EPH-B4 and anti-EpoR antibodies. It should be noted that there is a striking co-expression of both proteins restricted to certain cells only. These data suggest functional coupling of EPH-B4 and EPOR activity.

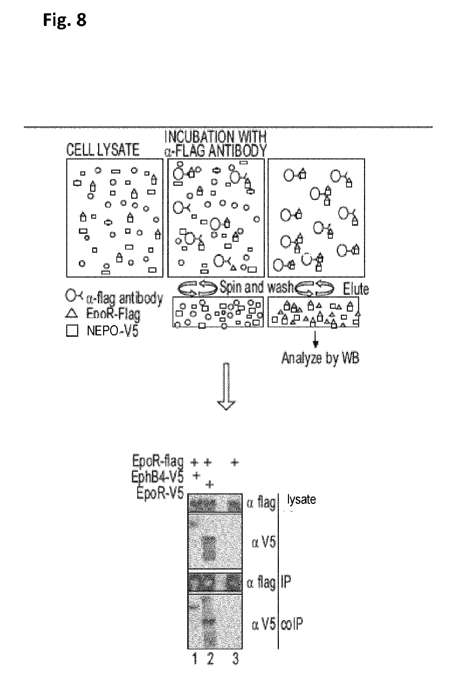

Figure 8 shows co-immunoprecipitation of EPH-B4 using flag-tagged EpoR. This finding is consistent with the notion that EPH-B4 and EPOR might heterodimerize.
Figure 9 shows the possible various species of NEPOR (without being bound by theory). In this representation, NEPOR homo/heterodimer species are shown as Fc constructs. This mimics the dimerization of separate receptor monomers. Any method which allows the production of such NEPOR dimers can be employed in screening for NEPOR specific agonists and antagonists, including small molecules, peptides, proteins and EPO variants.

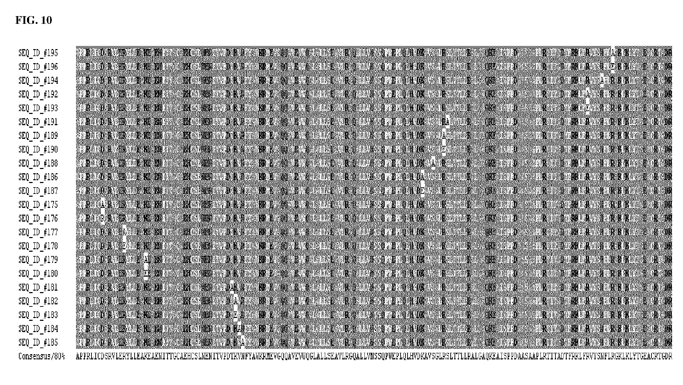

Figure 10 shows an alignment of EPO protein mutants which are predicted to bind NEPOR more favourably than EPOR. Such mutants are predicted to be primarily tissue protective as opposed to haematopoietic, particularly those versions combining the described mutations.

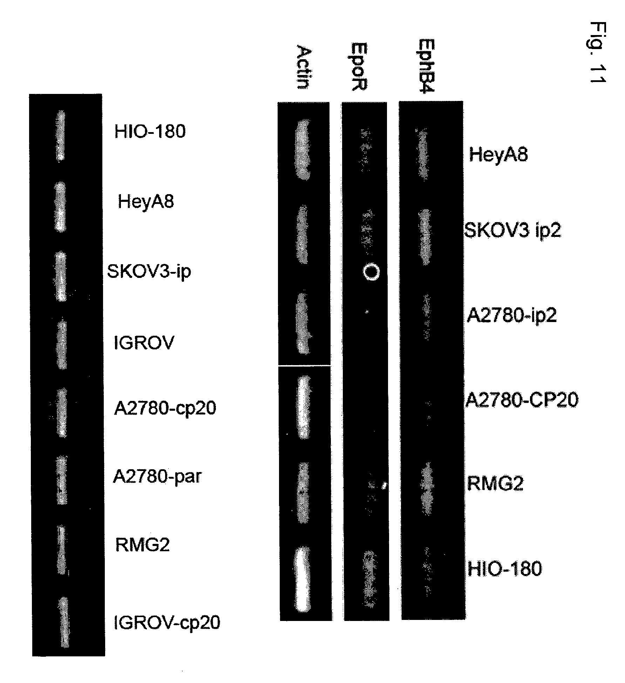

Figure 11 shows mRNA levels of ovarian cancer cell lines. RNA was isolated from a panel ovarian cancer cell lines and was reverse transcribed into cDNA. PCR was done using primers specific for EPO receptor, EPH-B4, Ephrin A1 and actin.

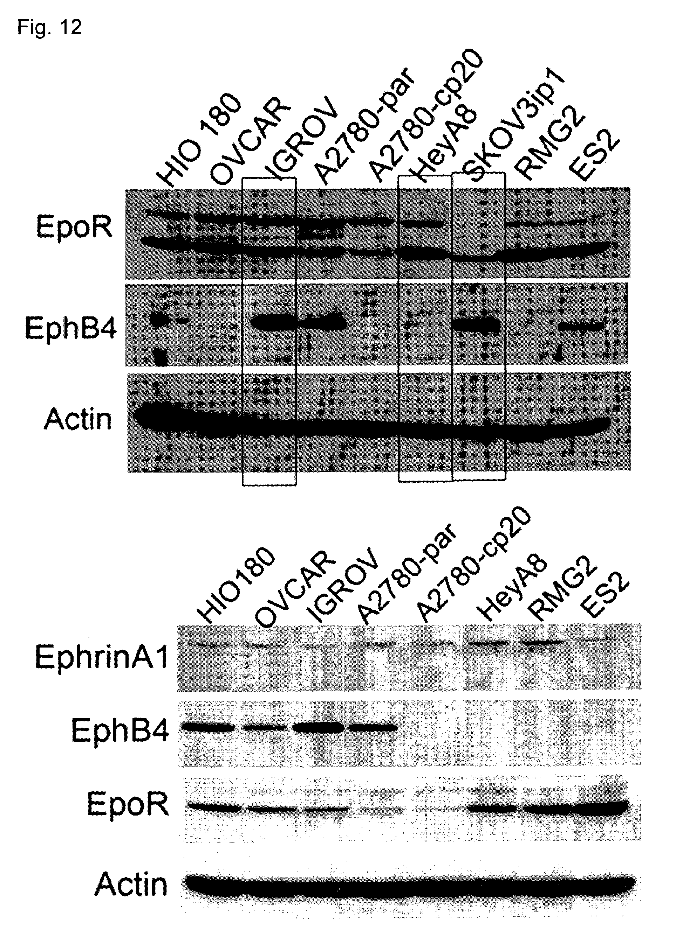

Figure 12 shows protein expression in ovarian cancer cell lines. Protein extracts were isolated from a panel ovarian cancer cell lines. Samples were separated using SDS-Page gel electrophoresis. Immunoblots using antibodies for EPO Receptor (R&D biosystems), EPH-B4 (a gift from Prakash Gil), Ephrin A1 and acting (Sigma Aldrich) were used to compare protein expression.

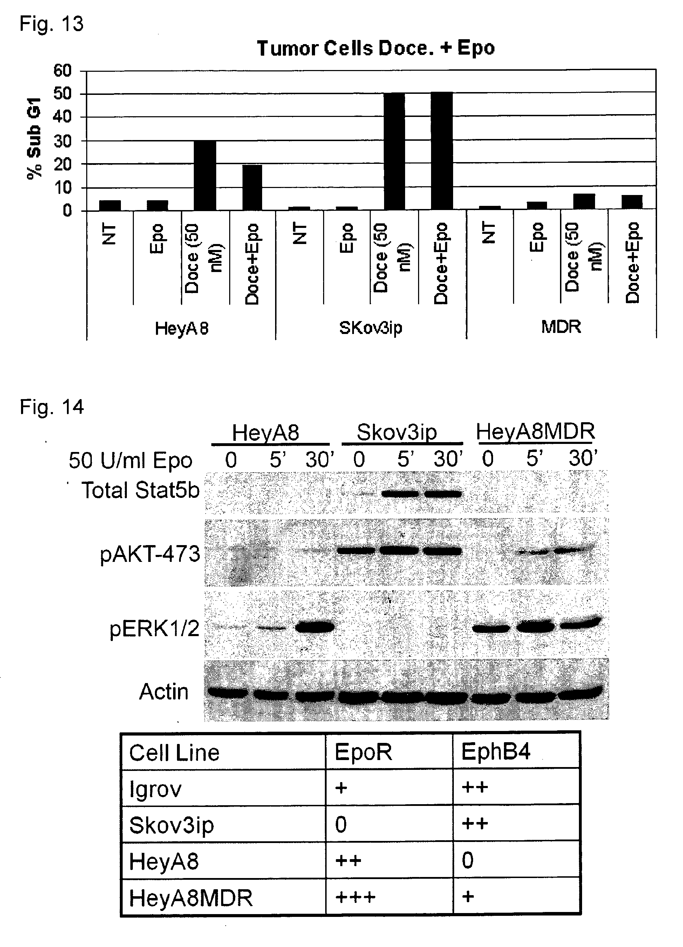

Figure 13 shows ESA protection from chemotherapy induced apoptosis. Ovarian cancer cell lines Hey A8, SkoV3 ipl, and HeyA8-MDR (chemoresistant) were treated with 50 U erythropoietin (EPO), 50 nM docetaxel, or a combination of EPO and docetaxes for 48 hours. Cells were then fixed and DNA stained with propidium iodide. Percentage of sub G1 cells were then quantified using flow cytometer (BD).
Figure 14 shows signalling pathways activated in response to EPO in ovarian cancer cell lines. Cell lines previously characterized for expression levels of EPOR, EPH-B4, and Ephrin A1 were washed and grown in serum free media for two hours. Cells were then treated with 50 U EPO and collected and designated time points (0, 5 and 30 minutes). Protein extracts were isolated and analyzed by immunoblots using antibodies for phosphor-STAT5 (Invitrogen), phosphor-AKT, phosphor-ERK (Cell Signaling) and acting (Sigma Aldrich).

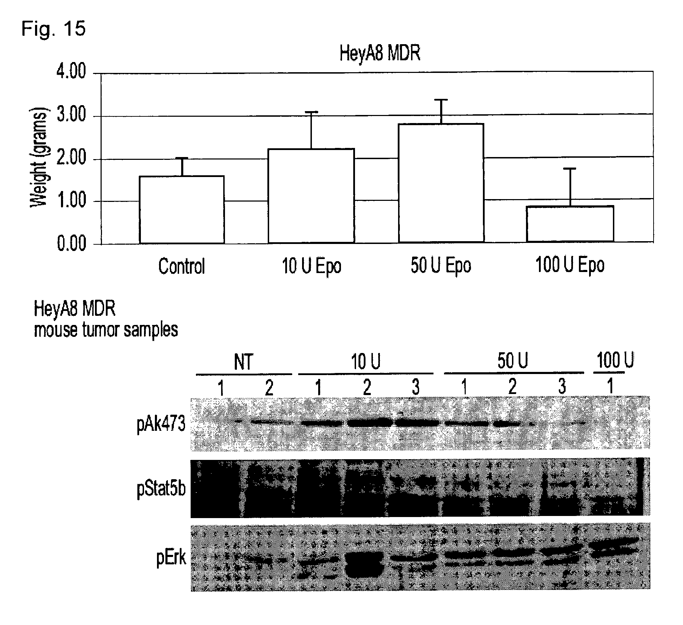

Figure 15 shows erythropoietin induced tumor growth in nude mice. Mice were injected i.p. with 1x10⁶ Hey MDR ovarian cancer cells. Day eight following injections mice were injected with designated amounts of EPO (10, 50, 100 U, three mice per group) every second day. A) Mice were sacrificed at day 26 and tumor weight was measured. B) Protein extracts were isolated from tumors and analyzed by immunoblot using antibodies specific for phosphor AKT ser 473, phosphor ERK (Cell Signaling) and pSTAT5b (Invitrogen).

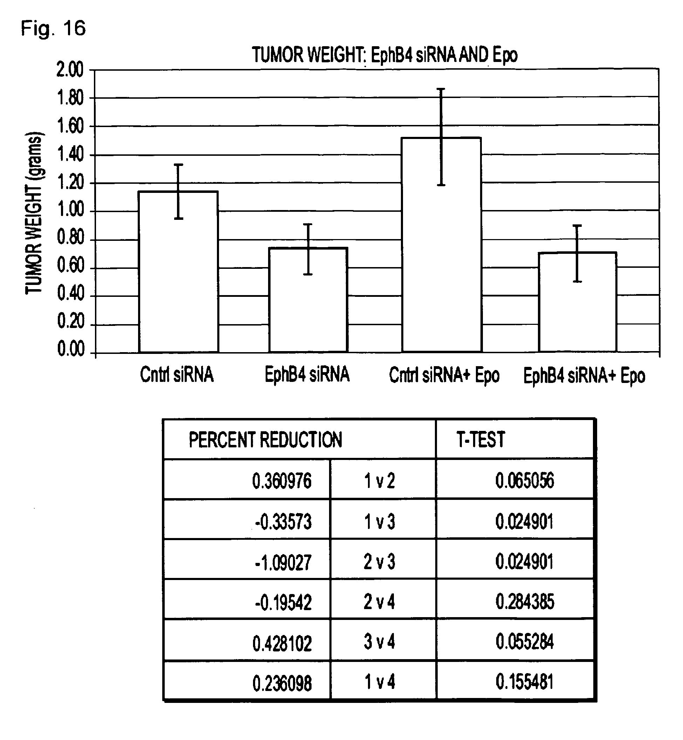

Figure 16 shows EPH-B4 expression effects tumor promoting effect of EPO. Female nude mice were injected i.p. with 1x10⁶ HeyA8-MDR cells. Day eight following injection the cells were treated with control siRNA - DOPC, EPH-B4 siRNA-DOPC, EPO, or in EPO + control or EPH-B4 siRNA-DOPC (10 per group). (50 U EPO given 3 x week, 5 µg siRNA 2 x week). Mice were sacrificed on day 25 and tumor weights were measured. Statistics were done using students T-test. B) Distribution of tumor weight per group.

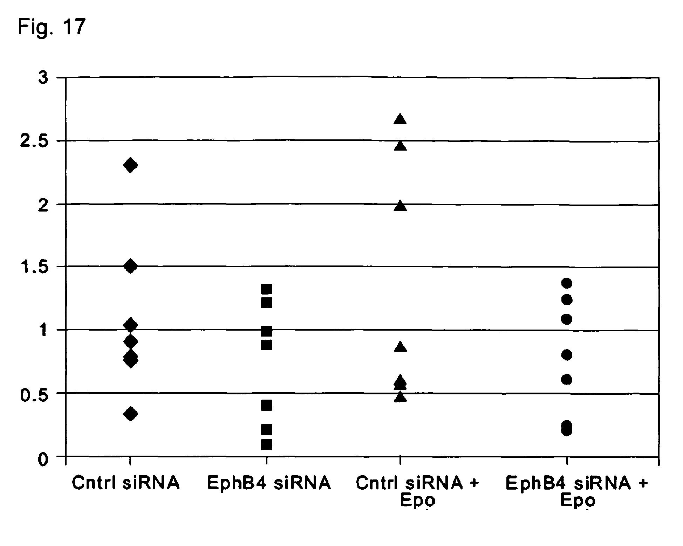

Figure 17 shows tumor weight distributions.

### Detailed Description

The present disclosure results from the identification of a novel EPO receptor, henceforth referred to as NEPOR. NEPOR was identified using a bioinformatics workflow encompassing both a functional and sequence based analysis of the human genome/proteome. Homology analysis involving an extracellular protein database (termed XtraCellDB) was used in conjunction text-mining and genome context analysis. These *in silico* predictions were subsequently verified in lab-based experiments. Thus, the present disclosure provides genomic, proteomic and experiment evidence that the protein EPH-B4 (Erythropoietin Producing Hepatoma protein B4) and/or Ephrin A1 act as EPO receptors.

Working on the theory that the adverse effects of EPO seen in many cancer patients may be mediated by a receptor complex distinct from the prototypical EPO receptor (EPOR) homodimer, we initiated an *in silico* discovery project to try to identify a novel EPO receptor. Should such a novel EPO receptor species exist, we hypothesized that it will be responsible for mediating EPO-induced cell survival activity, as opposed to EPO mediated haematopoietic activity. Thus, we proposed the existence of at least two species of EPO receptor; the prototypical EPOR homodimer which is primarily responsible for EPO's haematopoietic activity, and a novel EPO receptor, termed NEPOR, which is primarily responsible for EPO's cytoprotective activities. The existence of such a novel EPO receptor is compelling for three main reasons. Firstly it allows the prediction of a cancer patients response to EPO. Presence of NEPOR on a tumour cell would imply a negative response to EPO, since binding of EPO by NEPOR would induce a cascade of survival signals within tumour cells and tissues, thus contributing to cancer progression and poorer patient survival. Thus, detection of NEPOR expression in a tumour provides a novel biomarker for stratify cancer patients as suitable (*i.e.* NEPOR not present) or unsuitable (*i.e.* NEPOR present) for EPO treatment. A corollary of this model is a second interesting perspective. If NEPOR is capable of initiating survival signals on cancer cells, then it represents an excellent therapeutic target for treatment of cancers expressing this receptor. Thus, therapeutic molecules targeting and antagonizing the tissue protective function of this receptor should be efficacious anti-cancer agents. Finally, under conditions where induction of cell survival is favourable, such as in response to ischemic stroke, therapeutic molecules capable of activating NEPOR-mediated survival signals provide an efficacious path to treating a variety of neurological diseases. Definition of NEPOR's molecular composition therefore provides the molecular basis for designing such therapies.

It had previously been proposed that rHuEPO can promote tumour growth through stimulation of Epo receptor (EPOR) signalling in tumour cells, and via the stimulation of angiogenesis. Binding of EPO to EPOR homodimers was assumed to somehow confer survival advantage to cancer cells, leading to increased loco-regional progression and poorer survival rates in patients having a form of cancer. However, aware of the binding promiscuity of exogenously administered therapeutics, we were anxious to address the possibility as to whether another receptor might be responsible for the observed negative outcomes, either alone or in functional interaction with EPOR.

In an effort to identify such a novel cytoprotective EPO receptor, we developed an *in silico* based analysis approach specifically designed to mine the human proteome for candidate molecules. Combining the power of text-mining and in-depth bioinformatics analysis, this multi-evidence based approach successfully identified a putative novel EPO receptor. Subsequent lab-based validation supports these findings. Given its established physiological role, we propose that by impinging on this receptors activity, EPO can confer survival advantage to certain cells, including cancer cells and neurons. As a consequence, the expression of this protein on cancer cells can be used to stratify the suitability of cancer patients for EPO treatment. Patients with cancer associated NEPOR expression should be contraindicated for EPO treatment. However, a corollary of this finding is that these same individuals represent excellent candidates for treatment with antagonistic anti-NEPOR therapies. In addition, we also propose that by mediating EPO's cyto-protective activity, NEPOR represents an excellent therapeutic target for a variety of diseases involving tissue ischaemia (*e.g*. stroke).

Thus, in the first instance, the present disclosure provides a method for assessing a tumour for expression of NEPOR. The disclosure provides a method to stratify patients having a tumour as suitable (*i.e*. NEPOR not present) or non-suitable (*i.e*., NEPOR present) for EPO treatment. The method disclosed comprises: (a) isolating a tissue sample from an individual who is receiving or shall receive erythropoietin, (b) determining the level of expression of the NEPOR gene(s) (mRNA) and/or the presence of the NEPOR gene product (protein) from the isolated tissue, and (c) correlating the presence of an NEPOR gene expression product or the presence of NEPOR protein to a physiological response to the treatment with erythropoietin. In a second instance, the present disclosure provides a method for treating patients possessing NEPOR positive tumors. Furthermore, the present disclosure provides a method for treating stroke. Finally, by providing a means of comparing binding affinities of putative therapeutics to both NEPOR and EPOR, the present disclosure provides a method for screening for NEPOR specific therapeutics (both antagonistic therapeutics for cancer, and agonistic therapeutics for treatment of hypoxia associated disease such as stroke). Such therapeutics will lack the haematopoietic activity associated with EPOR binding and signaling.

### NEPOR - Molecular definition

We have identified a novel multimeric EPO receptor, which we term NEPOR. NEPOR comprises EPHB4 and/or Ephrin A1 molecules either as homodimers or heterodimers. Without being bound by theory, these components may also heterodimerize with the EPO receptor. A synopsis of the possible molecular compositions of NEPOR is provided in Figure 3. Despite the room for molecular promiscuity involving other components from ephrin biology, EPH-B4 and/or EphrinA1 are components of a novel EPO receptor (NEPOR). As such NEPOR is primarily composed of EPH-B4 and Ephrin A1, either as a homodimers and/or in heterodimeric association with each other, or the EPO receptor. Without being bound by theory, given the strong functional association between EPH-B4 and Ephrin B2, NEPOR may also comprise Ephrin B2 disclosed herein as SEQ ID NO. 4 (amino acid sequence), SEQ ID NO. 8 (mRNA sequence), and SEQ ID NO. 12 (binding region).

Table 5 shows, without being bound by theory, the possible molecular composition of dimeric EPO receptors. The prototypical haematopoietic EPO receptor (EPOR) represents a homodimer of two EPOR (SEQ ID NO. 1) monomers (1). Our results suggest that a novel tissue protective EPO receptor dimer is comprised of Ephrin A1 (SEQ ID NO. 3) and EPH-B4 (SEQ ID NO.2). Possible scenarios are shown in Table 5.

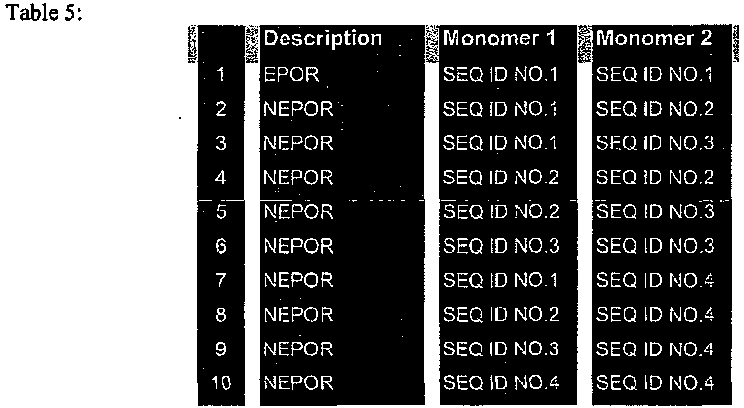

SEQ ID NO.1
   >EPOR 
MDHLGASLWPQVGSLCLLLAGAAWAPPPNLPDPKFESKAALLAARGPEELLCFTERLEDL
VCFWEEAASAGVGPGNYSFSYQLEDEPWKLCRLHQAPTARGAVRFWCSLPTADTSSFVPL
ELRVTAASGAPRYHRVIHINEVVLLDAPVGLVARLADESGHVVLRWLPPPETPMTSHIRY
EVDVSAGNGAGSVQRVEILEGRTECVLSNLRGRTRYTFAVRARMAEPSFGGFWSAWSEPV
SLLTPSDLDPLILTLSLILVVILVLLTVLALLSHRRALKQKIWPGIPSPESEFEGLFTTH
KGNFQLWLYQNDGCLWWSPCTPFTEDPPASLEVLSERCWGTMQAVEPGTDDEGPLLEPVG
SEHAQDTYLVLDKWLLPRNPPSEDLPGPGGSVDIVAMDEGSEASSCSSALASKPSPEGAS
 
AASFEYTILDPSSQLLRPWTLCPELPPTPPHLKYLYLVVSDSGISTDYSSGDSQGAQGGL
SDGPYSNPYENSLIPAAEPLPPSYVACS
SEQ ID NO.2
   >EPH-B4
SEQ ID NO.3
   >EphrinA1 
MEFLWAPLLGLCCSLAAADRHTVFWNSSNPKFRNEDYTIHVQLNDYVDIICPHYEDHSVA
DAAMEQYILYLVEHEEYQLCQPQSKDQVRWQCNRPSAKHGPEKLSEKFQRFTPFTLGKEF
KEGHSYYYISKPIHQHEDRCLRLKVTVSGKITHSPQAHDNPQEKRLAADDPEVRVLHSIG
HSAAPRLFPLAWTVLLLPLLLLQTP
SEQ ID NO.4
   >EphrinB2

MAVRRDSVWKYCWGVLMVLCRTAISKSIVLEPIYWNSSNSKFLPGQGLVLYPQIGDKLDI
ICPKVDSKTVGQYEYYKVYMVDKDQADRCTIKKENTPLLNCAKPDQDIKFTIKFQEFSPN
LWGLEFQKNKDYYIISTSNGSLEGLDNQEGGVCQTRAMKILMKVGQDASSAGSTRNKDPT
RRPELEAGTNGRSSTTSPFVKPNPGSSTDGNSAGHSGNNILGSEVALFAGIASGCIIFIV
IIITLVVLLLKYRRRHRKHSPQHTTTLSLSTLATPKRSGNNNGSEPSDIIIPLRTADSVF
CPHYEKVSGDYGHPVYIVQEMPPQSPANIYYKV

The present disclosure includes any splice variant of the polypeptides of SEQ ID NOS 1-4 components possessing the extracellular EPO binding region (for EPH-B4 this region of proposed to encompass the two fibronectinIII domains; the pink oval structures in Figure 3B, D and F) and the intracellular signalling part, is also capable of mediating EPO's (and derivatives thereof) cyto-protective effect.

### NEPOR: Prognostic implications

The type 1 cytokine, Erythropoietin (EPO), possesses both haematopoietic and tissue protective activities. The present disclosure provides that the latter functionality is mediated via interactions of EPO with a novel EPO receptor, termed NEPOR. The model provides that binding of EPO to NEPOR receptor complexes, on NEPOR positive cancer cells, confers survival advantage to such cells. The implicit physiological outcome for patients possessing NEPOR positive cancers is therefore increased loco-regional cancer progression and poorer overall survival.

Thus, the present disclosure provides a diagnostic or prognostic test that can predict whether or not cancer patients administered EPO will respond negatively in terms of survival outcome. The prognostic test comprises determining NEPOR (*i.e.* EPH-B4, and/or Ephrin A1) in tumour tissue, or more particularly cancer cells. In another embodiment NEPOR component gene expression levels in tumour cells can be compared to baseline levels or levels in surrounding normal cells or tissue. Therefore, a comparative analysis looking at elevated or normal baseline expression levels of NEPOR component expression, using standard gene expression analysis methods (such as q-PCR and DNA microarray analyses) provides a diagnostic test that can determine whether or not administration of EPO to cancer patients will unwittingly enhance tumour cell survival (a negative outcome).

As stated, one method that can be used for comparing levels of gene expression of components of NEPOR and/or EPH-B4, and/or Ephrin A1 is Quantitative polymerase chain reaction (qPCR). This is a modification of PCR or polymerase chain reaction used to rapidly measure the quantity of DNA present in a tissue sample. Like other forms of polymerase chain reaction, the process is used to amplify nucleic acid samples, via the temperature-mediated enzyme DNA polymerase. PCR amplifies DNA exponentially, doubling the number of molecules present with each amplification cycle. The number of amplification cycles and the amount of PCR end-product should allow one to calculate the initial quantity of NEPOR-specific genetic material and/or EPH-B4 and/or Ephrin A1 genetic material in particular mRNA molecules using NEPOR-specific component sequences in particular and/or EPH-B4, and/or Ephrin A1 sequences for the two primers used for amplification.

In addition, gene expression analysis of NEPOR components and/or EPH-B4, and/or Ephrin A1 can be done with a microarray analysis containing a plurality of capture probes specific for sequences of the NEPOR complex in particular and/or EPH-B4, and/or Ephrin A1. As EPO is proposed to stimulate survival of NEPOR positive cancer cells and/or EPH-B4, and/or Ephrin A1 positive cells, it is important to test all cancer patients for NEPOR status and/or and/or EPH-B4, and/or Ephrin A1 status prior to and during EPO administration. This is best done with a microarray analysis for expression status of NEPOR component genes in tumour tissue and with mRNA samples taken from tumour tissue. Ascertaining the levels of endogenous tumour associated NEPOR (*i.e.* EPH-B4, and/or EphrinA1) expression, provide correlations as to patient prognosis/survival rate.

The present disclosure thus provides a method to stratify patients having a tumour as suitable (*i.e.* NEPOR not present and/or EPH-B4, and/or Ephrin A1 present) or non-suitable (*i.e*., NEPOR present and/or and/or EPH-B4, and/or Ephrin A1 present) for EPO treatment. The method disclosed comprises: (a) isolating a tissue sample from an individual who is receiving or shall receive erythropoietin, (b) determining the level of expression of EPH-B4 and/or Ephrin A1 from the isolated tissue, and (c) correlating the presence of these component gene expression products to a negative physiological response to the treatment with erythropoietin.
SEQ ID NO.5
   >erythropoietin receptor (EPOR), mRNA 
ACTTAGAGGCGCCTGGTCGGAAGGGCCTGGTCAGCTGCGTCCGGCGGAGGCAGCTGCTGACCCAGCTGT
GGACTGTGCCGGGGGCGGGGACGGAGGGCAGGAGCCCTGGGCTCCCCGTGGCGGGGGCTGTATCATGG
ACCACCTCGGGGCGTCCCTCTGGCCCCAGGTCGGCTCCCTTTGTCTCCTGCTCGCTGGGGCCGCCTGGGC
GCCCCCGCCTAACCTCCCGGACCCCAAGTTCGAGAGCAAAGCGGCCTTGCTGGCGGCCCGGGGGCCCGAA
GAGCTTCTGTGCTTCACCGAGCGGTTGGAGGACTTGGTGTGTTTCTGGGAGGAAGCGGCGAGCGCTGGGG
TGGGCCCGGGCAACTACAGCTTCTCCTACCAGCTCGAGGATGAGCCATGGAAGCTGTGTCGCCTGCACCA
GGCTCCCACGGCTCGTGGTGCGGTGCGCTTCTGGTGTTCGCTGCCTACAGCCGACACGTCGAGCTTCGTG
CCCCTAGAGTTGCGCGTCACAGCAGCCTCCGGCGCTCCGCGATATCACCGTGTCATCCACATCAATGAAG
TAGTGCTCCTAGACGCCCCCGTGGGGCTGGTGGCGCGGTTGGCTGACGAGAGCGGCCACGTAGTGTTGCG
CTGGCTCCCGCCGCCTGAGACACCCATGACGTCTCACATCCGCTACGAGGTGGACGTCTCGGCCGGCAAC
GGCGCAGGGAGCGTACAGAGGGTGGAGATCCTGGAGGGCCGCACCGAGTGTGTGCTGAGCAACCTGCGGG
GCCGGACGCGCTACACCTTCGCCGTCCGCGCGCGTATGGCTGAGCCGAGCTTCGGCGGCTTCTGGAGCGC
CTGGTCGGAGCCTGTGTCGCTGCTGACGCCTAGCGACCTGGACCCCCTCATCCTGACGCTCTCCCTCATC
CTCGTGGTCATCCTGGTGCTGCTGACCGTGCTCGCGCTGCTCTCCCACCGCCGGGCTCTGAAGCAGAAGA
TCTGGCCTGGCATCCCGAGCCCAGAGAGCGAGTTTGAAGGCCTCTTCACCACCCACAAGGGTAACTTCCA
GCTGTGGCTGTACCAGAATGATGGCTGCCTGTGGTGGAGCCCCTGCACCCCCTTCACGGAGGACCCACCT
GCTTCCCTGGAAGTCCTCTCAGAGCGCTGCTGGGGGACGATGCAGGCAGTGGAGCCGGGGACAGATGATG
AGGGCCCCCTGCTGGAGCCAGTGGGCAGTGAGCATGCCCAGGATACCTATCTGGTGCTGGACAAATGGTT
GCTGCCCCGGAACCCGCCCAGTGAGGACCTCCCAGGGCCTGGTGGCAGTGTGGACATAGTGGCCATGGAT
GAAGGCTCAGAAGCATCCTCCTGCTCATCTGCTTTGGCCTCGAAGCCCAGCCCAGAGGGAGCCTCTGCTG
CCAGCTTTGAGTACACTATCCTGGACCCCAGCTCCCAGCTCTTGCGTCCATGGACACTGTGCCCTGAGCT
GCCCCCTACCCCACCCCACCTAAAGTACCTGTACCTTGTGGTATCTGACTCTGGCATCTCAACTGACTAC
AGCTCAGGGGACTCCCAGGGAGCCCAAGGGGGCTTATCCGATGGCCCCTACTCCAACCCTTATGAGAACA
GCCTTATCCCAGCCGCTGAGCCTCTGCCCCCCAGCTATGTGGCTTGCTCTTAGGACACCAGGCTGCAGAT
GATCAGGGATCCAATATGACTCAGAGAACCAGTGCAGACTCAAGACTTATGGAACAGGGATGGCGAGGCC
TCTCTCAGGAGCAGGGGCATTGCTGATTTTGTCTGCCCAATCCATCCTGCTCAGGAAACCACAACCTTGC
AGTATTTTTAAATATGTATAGTTTTTTTG
SEQ ID NO.6
   >EPH receptor B4 (EPHB4), mRNA 
TTCCAGCGCAGCTCAGCCCCTGCCCGGCCCGGCCCGCCCGGCTCCGCGCCGCAGTCTCCCTCCCTCCCGC
TCCGTCCCCGCTCGGGCTCCCACCATCCCCGCCCGCGAGGAGAGCACTCGGCCCGGCGGCGCGAGCAGAG
CCACTCCAGGGAGGGGGGGAGACCGCGAGCGGCCGGCTCAGCCCCCGCCACCCGGGGCGGACCCCGAGG
CCCCGGAGGGACCCCAACTCCAGCCACGTCTTGCTGCGCGCCCGCCCGGCGCGGCCACTGCCAGCACGCT
CCGGGCCCGCCGCCCGCGCGCGCGGCACAGACGCGGGGCCACACTTGGCGCCGCCGCCCGGTGCCCCGCA
CGCTCGCATGGGCCCGCGCTGAGGGCCCCGACGAGGAGTCCCGCGCGGAGTATCGGCGTCCACCCGCCCA
GGGAGAGTCAGACCTGGGGGGGCGAGGGCCCCCCAAACTCAGTTCGGATCCTACCCGAGTGAGGCGGCGC
CATGGAGCTCCGGGTGCTGCTCTGCTGGGCTTCGTTGGCCGCAGCTTTGGAAGAGACCCTGCTGAACACA
AAATTGGAAACTGCTGATCTGAAGTGGGTGACATTCCCTCAGGTGGACGGGCAGTGGGAGGAACTGAGCG
GCCTGGATGAGGAACAGCACAGCGTGCGCACCTACGAAGTGTGTGACGTGCAGCGTGCCCCGGGCCAGGC
CCACTGGCTTCGCACAGGTTGGGTCCCACGGCGGGGCGCCGTCCACGTGTACGCCACGCTGCGCTTCACC
ATGCTCGAGTGCCTGTCCCTGCCTCGGGCTGGGCGCTCCTGCAAGGAGACCTTCACCGTCTTCTACTATG
AGAGCGATGCGGACACGGCCACGGCCCTCACGCCAGCCTGGATGGAGAACCCCTACATCAAGGTGGACAC
GGTGGCCGCGGAGCATCTCACCCGGAAGCGCCCTGGGGCCGAGGCCACCGGGAAGGTGAATGTCAAGACG
CTGCGTCTGGGACCGCTCAGCAAGGCTGGCTTCTACCTGGCCTTCCAGGACCAGGGTGCCTGCATGGCCC
TGCTATCCCTGCACCTCTTCTACAAAAAGTGCGCCCAGCTGACTGTGAACCTGACTCGATTCCCGGAGAC
TGTGCCTCGGGAGCTGGTTGTGCCCGTGGCCGGTAGCTGCGTGGTGGATGCCGTCCCCGCCCCTGGCCCC
AGCCCCAGCCTCTACTGCCGTGAGGATGGCCAGTGGGCCGAACAGCCGGTCACGGGCTGCAGCTGTGCTC
CGGGGTTCGAGGCAGCTGAGGGGAACACCAAGTGCCGAGCCTGTGCCCAGGGCACCTTCAAGCCCCTGTC
AGGAGAAGGGTCCTGCCAGCCATGCCCAGCCAATAGCCACTCTAACACCATTGGATCAGCCGTCTGCCAG
TGCCGCGTCGGGTACTTCCGGGCACGCACAGACCCCCGGGGTGCACCCTGCACCACCCCTCCTTCGGCTC
CGCGGAGCGTGGTTTCCCGCCTGAACGGCTCCTCCCTGCACCTGGAATGGAGTGCCCCCCTGGAGTCTGG
TGGCCGAGAGGACCTCACCTACGCCCTCCGCTGCCGGGAGTGCCGACCCGGAGGCTCCTGTGCGCCCTGC
 
GGGGGAGACCTGACTTTTGACCCGGCCCCCGGGACCTGGTGGAGCCCTGGGTGGTGGTTCGAGGGCTAC
GTCCTGACTTCACCTATACCTTTGAGGTCACTGCATTGAACGGGGTATCCTCCTTAGCCACGGGGCCCGT
CCCATTTGAGCCTGTCAATGTCACCACTGACCGAGAGGTACCTCCTGCAGTGTCTGACATCCGGGTGACG
CGGTCCTCACCCAGCAGCTTGAGCCTGGCCTGGGCTGTTCCCCGGGCACCCAGTGGGGCTGTGCTGGACT
ACGAGGTCAAATACCATGAGAAGGGCGCCGAGGGTCCCAGCAGCGTGCGGTTCCTGAAGACGTCAGAAAA
CCGGGCAGAGCTGCGGGGGCTGAAGCGGGGAGCCAGCTACCTGGTGCAGGTACGGGCGCGCTCTGAGGCC
GGCTACGGGCCCTTCGGCCAGGAACATCACAGCCAGACCCAACTGGATGAGAGCGAGGGCTGGCGGGAGC
AGCTGGCCCTGATTGCGGGCACGGCAGTCGTGGGTGTGGTCCTGGTCCTGGTGGTCATTGTGGTCGCAGT
TCTCTGCCTCAGGAAGCAGAGCAATGGAGAGAAGCAGAATATTCGGACAAACACGGACAGTATCTCATC
GGACATGGTACTAAGGTCTACATCGACCCCTTCACTTATGAAGACCCTAATGAGGCTGTGAGGGAATTTG
CAAAAGAGATCGATGTCTCCTACGTCAAGATTGAAGAGGTGATTGGTGCAGGTGAGTTTGGCGAGGTGTG
CCGGGGGCGGCTCAAGGCCCCAGGGAAGAAGGAGAGCTGTGTGGCAATCAAGACCCTGAAGGGTGGCTAC
ACGGAGCGGCAGCGGCGTGAGTTTCTGAGCGAGGCCTCCATCATGGGCCAGTTCGAGCACCCCAATATCA
TCCGCCTGGAGGGCGTGGTCACCAACAGCATGCCCGTCATGATTCTCACAGAGTTCATGGAGAACGGCGC
CCTGGACTCCTTCCTGCGGCTAAACGACGGACAGTTCACAGTCATCCAGCTCGTGGGCATGCTGCGGGGC
ATCGCCTCGGGCATGCGGTACCTTGCCGAGATGAGCTACGTCCACCGAGACCTGGCTGCTCGCAACATCC
TAGTCAACAGCAACCTCGTCTGCAAAGTGTCTGACTTTGGCCTTTCCCGATTCCTGGAGGAGAACTCTTC
CGATCCCACCTACACGAGCTCCCTGGGAGGAAAGATTCCCATCCGATGGACTGCCCCGGAGGCCATTGCC
TTCCGGAAGTTCACTTCCGCCAGTGATGCCTGGAGTTACGGGATTGTGATGTGGGAGGTGATGTCATTTG
GGGAGAGGCCGTACTGGGACATGAGCAATCAGGACGTGATCAATGCCATTGAACAGGACTACCGGCTGCC
CCCGCCCCCAGACTGTCCCACCTCCCTCCACCAGCTCATGCTGGACTGTTGGCAGAAAGACCGGAATGCC
CGGCCCCGCTTCCCCCAGGTGGTCAGCGCCCTGGACAAGATGATCCGGAACCCCGCCAGCCTCAAAATCG
TGGCCCGGGAGAATGGCGGGGCCTCACACCCTCTCCTGGACCAGCGGCAGCCTCACTACTCAGCTTTTGG
CTCTGTGGGCGAGTGGCTTCGGGCCATCAAAATGGGAAGATACGAAGAAAGTTTCGCAGCCGCTGGCTTT
GGCTCCTTCGAGCTGGTCAGCCAGATCTCTGCTGAGGACCTGCTCCGAATCGGAGTCACTCTGGCGGGAC
ACCAGAAGAAAATCTTGGCCAGTGTCCAGCACATGAAGTCCCAGGCCAAGCCGGGAACCCCGGGTGGGAC
AGGAGGACCGGCCCCGCAGTACTGACCTGCAGGAACTCCCCACCCCAGGGACACCGCCTCCCCATTTTCC
GGGGCAGAGTGGGGACTCACAGAGGCCCCCAGCCCTGTGCCCCGCTGGATTGCACTTTGAGCCCGTGGGG
TGAGGAGTTGGCAATTTGGAGAGACAGGATTTGGGGGTTCTGCCATAATAGGAGGGGAAAATCACCCCCC
AGCCACCTCGGGGAACTCCAGACCAAGGGTGAGGGCGCCTTTCCCTCAGGACTGGGTGTGACCAGAGGAA
AAGGAAGTGCCCAACATCTCCCAGCCTCCCCAGGTGCCCCCCTCACCTTGATGGGTGCGTTCCCGCAGAC
CAAAGAGAGTGTGACTCCCTTGCCAGCTCCAGAGTGGGGGGGCTGTCCCAGGGGGCAAGAAGGGGTGTCA
GGGCCCAGTGACAAAATCATTGGGGTTTGTAGTCCCAACTTGCTGCTGTCACCACCAAACTCAATCATTT
TTTTCCCTTGTAAATGCCCCTCCCCCAGCTGCTGCCTTCATATTGAAGGTTTTTGAGTTTTGTTTTTGGT
CTTAATTTTTCTCCCCGTTCCCTTTTTGTTTCTTCGTTTTGTTTTTCTACCGTCCTTGTCATAACTTTGT
GTTGGAGGGAACCTGTTTCACTATGGCCTCCTTTGCCCAAGTTGAAACAGGGGCCCATCATCATGTCTGT
TTCCAGAACAGTGCCTTGGTCATCCCACATCCCCGGACCCCGCCTGGGACCCCCAAGCTGTGTCCTATGA
AGGGGTGTGGGGTGAGGTAGTGAAAAGGGCGGTAGTTGGTGGTGGAACCCAGAAACGGACGCCGGTGCTT
GGAGGGGTTCTTAAATTATATTTAAAAAAGTAACTTTTTGTATAAATAAAAGAAAATGGACGTGTCCCA
GCTCCAGGGGTAAAAAAAAAAAAAAAAAA
SEQ ID NO.7
   >Ephrin-A1 (EFNA1) mRNA 
GCCAGATCTGTGAGCCCAGCGCTGACTGCCGCGGAGAAAGCCAGTGGGAACCCAGACCCATAGGAGAC
CCGCGTCCCCGCTCGGCCTGGCCAGGCCCCGCGCTATGGAGTTCCTCTGGGCCCCTCTCTTGGGTCTGTG
CTGCAGTCTGGCCGCTGCTGATCGCCACACCGTCTTCTGGAACAGTTCAAATCCCAAGTTCCGGAATGAG
GACTACACCATACATGTGCAGCTGAATGACTACGTGGACATCATCTGTCCGCACTATGAAGATCACTCTG
TGGCAGACGCTGCCATGGAGCAGTACATACTGTACCTGGTGGAGCATGAGGAGTACCAGCTGTGCCAGCC
CCAGTCCAAGGACCAAGTCCGCTGGCAGTGCAACCGGCCCAGTGCCAAGCATGGCCCGGAGAAGCTGTCT
GAGAAGTTCCAGCGCTTCACACCTTTCACCCTGGGCAAGGAGTTCAAAGAAGGACACAGCTACTACTACA
TCTCCAAACCCATCCACCAGCATGAAGACCGCTGCTTGAGGTTGAAGGTGACTGTCAGTGGCAAAATCAC
TCACAGTCCTCAGGCCCATGACAATCCACAGGAGAAGAGACTTGCAGCAGATGACCCAGAGGTGCGGGTT
CTACATAGCATCGGTCACAGTGCTGCCCCACGCCTCTTCCCACTTGCCTGGACTGTGCTGCTCCTTCCAC
TTCTGCTGCTGCAAACCCCGTGAAGGTGTATGCCACACCTGGCCTTAAAGAGGGACAGGCTGAAGAGAGG
GACAGGCACTCCAAACCTGTCTTGGGGCCACTTTCAGAGCCCCCAGCCCTGGGAACCACTCCCACCACAG
GCATAAGCTATCACCTAGCAGCCTCAAAACGGGTCAGTATTAAGGTTTTCAACCGGAAGGAGGCCAACCA
GCCCGACAGTGCCATCCCCACCTTCACCTCGGAGGGATGGAGAAAGAAGTGGAGACAGTCCTTTCCCACC
ATTCCTGCCTTTAAGCCAAAGAAACAAGCTGTGCAGGCATGGTCCCTTAAGGCACAGTGGGAGCTGAGCT
GGAAGGGGCCACGTGGATGGGCAAAGCTTGTCAAAGATGCCCCCTCCAGGAGAGAGCCAGGATGCCCAGA
TGAACTGACTGAAGGAAAAGCAAGAAACAGTTTCTTGCTTGGAAGCCAGGTACAGGAGAGGCAGCATGCT
TGGGCTGACCCAGCATCTCCCAGCAAGACCTCATCTGTGGAGCTGCCACAGAGAAGTTTGTAGCCAGGTA
CTGCATTCTCTCCCATCCTGGGGCAGCACTCCCCAGAGCTGTGCCAGCAGGGGGCTGTGCCAACCTGTT
 
CTTAGAGTGTAGCTGTAAGGGCAGTGCCCATGTGTACATTCTGCCTAGAGTGTAGCCTAAAGGGCAGGGC
CCACGTGTATAGTATCTGTATATAAGTTGCTGTGTGTCTGTCCTGATTTCTACAACTGGAGTTTTTTTAT
ACAATGTTCTTTGTCTCAAAATAAAGCAATGTGTTTTTTCGGACATGCTTTTCTGCCACTCCATATTAAA
ACATATGACCATTGAGTCCCTGCTAAAAAAAAAAAAAAAAAAAAAAA
SEQ ID NO.8
   >ephrin-B2 (EFNB2), mRNA

GCGCGGAGCTGGGAGTGGCTTCGCCATGGCTGTGAGAAGGACTCCGTGTGGAAGTACTGCTGGGGTGTT
TTGATGGTTTTATGCAGAACTGCGATTTCCAAATCGATAGTTTTAGAGCCTATCTATTGGAATTCCTCGA
ACTCCAAATTTCTACCTGGACAAGGACTGGTACTATACCCACAGATAGGAGACAAATTGGATATTATTTG
CCCCAAAGTGGACTCTAAAACTGTTGGCCAGTATGAATATTATAAAGTTTATATGGTTGATAAAGACCAA
GCAGACAGATGCACTATTAAGAAGGAAAATACCCCTCTCCTCAACTGTGCCAAACCAGACCAAGATATCA
AATTCACCATCAAGTTTCAAGAATTCAGCCCTAACCTCTGGGGTCTAGAATTTCAGAAGAACAAAGATTA
TTACATTATATCTACATCAAATGGGTCTTTGGAGGGCCTGGATAACCAGGAGGGAGGGGTGTGCCAGACA
AGAGCCATGAAGATCCTCATGAAAGTTGGACAAGATGCAAGTTCTGCTGGATCAACCAGGAATAAAGATC
CAACAAGACGTCCAGAACTAGAAGCTGGTACAAATGGAAGAAGTTCGACAACAAGTCCCTTTGTAAAACC
AAATCCAGGTTCTAGCACAGACGGCAACAGCGCCGGACATTCGGGGAACAACATCCTCGGTTCCGAAGTG
GCCTTATTTGCAGGGATTGCTTCAGGATGCATCATCTTCATCGTCATCATCATCACGCTGGTGGTCCTCT
TGCTGAAGTACCGGAGGAGACACAGGAAGCACTCGCCGCAGCACACGACCACGCTGTCGCTCAGCACACT
GGCCACACCCAAGCGCAGCGGCAACAACAACGGCTCAGAGCCCAGTGACATTATCATCCCGCTAAGGACT
GCGGACAGCGTCTTCTGCCCTCACTACGAGAAGGTCAGCGGGGACTACGGGCACCCGGTGTACATCGTCC
AGGAGATGCCCCCGCAGAGCCCGGCGAACATTTACTACAAGGTCTGAGAGGGACCCTGGTGGTACCTGTG
CTTTCCCAGAGGACACCTAATGTCCCGATGCCTCCCTTGAGGGTTTGAGAGCCCGCGTGCTGGAGAATTG
ACTGAAGCACAGCACCGGGGGAGAGGGACACTCCTCCTCGGAAGAGCCCGTCGCGCTGGACAGCTTACCT
AGTCTTGTAGCATTCGGCCTTGGTGAACACACACGCTCCCTGGAAGCTGGAAGACTGTGCAGAAGACGCC
CATTCGGACTGCTGTGCCGCGTCCCACGTCTCCTCCTCGAAGCCATGTGCTGCGGTCACTCAGGCCTCTG
CAGAAGCCAAGGGAAGACAGTGGTTTGTGGACGAGAGGGCTGTGAGCATCCTGGCAGGTGCCCCAGGATG
CCACGCCTGGAAGGGCCGGCTTCTGCCTGGGGTGCATTTCCCCCGCAGTGCATACCGGACTTGTCACACG
GACCTCGGGCTAGTTAAGGTGTGCAAAGATCTCTAGAGTTTAGTCCTTACTGTCTCACTCGTTCTGTTAC
CCAGGGCTCTGCAGCACCTCACCTGAGACCTCCACTCCACATCTGCATCACTCATGGAACACTCATGTCT
GGAGTCCCCTCCTCCAGCCGCTGGCAACAACAGCTTCAGTCCATGGGTAATCCGTTCATAGAAATTGTGT
TTGCTAACAAGGTGCCCTTTAGCCAGATGCTAGGCTGTCTGCGAAGAAGGCTAGGAGTTCATAGAAGGGA
GTGGGGCTGGGGAAAGGGCTGGCTGCAATTGCAGCTCACTGCTGCCTCTGAAACAGAAAGTTGGAAA
GGAAAAAAGAAAAAAGCAATTAGGTAGCACAGCACTTTGGTTTTGCTGAGATCGAAGAGGCCAGTAGGAG
ACACGACAGCACACACAGTGGATTCCAGTGCATGGGAGGCACTCGCTGTTATCAAATAGCGATGTGCAG
GAAGAAAAGCCCCTCTTCATTCCGGGGAACAAAGACGGGTATTGTTGGGAAAGGAACAGGCTTGGAGGGA
AGGGAGAAAGTAGGCCGCTGATGATATATTCGGGCAGGACTGTTGTGGTACTGGCAATAAGATACACAGC
TCCGAGCTGTAGGAGAGTCGGTCTGCTTTGGATGATTTTTAAGCAGACTCAGCTGCTATACTTATCACA
TTTTATTAAACACAGGGAAAGCATTTAGGAGAATAGCAGAGAGCCAAATCTGACCTAAAAGTTGAAAAGC
CAAAGGTCAAACAGGCTGTAATTCCATCATCATCGTTGTTATTAAAGAATCCTTATCTATAAAAGGTAGG
TCAGATCCCCCTCCCCCAGGTTCCTCCTTCCCCTCCCGATTGAGCCTTACGACACTTTGGTTTATGCGG
TGCTGTCCGGGTGCCAGGGCTGCAGGGTCGGTACTGATGGAGGCTGCAGCGCCCGGTGCTCTGTGTCAAG
GTGAAGCACATACGGCAGACCTCTTAGAGTCCTTAAGACGGAAGTAAATTATGATGTCCAGGGGGAGAAG
GAAGATAGGACGTATTTATAATAGGTATATAGAACACAAGGGATATAAAATGAAAGATTTTTACTAATAT
ATATTTTAAGGTTGCACACAGTACACACCAGAAGATGTGAAATTCATTTGTGGCAATTAAGTGGTCCCAA
TGCTCAGCGCTTAAAAAAACAAATTGGACAGCTACTTCTGGGAAAAACAACATCATTCCAAAAAGAACAA
TAATGAGAGCAAATGCAAAAATAACCAAGTCCTCCGAAGGCATCTCACGGAACCGTAGACTAGGAAGTAC
GAGCCCCACAGAGCAGGAAGCCGATGTGACTGCATCATATATTTAACAATGACAAGATGTTCCGGCGTTT
ATTTCTGCGTTGGGTTTTCCCTTGCCTTATGGGCTGAAGTGTTCTCTAGAATCCAGCAGGTCACACTGGG
GGCTTCAGGTGACGATTTAGCTGTGGCTCCCTCCTCCTGTCCTCCCCCGCACCCCCTCCCTTCTGGGAAA
CAAGAAGAGTAAACAGGAAACCTACTTTTTATGTGCTATGCAAAATAGACATCTTTAACATAGTCCTGTT
ACTATGGTAACACTTTGCTTTCTGAATTGGAAGGGAAAAAAAATGTAGCGACAGCATTTTAAGGTTCTCA
GACCTCCAGTGAGTACCTGCAAAAATGAGTTGTCACAGAAATTATGATCCTCTATTTCCTGAACCTGGAA
ATGATGTTGGTCCAAAGTGCGTGTGTGTATGTGTGAGTGGGTGCGTGGTATACATGTGTACATATATGTA
TAATATATATCTACAATATATATTATATATATCTATATCATATTTCTGTGGAGGGTTGCCATGGTAACCA
GCCACAGTACATATGTAATTCTTTCCATCACCCCAACCTCTCCTTTCTGTGCATTCATGCAAGAGTTTCT
TGTAAGCCATCAGAAGTTACTTTTAGGATGGGGGAGAGGGGCGAGAAGGGGAAAAATGGAAATAGTCTG
ATTTTAATGAAATCAAATGTATGTATCATCAGTTGGCTACGTTTTGGTTCTATGCTAAACTGTGAAAAAT
CAGATGAATTGATAAAAGAGTTCCCTGCAACCAATTGAAAAGTGTTCTGTGCGTCTGTTTTGTGTCTGGT
GCAGAATATGACAATCTACCAACTGTCCCTTTGTTTGAAGTTGGTTTAGCTTTGGAAAGTTACTGTAAAT
GCCTTGCTTGTATGATCGTCCCTGGTCACCCGACTTTGGAATTTGCACCATCATGTTTCAGTGAAGATGC
TGTAAATAGGTTCAGATTTTACTGTCTATGGATTTGGGGTGTTACAGTAGCCTTATTCACCTTTTTAATA
AAAATACACATGAAAACAAGAAAGAAATGGCTTTTCTTACCCAGATTGTGTACATAGAGCAATGTTGGTT

TTTTATAAAGTCTAAGCAAGATGTTTTGTATAAAATCTGAATTTTGCAATGTATTTAGCTACAGCTTGTT
TAACGGCAGTGTCATTCCCCTTTGCACTGTAATGAGGAAAAAATGGTATAAAAGGTTGCCAAATTGCTGC
ATATTTGTGCCGTAATTATGTACCATGAATATTTATTTAAAATTTCGTTGTCCAATTTGTAAGTAACACA
GTATTATGCCTGAGTTATAAATATTTTTTTCTTTCTTTGTTTTATTTTAATAGCCTGTCATAGGTTTTAA
ATCTGCTTTAGTTTCACATTGCAGTTAGCCCCAGAAAATGAAATCCGTGAAGTCACATTCCACATCTGTT
TCAAACTGAATTTGTTCTTAAAAAAATAAAATATTTTTTTCCTATGGAAAAAAAAAAAAAAAAA

Detection of NEPOR component mRNA (SEQ ID NOs 5-8) should preferentially be performed using probes complementary to the sub-region of SEQ ID NO's 5-8, encoding the EPO binding domain and is particular SEQ Id NO. 6 and/or 7 encoding EPH-B4 and Ephrin A1. This implies for EPH-B4, probes complementary to SEQ ID NO. 10.; for Ephrin A1, probes complementary to SEQ ID NO. 11.
SEQ ID NO.9
   >epor_epobinding_coding region 
AGCAAAGCGGCCTTGCTGGCGGCCCGGGGCCCGAAGAGCTTCTGTGCTTCACCGAGCGGTTGGAGGACTTGGTGTG
TTTCTGGGAGGAAGCGGCGAGCGCTGGGGTGGGCCCGGGCAACTACAGCTTCTCCTACCAGCTCGAGGATGAGCCAT
GGAAGCTGTGTCGCCTGCACCAGGCTCCCACGGCTCGTGGTGCGGTGCGCTTCTGGTGTTCGCTGCCTACAGCCGAC
ACGTCGAGCTTCGTGCCCCTAGAGTTGCGCGTCACAGCAGCCTCCGGCGCTCCGCGATATCACCGTGTCATCCACAT
CAATGAAGTAGTGCTCCTAGACGCCCCCGTGGGGCTGGTGGCGCGGTTGGCTGACGAGAGCGGCCACGTAGTGTTGC
GCTGGCTCCCGCCGCCTGAGACACCCATGACGTCTCACATCCGCTACGAGGTGGACGTCTCGGCCGGCAACGGCGCA
GGGAGCGTACAGAGGGTGGAGATCCTGGAGGGCCGCACCGAGTGTGTGCTGAGCAACCTGCGGGGCCGGACGCGCTA
CACCTTCGCCGTCCGCGCGCGTATGGCTGAGCCGAGCTTCGGCGGCTTCTGGAGCGCCTGGTCGGAGCCTGTGTCGC
TGCTGACGCCTAGCGACCTGGACCCC
SEQ ID NO.10
   >ephb4_epobinding_coding region 
CCTTCGGCTCCGCGGAGCGTGGTTTCCCGCCTGAACGGCTCCTCCCTGCACCTGGAATGGAGTGCCCCCCTGGAGTC
TGGTGGCCGAGAGGACCTCACCTACGCCCTCCGCTGCCGGGAGTGCCGACCCGGAGGCTCCTGTGCGCCCTGC
GGGGGAGACCTGACTTTTGACCCCGGCCCCCGGGACCTGGTGGAGCCCTGGGTGGTGGTTCGAGGGCTACGTCCTGA
CTTCACCTATACCTTTGAGGTCACTGCATTGAACGGGGTATCCTCCTTAGCCACGGGGCCCGTCCCATTTGAGCCTG
TCAATGTCACCACTGACCGAGAGGTACCTCCTGCAGTGTCTGACATCCGGGTGACGCGGTCCTCACCCAGCAGCTTG
AGCCTGGCCTGGGCTGTTCCCCGGGCACCCAGTGGGGCTGTGCTGGACTACGAGGTCAAATACCATGAGAAGGGCGC
CGAGGGTCCCAGCAGCGTGCGGTTCCTGAAGACGTCAGAAAACCGGGCAGAGCTGCGGGGGCTGAAGCGGGGAGCCA
GCTACCTGGTGCAGGTACGGGCGCGCTCTGAGGCCGGCTACGGGCCCTTCGGCCAGGAACATCACAGCCAGACCCAA
CTGGATGAGAGCGAGGGCTGGCGGGAGCAGCTGGCCCTG
SEQ ID NO.11
   >ephrinA1_epobinding_coding region 
CTGGCCGCTGCTGATCGCCACACCGTCTTCTGGAACAGTTCAAATCCCAAGTTCCGGAATGAGGACTACACCATACA
TGTGCAGCTGAATGACTACGTGGACATCATCTGTCCGCACTATGAAGATCACTCTGTGGCAGACGCTGCCATGGAGC
AGTACATACTGTACCTGGTGGAGCATGAGGAGTACCAGCTGTGCCAGCCCCAGTCCAAGGACCAAGTCCGCTGGCAG
TGCAACCGGCCCAGTGCCAAGCATGGCCCGGAGAAGCTGTCTGAGAAGTTCCAGCGCTTCACACCTTTCACCCTGGG
CAAGGAGTTCAAAGAAGGACACAGCTACTACTACATCTCCAAACCCATCCACCAGCATGAAGACCGCTGCTTGAGGT
TGAAGGTGACTGTCAGTGGCAAAATCACTCAC
SEQ ID NO.12
   >ephrinb2_epobinding_coding region

TCCAAATCGATAGTTTAGAGCCTATCTATTGGAATTCCTCGAACTCCAAATTTCTACCTGGACAAGGACTGGTACT
ATACCCACAGATAGGAGACAAATTGGATATTATTTGCCCCAAAGTGGACTCTAAAACTGTTGGCCAGTATGAATATT
ATAAAGTTTATATGGTTGATAAAGACCAAGCAGACAGATGCACTATTAAGAAGGAAAATACCCCTCTCCTCAACTGT
GCCAAACCAGACCAAGATATCAAATTCACCATCAAGTTTCAAGAATTCAGCCCTAACCTCTGGGGTCTAGAATTTCA
GAAGAACAAAGATTATTACATTATATCTACATCAAATGGGTCTTTGGAGGGCCTGGATAACCAGGAGGGAGGGGTGT
GCCAGACAAGAGCCATGAAGATCCTCATGAAAGTTGGACAA

The determination of the presence of the Ephrin A1 and/or the determination of the presence of the EPH-B4 gene product (mRNA) may be done by using a hybridization technique or an amplification technique. It is preferred that the technique is selected from the group of, real-time-PCR, northern-blot analysis, reverse transcription and amplification, zymography, ligase-chain-reaction, NASBA, RNase Protection Assay (RPA), capillary electrophoresis with laser induced fluorescence (CE-LIF) and combinations thereof.

Specifically, the individual is a cancer patient who is to be treated with erythropoietin or is being treated with erythropoietin. Preferably, the negative physiological effect is poorer patient survival due to enhanced tumor progression. Preferably, the presence of a higher level of NEPOR component genes (mRNA) and/or the presence of NEPOR component gene expression products (proteins) and/or EPH-B4 and/or Ephrin A1 on tumor tissues is indicative of poorer survival prognosis upon treatment with erythropoietin.

Preferably, the determination of the presence of the NEPOR dimer complex is done by detecting the respective NEPOR proteins with an immunoassay. Also peptides thereof may be detected. The immunoassay is selected from the group of immunoprecipitation, a protein array or binding to a mass microbalance instrument (for example, Q-Sense or Attana), enzyme immunoassay (EIA), radioimmunoassay (RIA) or fluorescent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a luciferase-assay. Preferably, the immunoassay is an ELISA. Preferably, the anti-NEPOR antibody and/or EPH-B4 and/or Ephrin A1 antibody is a monoclonal or polyclonal antibody, for example selected from - or similar to - the antibodies listed in Table 6.

Preferably, detection of NEPOR component proteins should preferentially be performed using antibodies detecting the sub-regions of SEQ ID NOs 6 and 7, representing the EPO binding domain. This implies for EPH-B4, antibodies specific to SEQ ID NO. 14.; for Ephrin A1, antibodies specific to SEQ ID NO. 15.
SEQ ID NO.13
   >epor_epobinding_region 
SKAALLAARGPEELLCFTERLEDLVCFWEEAASAGVGPGNYSFSYQLEDEPWKLCRLHQAPTARGAVRFWCSLPTAD
TSSFVPLELRVTAASGAPRYHRVIHINEVVLLDAPVGLVARLADESGHVVLRWLPPPETPMTSHIRYEVDVSAGNGA
GSVQRVEILEGRTECVLSNLRGRTRYTFAVRARMAEPSFGGFWSAWSEPVSLLTPSDLDP
SEQ ID NO.14
   >ephb4_epobinding_region 
PSAPRSVVSRLNGSSLHLEWSAPLESGGREDLTYALRCRECRPGGSCAPCGGDLTFDPGPRDLVEPWVVVRGLRPDF
TYTFEVTALNGVSSLATGPVPFEPVNVTTDREVPPAVSDIRVTRSSPSSLSLAWAVPRAPSGAVLDYEVKYHEKGAE
GPSSVRFLKTSENRAELRGLKRGASYLVQVRARSEAGYGPFGQEHHSQTQLDESEGWREQLAL
SEQ ID NO.15
   >ephrinA1_epobinding_region 
LAAADRHTVFWNSSNPKFRNEDYTIHVQLNDYVDIICPHYEDHSVADAAMEQYILYLVEHEEYQLCQPQSKDQVRWQ
CNRPSAKHGPEKLSEKFQRFTPFTLGKEFKEGHSYYYISKPIHQHEDRCLRLKVTVSGKITH
SEQ ID NO.16
   >ephrinb2_epobinding_region

SKSIVLEPIYWNSSNSKFLPGQGLVLYPQIGDKLDIICPKVDSKTVGQYEYYKVYMVDKDQADRCTIKKENTPLLNC
AKPDQDIKFTIKFQEFSPNLWGLEFQKNKDYYIISTSNGSLEGLDNQEGGVCQTRAMKILMKVGQ

Preferably, the individual is a cancer patient who is to be treated with erythropoietin or is being treated with erythropoietin. The tissue sample may be selected from the group of biological tissues and fluids such as blood, lymph, urine, cerebral fluid. The tissue sample may also be a tumor biopsy sample. It is preferred that the tissue sample is from the cancer tissue or circulating cells derived from same.

It is preferred that the cancer of the cancer patient is selected from the group of, head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer. It is particularly preferred that the cancer is selected from the group of squamous cell carcinoma of the head and neck (HNSCC), prostate cancer, colorectal cancer, lung cancer, kidney cancer, brain cancer and bladder cancer.

### NEPOR and Disease Intervention and Therapy Design/Screening.

Without being bound by theory, NEPOR is proposed to mediate the cyto-protective effects of EPO and its variants. Thus, EPO and variants that have been shown to possess cyto-protective (but not haematopoietic) activity can affect NEPOR function. Therefore, the present disclosure provides knowledge of NEPOR's composition that can be used to optimize the structure and efficacy of such therapeutic molecules (that is, better manage the structure-activity relationship or SAR of the EPO pharmacophore). Moreover, the present disclosure provides knowledge of NEPOR's composition that can be used to identify novel NEPOR regulating compounds. For example, in diseases associated with hypoxic conditions (*e.g*., stroke, heart attack), NEPOR binding compounds of enhanced efficacy can be developed to mimic the effects of EPO on NEPOR. Similarly, NEPOR specific antagonists (such as those molecules that bind the active site of NEPOR yet do not transducer signal are antagonists of EPO function. Such EPO antagonist agents, when concomitantly administered with EPO, can allow for EPO effects to improve haematopoiesis (that is, treat the anaemia) yet prevent the side effect of promoting tumour cell growth, survival and angiogenesis in NEPOR positive cancers such as HNSCC. Moreover, contrasting the relative activity of compounds to the tissue protective NEPOR receptor complex in comparison to the EPOR receptor homodimer provides for generating NEPOR specific/directed therapies.

Definition of NEPOR provides methods for identifying therapeutic molecules that modulate NEPOR's tissue protective signalling activity. This comprises: (a) contacting a test compound with the NEPOR receptor complex and/or EPH-B4 and/or Ephrin A1 and an EPOR homodimer complex; (b) measuring and comparing the level of tissue protective activity initiated by NEPOR activation with the activation of EPOR homodimer signalling; (c) identifying a test compound which increases or decreases the level of tissue protective NEPOR complex activity as compared to the level of EPOR complex activation; and (d) assaying the identified therapeutics for tissue protective activity mediated via NEPOR, but lack of EPOR activation and (e) assaying the identified therapeutics for NEPOR inhibitory activity. The method is useful for identifying therapeutics that modulates the interaction between a tissue protective NEPOR complex and/or EPH-B4 and/or Ephrin A1 and the EPO ligand. The method is furthermore useful for identifying therapies for treating diseases of the central nervous system or peripheral nervous system which have primarily neurological or psychiatric symptoms, ophthalmic diseases, cardiovascular diseases, cardiopulmonary diseases, respiratory diseases, kidney, urinary and reproductive diseases, bone diseases, skin diseases, gastrointestinal diseases and endocrine and metabolic abnormalities and cancer.

More specifically, identification of NEPOR provides a method identifying (II) a compound that modulates the tissue protective activity of NEPOR, comprising:
(a) contacting a test compound with a tissue protective NEPOR receptor complex (N) and/or EPH-B4 and/or Ephrin A1 or tissue protective cytokine receptor complex-expressing cell; measuring the level of the activity of (N) in the cell; identifying a test compound that increases or decreases the level of activity of (N) as compared to the level of activity of (N) measured in the absence of the test compound; and assaying the identified test compound for tissue protective activity;
(b) contacting a test compound with a cell that is recombinantly engineered to express (N), where the cell or the recombinant cell is transformed with a nucleic acid comprising a nucleotide sequence that is functionally linked to a promoter and encodes EPH-B4 and/or Ephrin A1 polypeptides; measuring the level of activity of (N) in the cell; and
(c) contacting a test compound with a tissue protective NEPOR receptor complex-expressing cell, where the cell is transformed with a nucleic acid comprising a nucleotide sequence that encodes a reporter gene functionally linked to regulatory element associated with the activity of (N); identifying a test compound that increases or decreases the level of reporter gene expression relative to the level of reporter gene expression measured in the absence of the test compound; and assaying the identified test compound for a tissue protective activity.

The present disclosure further provides a method for identifying (I2) a compound that binds to (N), comprising:
(a) contacting (N) with a tissue protective NEPOR receptor complex ligand and/or EPH-B4 and/or Ephrin A1 ligànd attached to a first label, and an equivalent amount of a test compound attached to a second label under conditions conducive to binding, removing unbound material from (N), and detecting the level of the first and second labels, where if the second label is present the compound binds (N) and if the level of the first label decreases relative to the level of the first label when the labelled ligand is contacted with (N) under conditions conducive to binding in the absence of a test compound after removal of unbound material, then a compound that binds to (N) is identified; or
(b) contacting a test compound with a ligand-binding tissue protective receptor NEPOR complex fragment comprising at least one EPH-B4 receptor or Ephrin A1 receptor, extracellular domain fused to a Fc fragment attached to a solid support, removing unbound test compounds from the solid support, and identifying the compound attached to the tissue protective NEPOR receptor complex fragment, such that a compound bound to the solid support is identified as a compound that binds to a tissue protective NEPOR receptor complex; and identifying (I3) a compound that modulates the binding of a tissue protective NEPOR receptor complex ligand to (N), or compound that modulates the interaction between (N) and tissue protective cytokine receptor complex ligand, involves (i) contacting a tissue protective NEPOR receptor complex ligand with (N) in the presence of one or more test compounds under conditions conducive to binding, and measuring the amount of tissue protective cytokine receptor complex ligand bound to (N).

The present disclosure further provides novel tissue protective NEPOR receptor complexes in particular EPH-B4 and/or Ephrin A1 containing complexes that can be used to provide an *in vitro* screening assay for NEPOR specific therapies; by measuring the binding of test compounds to the tissue protective NEPOR receptor complex in comparison to EPOR homodimer complexes. The test compound is labelled and binding of the labelled test compound to the receptor complexes detailed in Figure 9 is measured by detecting the label attached to the test compound. Alternatively, a label free detection approach such as surface plasmon resonance may be employed. Such an approach can provide for novel neuroprotective therapies (*i.e.* NEPOR agonists) which lack haematopoietic activity. Such an approach can also provide for novel oncotherapies (*i.e.* NEPOR antagonists i.e. at least a and/or EPH-B4 and/or Ephrin A1 agonist) which do not inhibit haematopoiesis. The nature of such screening arrays involving recombinant receptor constructs is demonstrated in Figure 9 (in the exemplified case as Fc constructs).

### Use

(I1) is useful for identifying a compound that modulates NEPOR's tissue protective activity. (I2) is useful for identifying a compound that binds to NEPOR. (I3) is useful for identifying a compound that modulates the binding of a tissue protective NEPOR receptor complex ligand to (N), or compound that modulates the interaction between (N) and tissue protective cytokine receptor complex ligand (claimed). The compounds identified using (I1)-(I3) are useful for treating various conditions of the central and peripheral nervous systems (*e.g*., hypoxia, and/or ischemia, epilepsy, chronic seizure disorders, neurotoxin poisoning, septic shock, anaphylactic shock), neuropsychologic disorders (senile dementia, Alzheimer's disease, Parkinson's disease, dermentia, multiple sclerosis, Creutzfeldt-Jakob disease, Huntington's disease), inflammatory diseases (*e.g*., chronic bronchitis, rheumatoid arthritis, glomerulonephritis, encephalitis, meningitis, polymyositis), opthalamic diseases (*e.g*., angiitis, retinal ischemia), cardiovascular diseases (*e.g*., myocardial infraction, myocarditis), cardiopulmonary diseases (*e.g*., asthma, pulmonary thrombosis), respiratory diseases, kidney, urinary, and reproductive diseases (*e.g*., myasthenia gravis, diabetes, autoimmune diseases), bone diseases (*e.g*., osteopenia, Paget's disease), gastrointestinal diseases and endocrine and metabolic abnormalities.

The compounds identified using (I1)-(I3) are also useful for treating NEPOR positive cancers in particular and/or EPH-B4 and/or Ephrin A1 positive cancers including, head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer. It is particularly preferred that the cancer is selected from the group of squamous cell carcinoma of the head and neck (HNSCC), prostate cancer, colorectal cancer, lung cancer, kidney cancer, brain cancer and bladder cancer.

### NEPOR in Oncology Therapy

The hypothesis of the present disclosure is that EPO results in poorer survival outcomes (at least in some cancers) because of its effects on NEPOR activity i.e. in particular EPH-B4 and/or Ephrin A1 activity. Therefore, treatment of these NEPOR positive patients with a NEPOR targeted therapy is a prudent path to disease intervention. Specific approaches to antagonising NEPOR mediated survival signals include, for example:
a) NEPOR specific antagonistic antibodies. Such antibodies block and antagonise the extracellular regions of the molecule specifically associated with the mediation of NEPOR's cyto-protective activity.
b) NEPOR specific small-molecules. Such small molecules block and antagonise the extracellular regions of the molecule specifically associated with the mediation of NEPOR's cytoprotective activity.
c) high-affinity peptides which specifically target NEPOR to block and antagonise the mediation of EPO's cytoprotective activity.
d) Small molecules targeting EPH-B4's intracellular tyrosine kinase domain (*e.g.* Dasatinib), including:
   1: CID: 1095868, AKI-STT-00166305; ZINC00818264; BAS 09636496 IUPAC: N-[5-[(3-chlorophenyl)methyl]-1,3-thiazol-2-yl]-2-(4,6-dimethylpyrimidin-2-yl)sulfanylacetamide. MW: 404.93678 | MF: C18H17ClN4OS2. (MW is molecular weight and MF is molecular formula)
   2: CID: 1465558, IUPAC: 2-[(3-chlorobenzoyl)amino]-4-methyl-N-pyridin-3-yl-1,3-thiazole-5-carboxamide, MW: 372.82872 | MF: C17H13ClN4O2S.
   3: CID: 1468201, IUPAC: N-[5-[(2-chlorophenyl)carbamoyl]-4-methyl-1,3-thiazol-2-yl]pyridine-4-carboxamide, MW: 372.82872 | MF: C17H13ClN4O2S.
   4: CID: 3062316, Dasatinib; Sprycel; BMS Dasatinib, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 488.00554 | MF: C22H26ClN7O2S.
   5: CID: 3072360, 142287-40-9; Pyrimido(4,5-d)pyrimidin-4(1H)-one, 7-methyl-1-phenyl-2-((3-(4-(2-thiazolyl)-1-piperazinyl)propyl)thio)- IUPAC: 2-methyl-8-phenyl-7-[3-[4-(1,3-thiazol-2-yl)piperazin-1-yl]propylsulfanyl]pyrimido[6,5-d]pyrimidin-5-one, MW: 479.6209 | MF: C23H25N7OS2.
   6: CID: 5041467, STK154706; ZINC04687922, IUPAC: [2-[(2-methylghenyl)amino]-1,3-thiazol-4-yl]-(4-pyrimidin-2-ylpiperazin-1-yl)methanane, MW: 380.4667 | MF: C19H20N60S.
   7: CID: 9822929, IUPAC: N-(2-chloro-6-methylphenyl)-2-[(6-imidazol-1-ylpyridazin-3-yl)amino]-1,3-thiazole-5-carboxamide, MW: 411.869 | MF: C18H14ClN7OS.
   8: CID: 9927718, IUPAC: N-(2-chloro-6-methylphenyl)-2-(cyclopropanecarbonylamino)-1,3-thiazole-5-carboxamide, MW: 335.809 | MF: C15H14ClN3O2S.
   9: CID: 10006113, IUPAC: N-[4-chloro-2-[(5-chloropyridin-2-yl)carbamoyl]phenyl]-5-methyl-6,7-dihydro-4H-[1,3]thiazolo[5,4-c]pyridine-2-carboxamide hydrochloride, MW: 498.81322 | MF: C20H18Cl3N5O2S.
   10: CID: 10006114, IUPAC: N-[4-chloro-2-[(5-chloropyridin-2-yl)carbamoyl]phenyl]-5-methyl-6,7-dihydro-4H-[1,3]thiazolo[5,4-c]pyridine-2-carboxamide, MW: 462.35228 | MF: C20H17Cl2N5O2S.
   11: CID: 10052635, IUPAC: 2-[[2-methyl-5-[[6-[(4-methylpiperazin-1-yl)methyl]pyridin-2-yl]amino]phenyl]amino]-N-(2-methylphenyl)-1,3-thiazole-5-carboxamide, MW: 527.68362 | MF: C29H33N70S.
   12: CID: 10195898, IUPAC: N-[(4-chlorophenyl)methyl]-2-[[[(2S)-2-hydroxy-2-pyrimidin-2-ylethyl]-methylamino]methyl]-4-methyl-7-oxothieno[2,3-e]pyridine-6-carboxamide, MW: 497.99706 | MF: C24H24ClN5O3S.
   13: CID: 10206276, IUPAC: N-[4-[(5-chloropyridin-2-yl)carbamoyl]-2-phenyl-1,3-thiazol-5-yl]-1-propan-2-ylpiperidine-4-carboxamide, MW: 484.01354 | MF: C24H26ClN5O2S.
   14: CID: 10252208, IUPAC: 2-[4-(5-amino-1,3-thiazol-2-yl)phenyl]-3-(5-chloropyridin-2-yl)quinazolin-4-one, MW: 431.89746 MF: C22H14ClN5OS.
   15: CID: 10253695, IUPAC: 2-[4-[3-(5-chloropyridin-2-yl)-4-oxoquinazolin-2-yl]phenyl]-1,3-thiazole-5-carboxamide, MW: 459.90756 | MF: C23H14ClN5O2S.
   16: CID: 10301604, IUPAC: N-[4-[(5-chloropyridin-2-yl)carbamoyl]-2-(3,4-difluorophenyl)-1,3-thiazol-5-yl]-1-propan-2-ylpiperidine-4-carboxamide, MW: 519.994466 | MF: C24H24ClF2N5O2S.
   17: CID: 10344807, IUPAC: N-[2-[4-[3-(5-chloropyridin-2-yl)-4-oxoquinazolin-2-yl]phenyl]-1,3-thiazol-4-yl]acetamide, MW: 473.93414 | MF: C24H16ClN5O2S.
   18: CID: 10368624, IUPAC: N-[(4-chlorophenyl)methyl]-2-[[(2-hydroxy-2-pyrimidin-2-ylethyl)-methylamino]methyl]-7-methyl-4-oxothieno[3,2-e]pyridine-5-carboxamide, MW: 497.99706 | MF:C24H24ClN5O3S.
   19: CID: 10370949, IUPAC: (3Z)-4-[[(2S)-2-(3-chlorophenyl)-2-hydroxyethyl]amino]-3-[6-methyl-2-[4-(1,3-thiazol-2-ylmethyl)piperazin-1-yl]-7,9-dihydropurin-8-ylidene]pyridin-2-one, MW: 578.08832 | MF: C27H28ClN9O2S.
   20: CID: 10412586, IUPAC: N-[2-[4-[3-(5-chloropyridin-2-yl)-4-oxoquinazolin-2-yl]phenyl]-1,3-thiazol-5-yl]acetamide, MW: 473.93414 | MF: C24H16ClN5O2S.
   21: CID: 10413555, IUPAC: N-[(4-chlorophenyl)methyl]-2-[[[(2R)-2-hydroxy-2-pyrimidin-2-ylethyl]-methylamino]methyl]-7-methyl-4-oxothieno[3,2-e]pyridine-5-carboxamide, MW: 497.99706 MF: C24H24ClN5O3S.
   22: CID: 10456156, IUPAC: 4-[(3-chlorothiophen-2-yl)methylamino]-2-[(4-morpholin-4-ylphenyl)amino]pyrimidine-5-carboxamide, MW: 444.93774 | MF: C20H21ClN6O2S.
   23: CID: 10458706, IUPAC: N-[5-[2-[(4-chlorophenyl)amino]pyrimidin-4-yl]-4-methyl-1,3-thiazol-2-yl]-3-(2-morpholin-4-ylethylamino)propanamide, MW: 502.03212 | MF: C23H28ClN7O2S.
   24: CID: 11153014, IUPAC: N-(2-chloro-6-methylphenyl)-2-[(2,6-dimethylpyrimidin-4-yl)amino]-1,3-thiazole-5-carboxamide, MW: 373.85984 | MF: C17H16ClN5OS.
   25: CID: 11167695, IUPAC: N-(2-chloro-6-methylphenyl)-2-[[2-methyl-6-(2-morpholin-4-ylethylamino)pyrimidin-4-yl]amino]-1,3-thiazole-5-carboxamide, MW: 488.00554 | MF: C22H26ClN7O2S.
   26: CID: 11168231, IUPAC: N-(2-chloro-6-methylphenyl)-2-[(6-chloro-2-methylpyrimidin-4-yl)amino]-N-[(4-methoxyphenyl)methyl]-1,3-thiazole-5-carboxamide, MW: 514.42684 | MF: C24H21Cl2N5O2S.
   27: CID: 11200510, IUPAC: N-(2-chloro-6-methylphenyl)-2-[[6-(2-hydroxyethylamino)pyridin-2-yl]amino]-1,3-thiazole-5-carboxamide, MW: 403.88582 | MF: C18H18ClN5O2S.
   28: CID: 11247793, IUPAC: N-(2-chloro-6-methylphenyl)-2-[[6-(methyl-(3-methylaminopropyl)amino)pyridin-2-yl]amino]-1,3-thiazole-5-carboxamide, MW: 444.9808 | MF: C21H25ClN6OS.
   29: CID: 11260009, IUPAC: N-(2-chloro-6-methylphenyl)-2-[[6-[4-(hydroxymethyl)piperidin-1-yl]-2-methylpyrimidin-4-yl]amino]-1,3-thiazole-5-carboxamide, MW: 472.9909 | MF: C22H25ClN6O2S.
   30: CID: 11269410, IUPAC: N-(2-chloro-6-methylphenyl)-2-[(6-chloro-2-methylpyrimidin-4-yl)amino]-1,3-thiazole-5-carboxamide, MW: 394.27832 | MF: C16H13Cl2N5OS.
   31: CID: 11282881, IUPAC: N-(2-chloro-6-methylphenyl)-2-[[6-(2-morpholin-4-ylethylamino)pyrimidin-4-yl]amino]-1,3-thiazole-5-carboxamide, MW: 473.97896 | MF: C21H24ClN7O2S.
   32: CID: 11283174, IUPAC: N-(2-chloro-6-methylphenyl)-2-[[6-(3-morpholin-4-ylpropylamino)pyridin-2-yl]amino]-1,3-thiazole-5-carboxamide, MW: 487.01748 | MF: C23H27ClN6O2S.
   33: CID: 11328827, IUPAC: N-(2-chloro-6-methylphenyl)-2-[[6-(3-imidazol-1-ylpropylamino)pyridin-2-yl]amino]-1,3-thiazole-5-carboxamide, MW: 467.97438 | MF: C22H22ClN7OS.
   34: CID: 11407465, IUPAC: N-(2-chloro-6-methylphenyl)-2-[[6-(2-hydroxyethylamino)-2-methylpyrimidin-4-y1]amino]-1,3-thiazole-5-carboxamide, MW: 418.90046 | MF: C18H19ClN6O2S.
   35: CID: 11466196, IUPAC: N-(2-chloro-6-methylphenyl)-2-[[2-methyl-6-(3-morpholin-4-ylpropylamino)pyrimidin-4-yl]amino]-1,3-thiazole-5-carboxamide. MW: 502.03212 | MF: C23H28ClN7O2S.
   36: CID: 11466607, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrochloride, MW: 524.46648 | MF: C22H27Cl2N7O2S.
   37: CID: 11487256, IUPAC: N-(2-chloro-6-methylphenyl)-2-[(6-morpholin-4-ylpyrimidin-4-yl)amino]-1,3-thiazole-5-carboxamide, MW: 430.91116 | MF: C19H19ClN6O2S.
   38: CID: 11505502, IUPAC: 2-[[6-[4-(2-hydroxyethyl)piperazin-1-yl]pyrimidin-4-yl]amino]-N-[2-methyl-5-[[3-(trifluoromethyl)benzoyl]amino]phenyl]-1,3-thiazole-5-carboxamide. MW: 626.65257 | MF: C29H29F3N8O3S.
   39: CID: 11512538, IUPAC: 2-[4-[6-[15-[(2-chloro-6-methylphenyl)carbamoyl]-1,3-thiazol-2-yl]amino]-2-methylpyrimidin-4-yl]piperazin-1-yl]ethyl 2,2-dimethylpropanoate, MW: 572.12196 | MF: C27H34ClN7O3S.
   40: CID: 11539665, IUPAC: (3-chloro-2-fluorophenyl)-[4-[[6-[(5-fluoro-1,3-thiazol-2-yl)amino]pyridin-2-yl]methyl]piperazin-1-yl]methanone, MW: 449.904626 | MF: C20H18ClF2N5OS.
   41: CID: 11540687, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrate, MW: 506.02082 | MF: C22H28ClN7O3S.
   42: CID: 11569328, IUPAC: N-(2-chloro-6-methylphenyl)-2-[[3-[4-(2-hydroxyethyl)piperazin-1-yl]-5-methylphenyl]amino]-1,3-thiazole-5-carboxamide, MW: 486.02942 | MF: C24H28ClN5O2S.
   43: CID: 11570976, IUPAC: 2-[[6-[4-(2-hydroxyethyl)piperazin-1-yl]-2-methylpyrimidin-4-yl]amino]-N-[2-methyl-5-[[3-(trifluoromethyl)phenyl]carbamoyl]phenyl]-1,3-thiazole-5-carboxamide, MW: 640.67915 | MF: C30H31F3N8O3S.
   44: CID: 11577776, IUPAC: 2-[[6-(2-hydroxyethylamino)-2-methylpyrimidin-4-yl]amino]-N-[2-methyl-5-[[3-(trifluoromethyl)benzoyl]amino]phenyl]-1,3-thiazole-5-carboxamide, MW: 571.57407 MF: C26H24F3N7O3S.
   45: CID: 11590089, IUPAC: (3-chloro-2-fluorophenyl)-[4-[5-methyl-6-(1,3-thiazol-2-ylamino)pyridin-2-yl]piperazin-1-yl]methanone, MW: 431.914163 | MF: C20H19ClFN5OS.
   46: CID: 11606973, IUPAC: N-[5-[[3-[4-(2-hydroxyethyl)piperazin-1-yl]-5-(trifluoromethyl)benzoyl]amino]-2-methylphenyl]-2-(pyridin-2-ylamino)-1,3-thiazole-5-carboxamide, MW: 625.664 | MF: C30H30F3N7O3S.
   47: CID: 11650098, IUPAC: 2-[[6-(4-methylpiperazin-1-yl)pyrimidin-4-yl]amino]-N-[2-methyl-5-[[3-(trifluoromethyl)benzoyl]amino]phenyl]-1,3-thiazole-5-carboxamide,MW: 596.62659 | MF: C28H27F3N8O2S.
   48: CID: 11650132, IUPAC: pentyl N-[5-[(2-chloro-6-methylphenyl)carbamoyl]-1,3-thiazol-2-yl]-N-[6-[4-(2-hydroxyethyl)piperazin-1-yl]-2-methylpyrimidin-4-yl]carbamate, MW: 602.14794 | MF: C28H36ClN7O4S.
   49: CID: 11650511, IUPAC: N-[5-[[3-(4-ethylpiperazin-1-yl)-5-(trifluoromethyl)benzoyl]amino]-2-methylphenyl]-2-[[6-(2-hydroxyethylamino)-2-methylpyrimidin-4-yl]amino]-1,3-thiazole-5-carboxamide, MW: 683.74695 | MF: C32H36F3N9O3S.
   50: CID: 11664355, IUPAC: 2-[(2-methyl-6-morpholin-4-ylpyrimidin-4-yl)amino]-N-[2-methyl-5-[[3-(trifluoromethyl)benzoyl]amino]phenyl]-1,3-thiazole-5-carboxamide, MW: 597.61135 | MF: C28H26F3N7O3S.
   51: CID: 11664511, IUPAC: 2-[[4-[4-(2-hydroxyethyl)piperazin-1-yl]pyridin-2-yl]amino]-N-[2-methyl-5-[[3-(trifluoromethyl)benzoyl]amino]phenyl]-1,3-thiazole-5-carboxamide, MW: 625.66451 | MF: C30H30F3N7O3S.
   52: CID: 11669430, IUPAC: N-(2-chloro-6-methylphenyl)-2-[(2-methyl-6-piperazin-1-ylpyrimidin-4-yl)amino]-1,3-thiazole-5-carboxamide, MW: 443.95298 | MF: C20H22ClN7OS. 53: CID: 11676373, IUPAC: (3-chloro-2-fluorophenyl)-[4-[[6-(1,3-thiazol-2-ylamino)pyridin-2-yl]methyl]piperazin-1-yl]methanone, MW: 431.914163 | MF: C20H19ClFN5OS.
   54: CID: 11684148, IUPAC: (3-chloro-2-fluorophenyl)-[4-[[6-[(5-chloro-1,3-thiazol-2-yl)amino]pyridin-2-yl]methyl]piperazin-1-yl]methanone, MW: 466.359223 | MF: C20H18Cl2FN5OS.
   55: CID: 11700117, IUPAC: 2-[[6-(4-ethylpiperazin-1-yl)-2-methylpyrimidin-4-yl]amino]-N-[2-methyl-5-[[3-(trifluoromethyl)benzoyl]amino]phenyl]-1,3-thiazole-5-carboxamide, MW: 624.67975 | MF: C30H31F3N8O2S.
   56: CID: 11707091, IUPAC: 2-[[2-methyl-6-(4-methylpiperazin-1-yl)pyrimidin-4-yl]amino]-N-[2-methyl-5-[[3-(trifluoromethyl)benzoyl]amino]phenyl]-1,3-thiazole-5-carboxamide, MW: 610.65317 | MF: C29H29F3N8O2S.
   57: CID: 11714286, IUPAC: 2-[[5-[4-(2-hydroxyethyl)piperazin-1-yl]pyridin-2-yl]amino]-N-[2-methyl-5-[[3-(trifluoromethyl)benzoyl]amino]phenyl]-1,3-thiazole-5-carboxamide, MW: 625.66451 | MF: C30H30F3N7O3S.
   58: CID: 11714353, IUPAC: 2-[[6-[4-(2-hydroxyethyl)piperazin-1-yl]-2-methylpyrimidin-4-yl]amino]-N-[2-methyl-5-[[3-(trifluoromethyl)benzoyl]amino]phenyl]-1,3-thiazole-5-carboxamide, MW: 640.67915 | MF: C30H31F3N8O3S.
   59: CID: 11752136, IUPAC: N-(2-chloro-6-methylphenyl)-2-[[5-[4-(2-hydroxyethyl)piperazin-1-yl]-2-methylpyrimidin-4-yl]amino]-1,3-thiazole-5-carboxamide, MW: 488.00554 | MF: C22H26ClN7O2S.
   60: CID: 11772766, IUPAC: 4-[2-(3-chlorophenyl)ethylamino]-2-pyridin-4-yl-1,3-thiazole-5-carboxamide, MW: 358.8452 | MF: C17H15ClN4OS.
   61: CID: 11775143, IUPAC: N-(2-chloro-6-methylphenyl)-2-[(2-methyl-6-morpholin-4-ylpyrimidin-4-yl)amino]-1,3-thiazole-5-carboxamide, MW: 444.93774 | MF: C20H21ClN6O2S. 62: CID: 11854012, IUPAC: 2-[4-[6-[[5-[(2-chloro-6-methylphenyl)carbamoyl]-1,3-thiazol-2-yl]amino]-2-methylpyrimidin-4-yl]piperazin-1-yl]acetic acid, MW: 501.98906 | MF: C22H24ClN7O3S.
   63: CID: 11854269, IUPAC: 2-[4-[6-[[5-[(2-chloro-6-methylphenyl)carbamoyl]-1,3-thiazol-2-yl]amino]-2-methylpyrimidin-4-yl]piperazin-1-yl]ethyl hydrogen sulfate, MW: 568.06874 | MF: C22H26ClN7O5S2.
   64: CID: 11854270, IUPAC: N-(2-chloro-6-methylphenyl)-2-[[6-[2-(2-hydroxyethylamino)ethylamino]-2-methylpyrimidin-4-yl]amino]-1,3-thiazole-5-carboxamide, MW: 461.96826 MF: C20H24ClN7O2S
   65: CID: 11854271, IUPAC: 2-[[6-(2-aminoethylamino)-2-methylpyrimidin-4-yl]amino]-N-(2-chloro-6-methylphenyl)-1,3-thiazole-5-carboxamide, MW: 417.9157 | MF: C18H20ClN7OS. 66: CID: 11854272, IUPAC: 2-[[2-[4-[6-[[5-[(2-chloro-6-methylphenyl)carbamoyl]-1,3-thiazol-2-yl]amino]-2-methylpyrimidin-4-yl]piperazin-1-yl]acetyl]amino]ethanesulfonic acid, MW: 609.12066 | MF: C24H29ClN8O5S2.
   67: CID: 11854533, IUPAC: N-(2-chloro-4-hydroxy-6-methylphenyl)-2-[[6-[4-(2-hydroxyethyl)piperazin-1-yl]-2-methylpyrimidin-4-yl]amino]-1,3-thiazole-5-carboxamide, MW: 504.00494 | MF: C22H26ClN7O3S.
   68: CID: 11854534, IUPAC: N-[2-chloro-6-(hydroxymethyl)phenyl]-2-[[6-[4-(2-hydroxyethyl)piperazin-1-yl]-2-methylpyrimidin-4-yl]amino]-1,3-thiazole-5-carboxamide, MW: 504.00494 | MF: C22H26ClN7O3S.
   69: CID: 11854535, IUPAC: N-(2-chloro-6-methylphenyl)-2-[[6-[4-(2-hydroxyethyl)-4-oxidopiperazin-4-ium-1-yl]-2-methylpyrimidin-4-yl]amino]-1,3-thiazole-5-carboxamide, MW: 504.00494 | MF: C22H26ClN7O3S.
   70: CID: 11854536, IUPAC: 2-[4-[6-[[5-[(2-chloro-6-methylphenyl)carbamoyl]-1,3-thiazol-2-yl]amino]-2-methylpyrimidin-4-yl]-1-oxidopiperazin-1-ium-1-yl]acetic acid, MW: 517.98846 | MF: C22H24ClN7O4S.
   71: CID: 11949914, IUPAC: 4-[[2-(5-chloro-2-fluorophenyl)-5-dimethylaminopyrimidin-4-yl]amino]-N-[2-(2-hydroxyethylamino)ethyl]pyridine-3-carboxamide, MW: 473.931003 | MF: C22H25ClFN7O2.
   72: CID: 11951866, IUPAC: 4-[[2-(5-chloro-2-fluorophenyl)-5-pyrrolidin-1-ylpyrimidin-4-yl]amino]-N-(2-hydroxyethyl)pyridine-3-carboxamide, MW: 456.900483 | MF: C22H22ClFN6O2.
   73: CID: 11952045, IUPAC: 4-[[2-(5-chloro-2-fluorophenyl)-5-pyrrolidin-1-ylpyrimidin-4-yl]amino)-N-[(2S)-2-hydroxypropyl]pyridine-3-carboxamide, MW: 470.927063 | MF: C23H24ClFN6O2.
   74: CID: 15979866, IUPAC: 5-[2-[[4-(4-acetylpiperazin-1-yl)pyridin-2-yl]amino]-1,3-thiazol-5-yl]-N-methylpyridine-3-carboxamide, MW: 437.51802 | MF: C21H23N7O2S.
   75: CID: 15980109, IUPAC: N-(2-aminoethyl)-5-[2-[(4-morpholin-4-ylpyridin-2-yl)amino]-1,3-thiazol-5-yl]pyridine-3-carboxamide, MW: 425.50732 | MF: C20H23N7O2S
   76: CID: 15980233, IUPAC: N-(2-hydroxyethyl)-5-[2-[(4-morpholin-4-ylpyridin-2-yl)amino]-1,3-thiazol-5-yl]pyridine-3-carboxamide, MW: 426.49208 | MF: C20H22N6O3S.
   77: CID: 15980347, IUPAC: N-(2-methylaminoethyl)-5-[2-[(4-morpholin-4-ylpyridin-2-yl)amino]-1,3-thiazol-5-yl]pyridine-3-carboxamide, MW: 439.5339 | MF: C21H25N7O2S.
   78: CID: 15980351, IUPAC: 5-[2-[[4-[4-(2-hydroxyacetyl)piperazin-1-yl]pyridin-2-yl]amino]-1,3-thiazol-5-yl]-N-(2,2,2-trifluoroethyl)pyridine-3-carboxamide, MW: 521.51539 | MF: C22H22F3N7O3S.
   79: CID: 15982537, IUPAC: (3-chloro-2-fluorophenyl)-[4-[6-[(5-fluoro-1,3-thiazol-2-yl)amino]-5-methylpyridin-2-yl]piperazin-1-yl]methanone, MW: 449.904626 | MF: C20H18ClF2N5OS.
   80: CID: 16034848, IUPAC: N-(2-chloro-6-methylphenyl)-2[[6-[4-(2-hydroxyethyl)piperazin-1-yl]-2-methylpyrimidin-4-yl]amino]-1,3-thiazole-5-carboxamide; 2,3-dihydroxybutanedioic acid, MW: 638.09238 | MF: C26H32ClN7O8S.
   81: CID: 16037977, IUPAC: N-(2-chloro-6-methylphenyl)-2-[[6-[4-(2-hydroxyethyl)piperazin-1-yl]-5-methylpyrimidin-4-yl]amino]-1,3-thiazole-5-carboxamide, MW: 488.00554 | MF: C22H26ClN7O2S.
   82: CID: 16061431, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 4-[(4-methylpiperazin-1-yl)methyl]-N-[4-methyl-3-[(4-pyridin-3-ylpyrimidin-2-yl)amino]phenyl]benzamide, MW: 981.60828 | MF: C51H57ClN14O3S.
   83: CID: 16223227, IUPAC: but-2-enedioic acid; N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 604.0777 | MF: C26H30ClN7O6S.
   84: CID: 16223228, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrobromide, MW: 568.91748 | MF: C22H27BrClN7O2S.
   85: CID: 16223229, IUPAC: but-2-enedioic acid; N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 604.0777 | MF: C26H30ClN7O6S.
   86: CID: 16223316, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; methanesulfonic acid, MW: 584.1112 | MF: C23H30ClN7O5S2.
   87: CID: 16223317, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; phosphoric acid, MW: 586.000721 | MF: C22H29ClN7O6PS.
   88: CID: 16223318, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 2-hydroxybenzoic acid, MW: 626.12628 | MF: C29H32ClN7O5S.
   89: CID: 16223319, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; sulfuric acid, MW: 586.08402 | MF: C22H28ClN7O6S2.
   90: CID: 16223320, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 4-methylbenzenesulfonic acid, MW: 660.20716 | MF: C29H34ClN7O5S2.
   91: CID: 16584134, AKE-PB223730486, IUPAC: N-(4-chlorophenyl)-2-[(4,5-dimethyl-1,3-thiazol-2-yl)amino]-4-methylpyrimidine-5-carboxamide, MW: 373.85984 | MF: C17H16ClN5OS.
   92: CID: 16584137, AKE-PB223730492, IUPAC: N-(3-chlorophenyl)-2-[(4,5-dimethyl-1,3-thiazol-2-yl)amino]-4-methylpyrimidine-5-carboxamide, MW: 373.85984 | MF: C17H16ClN5OS.
   93: CID: 16584139, AKE-PB223730496, IUPAC: 2-[(4,5-dimethyl-1,3-thiazol-2-yl)amino]-4-methyl-N-(2-methylphenyl)pyrimidine-5-carboxamide, MW: 353.44136 | MF: C18H19N5OS. 94: CID: 16655683, IUPAC: 2-[(6-chloro-2-methylpyrimidin-4-yl)amino]-N-(2,6-dichlorophenyl)-1,3-thiazole-5-carboxamide, MW: 414.6968 | MF: C15H10Cl3N5OS.
   95: CID: 16655839, IUPAC: N-(2,6-dichlorophenyl)-2-[[6-[4-(2-hydroxyethyl)piperazin-1-yl]-2-methylpyrimidin-4-yl]amino]-1,3-thiazole-5-carboxamide, MW: 508.42402 | MF: C21H23Cl2N7O2S.
   96: CID: 16660745, IUPAC: N-(4-fluorophenyl)-4-(2-hydroxyethylamino)-6-methylsulfanyl-2-pyridin-4-ylpyrimidine-5-carboxamide, MW: 399.441923 | MF: C19H18FN5O2S.
   97: CID: 16660747, IUPAC: N-(4-ethylphenyl)-4-(2-hydroxyethylamino)-6-methylsulfanyl-2-pyridin-4-ylpyrimidine-5-carboxamide, MW: 409.50462 MF: C21H23N5O28.
   98: CID: 16660907, IUPAC: 4-(2-hydroxyethylamino)-N-(4-methylphenyl)-6-methylsulfanyl-2-pyridin-4-ylpyrimidine-5-carboxamide, MW: 395.47804 | MF: C20H21N5O2S.
   99: CID: 16661063, IUPAC: N-(4-chlorophenyl)-4-(2-hydroxyethylamino)-6-methylsulfanyl-2-pyridin-4-ylpyrimidine-5-carboxamide, MW: 415.89652 | MF: C19H18ClN5O2S.
   100: CID: 16661212, IUPAC: N-(2,4-dimethylphenyl)-4-(2-hydroxyethylamino)-6-methylsulfanyl-2-pyridin-4-ylpyrimidine-5-carboxamide, MW: 409.50462 | MF: C21H23N5O2S.
   101: CID: 16661214, IUPAC: 4-(1-hydroxybutan-2-ylamino)-N-(4-methylphenyl)-6-methylsulfanyl-2-pyridin-4-ylpyrimidine-5-carboxamide, MW: 423.5312 | MF: C22H25N502S.

Herein, CID is the compound identifier as defined in Pubchem.
e) Small molecules targeting and antagonising downstream components of the NEPOR signalling pathway, particularly EPH-B4 tyrosine kinase inhibitors.
f) Combination therapies involving one or more of approaches a-e.
g) The present disclosure also provides a combination therapy. Co-administration of EPO
with an intracellular inhibitor of NEPOR signalling (*e.g*. Dasatinib) is proposed to maintain EPO signalling via EPOR (and thus promote haematopoiesis) while inhibiting survival of NEPOR positive tumour cells.

### NEPOR Based Therapeutics to Treat Neuronal Insults

Without being bound by theory, the present disclosure provides that EPO is neuroprotective because of its effects on NEPOR activity, i.e. in particular and/or EPH-B4 and/or Ephrin A1 activity. Therefore, the present disclosure provides a method for treating ischemic stroke, trauma, epilepsy, neurodegenerative diseases, and cognitive dysfunction with an agonistic NEPOR targeted therapy. Specific approaches to positively enhance EPOR mediated survival signals include:
a) NEPOR specific antibodies. Such antibodies bind and initiate/enhance the mediation of NEPOR's cyto-protective activity.
b) NEPOR specific small-molecules. Such small molecules bind and initiate/enhance the mediation of NEPOR's cytoprotective activity.
c) NEPOR-targeting EPO mutants and glycosylated versions thereof. Due to EPOR's strict conformational requirements for mediating signalling in response to EPO, the following EPOR mutants (SEQ ID NO. 17 - SEQ ID NO. 212.) favour binding to NEPOR as opposed to EPOR and thus primarily act as tissue protective.

SEQ ID NO.17 
APPRLICDSRVLERYLLEAKEAENITRVGQQAVEVWQGLALLSEAVLRGQALLVNSSQPWEPLQLHVDKAVSGLRSL
TTLLRALGAQKEAISPPDAASAAPLRTITADTFRKLFRVYSNFLRGKLKLYTGEACRTGDR
SEQ ID NO.18 
APPRLICDSRVLERYLLEAKEAENITTGCAEHCSLNENITVPDTKVNFYAWKRMEVGQQALLVNSSQPWEPLQLHVD
KAVSGLRSLTTLLRALGAQKEAISPPDAASAAPLRTITADTFRKLFRVYSNFLRGKLKLYTGEACRTGDR
SEQ ID NO.19 
APPRLICDSRVLERYLLEAKEAENITTGCAEHCSLNENITVPDTKVNFYALLVNSSQPWEPLQLHVDKAVSGLRSLT
TLLRALGAQKEAISPPDAASAAPLRTITADTFRKLFRVYSNFLRGKLKLYTGEACRTGDR
SEQ ID NO.20 
APPRLICDSRVLERYLLEAKEAENITTGCAEHCSLNENITVPDTKVNFYAWKRMEPWEPLQLHVDKAVSGLRSLTTL
LRALGAQKEAISPPDAASAAPLRTITADTFRKLFRVYSNFLRGKLKLYTGEACRTGDR
SEQ ID N0.21
   APPRLICDSRVLERYLLEAKEAENITTGCAEHCSLNENITVPPGVGQLFPAVGAPAAACG
SEQ ID NO.22
   APPRLICDSRVLERYLLEAKEAENITTGCAEHCSLNENNHC
SEQ ID NO.23
   APPRLICDSRVLERYLLEAKEAENIT
SEQ ID NO.24
   APPRLICDSRVLEAYLLEAKEAENIT
SEQ ID NO.25
   APPRLICDSRVLEEYLLEAKEAENIT
SEQ ID NO.26
   APPRLICDSRVLERYL
SEQ ID NO.27
   APPRLI
SEQ ID NO.28

APPRLICDSRVLERYILEAKEAENVTMGCAEGPRLSENITVPDTKVNFYAWKRMEKELMSPPDTTPPAPLRTLTVDT
FCKLFRVYANFLRGKLKLYTGEVCRRGDR

Deletions of hWT 4 EPOR interaction sites
SEQ ID NO.29 
APPRLICEAENITTGCAEHCSLNENITVPDTKVNFYAWKRMEVGQQAVEVWQGLALLSEAVLRGQALLVNSSQPWEP
LQLHVDKAVSGLRSLTTLLRALGAQKEAISPPDAASAAPLRTITADTFRKLFRVYSNFLRGKLKLYTGEACRTGDR
SEQ ID NO.30 
APPRLICDSRVLERYLLEAKEAENITTGCAEHCSLNENITVPDFYAWKRMEVGQQAVEVWQGLALLSEAVLRGQALL
VNSSQPWEPLQLHVDKAVSGLRSLTTLLRALGAQKEAI SPPDAASAAPLRTITADTFRKLFRVYSNFLRGKLKLYTG
EACRTGDR
SEQ ID N0.31 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDLTTLLRALGAQKEAISPPDAASAAPLRTITADTFRKLFRVYSNFLRGKLKLYTGEACR
TGDR
SEQ ID NO.32

APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFGKLKLYTGEACR
TGDR

C-term deletions beginning at the last Cysteine bridge C161
SEQ ID NO.33 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEA
SEQ ID NO.34 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGE
SEQ ID NO.35 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTG
SEQ ID NO.36 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYT
SEQ ID NO.37 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LY
SEQ ID NO.38 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
L
SEQ ID NO.39 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
SEQ ID NO.40 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKL
SEQ ID NO.41 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGK
SEQ ID NO.42 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRG
SEQ ID NO.43 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLR
SEQ ID NO.44 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFL
SEQ ID NO.45 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNF
SEQ ID NO.46 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSN
SEQ ID NO.47 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYS
SEQ ID NO.48 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVY
SEQ ID NO.49 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRV
SEQ ID NO.50 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFR
SEQ ID NO.51 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLF
SEQ ID NO.52 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKL
SEQ ID NO.53 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRK
SEQ ID NO.54 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFR
SEQ ID NO.55 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTF
SEQ ID NO.56 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADT
SEQ ID NO.57 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITAD
SEQ ID NO.58 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITA
SEQ ID NO.59 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTIT
SEQ ID NO.60 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTI
SEQ ID NO.61 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRT
SEQ ID NO.62 
APPRLICDSRVLERYLLEAKEAENITT'GCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLR
SEQ ID NO.63 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPL
SEQ ID NO.64 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAP
SEQ ID NO.65 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAA
SEQ ID NO.66 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASA
SEQ ID NO.67 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAAS
SEQ ID NO.68 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAA
SEQ ID NO.69 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDA
SEQ ID NO.70 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPD
SEQ ID NO.71 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPP
SEQ ID NO.72 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISP
SEQ ID NO.73 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAIS
SEQ ID NO.74 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAI
SEQ ID NO.75 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEA
SEQ ID NO.76 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKE
SEQ ID NO.77 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQK
SEQ ID NO.78 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQ
SEQ ID NO.79 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGA
SEQ ID NO.80 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALG
SEQ ID NO.81 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRAL
SEQ ID NO.82 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRA
SEQ ID NO.83 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLR
SEQ ID NO.84 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLL
SEQ ID NO.85 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTL
SEQ ID NO.86 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTT
SEQ ID NO.87 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLT
SEQ ID NO.88 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSL
SEQ ID NO.89 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRS
SEQ ID NO.90 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLR
SEQ ID NO.91 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGL
SEQ ID NO.92 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSG
SEQ ID NO.93 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVS
SEQ ID NO.94 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAV
SEQ ID NO.95 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKA
SEQ ID NO.96 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDK
SEQ ID NO.97 
APPRLICDSRVLERYLLEAKEAENITTGCAEHCSLNENITVPDTKVNFYAWKRMEVGQQAVEVWQGLALLSEAVLRG
QALLVNSSQPWEPLQLHVD
SEQ ID NO.98 
APPRLICDSRVLERYLLEAKEAENITTGCAEHCSLNENITVPDTKVNFYAWKRMEVGQQAVEVWQGLALLSEAVLRG
QALLVNSSQPWEPLQLHV
SEQ ID NO.99 
APPRLICDSRVLERYLLEAKEAENITTGCAEHCSLNENITVPDTKVNFYAWKRMEVGQQAVEVWQGLALLSEAVLRG
QALLVNSSQPWEPLQLH
SEQ ID NO.100 
APPRLICDSRVLERYLLEAKEAENITTGCAEHCSLNENITVPDTKVNFYAWKRMEVGQQAVEVWQGLALLSEAVLRG
QALLVNSSQPWEPLQL
SEQ ID NO.101 
APPRLICDSRVLERYLLEAKEAENITTGCAEHCSLNENITVPDTKVNFYAWKRMEVGQQAVEVWQGLALLSEAVLRG
QALLVNSSQPWEPLQ
SEQ ID NO.102 
APPRLICDSRVLERYLLEAKEAENITTGCAEHCSLNENITVPDTKVNFYAWKRMEVGQQAVEVWQGLALLSEAVLRG
QALLVNSSQPWEPL
SEQ ID NO.103 
APPRLICDSRVLERYLLEAKEAENITTGCAEHCSLNENITVPDTKVNFYAWKRMEVGQQAVEVWQGLALLSEAVLRG
QALLVNSSQPWEP
SEQ ID NO.104 
APPRLICDSRVLERYLLEAKEAENITTGCAEHCSLNENITVPDTKVNFYAWKRMEVGQQAVEVWQGLALLSEAVLRG
QALLVNSSQPWE
SEQ ID NO.105 
APPRLICDSRVLERYLLEAKEAENITTGCAEHCSLNENITVPDTKVNFYAWKRMEVGQQAVEVWQGLALLSEAVLRG
QALLVNSSQPW
SEQ ID NO.106 
APPRLICDSRVLERYLLEAKEAENITTGCAEHCSLNENITVPDTKVNFYAWKRMEVGQQAVEVWQGLALLSEAVLRG
QALLVNSSQP
SEQ ID NO.107 
APPRLICDSRVLERYLLEAKEAENITTGCAEHCSLNENITVPDTKVNFYAWKRMEVGQQAVEVWQGLALLSEAVLRG
QALLVNSSQ
SEQ ID NO.108 
APPRLICDSRVLERYLLEAKEAENITTGCAEHCSLNENITVPDTKVNFYAWKRMEVGQQAVEVWQGLALLSEAVLRG
QALLVNSS
SEQ ID NO.109 
APPRLICDSRVLERYLLEAKEAENITTGCAEHCSLNENITVPDTKVNFYAWKRMEVGQQAVEVWQGLALLSEAVLRG
QALLVNS
SEQ ID NO.110 
APPRLICDSRVLERYLLEAKEAENITTGCAEHCSLNENITVPDTKVNFYAWKRMEVGQQAVEVWQGLALLSEAVLRG
QALLVN
SEQ ID NO.111 
APPRLICDSRVLERYLLEAKEAENITTGCAEHCSLNENITVPDTKVNFYAWKRMEVGQQAVEVWQGLALLSEAVLRG
QALLV
SEQ ID NO.112 
APPRLICDSRVLERYLLEAKEAENITTGCAEHCSLNENITVPDTKVNFYAWKRMEVGQQAVEVWQGLALLSEAVLRG
QALL
SEQ ID NO.113 
APPRLICDSRVLERYLLEAKEAENITTGCAEHCSLNENITVPDTKVNFYAWKRMEVGQQAVEVWQGLALLSEAVLRG
QAL
SEQ ID NO.114 
APPRLICDSRVLERYLLEAKEAENITTGCAEHCSLNENITVPDTKVNFYAWKRMEVGQQAVEVWQGLALLSEAVLRG
QA
SEQ ID NO.115 
APPRLICDSRVLERYLLEAKEAENITTGCAEHCSLNENITVPDTKVNFYAWKRMEVGQQAVEVWQGLALLSEAVLRG
Q
SEQ ID NO.116
   APPRLICDSRVLERYLLEAKEAENITTGCAEHCSLNENITVPDTKVNFYAWKRMEVGQQAVEVWQGLALLSEAVLRG
SEQ ID NO.117
   APPRLICDSRVLERYLLEAKEAENITTGCAEHCSLNENITVPDTKVNFYAWKRMEVGQQAVEVWQGLALLSEAVLR
SEQ ID NO.118
   APPRLICDSRVLERYLLEAKEAENITTGCAEHCSLNENITVPDTKVNFYAWKRMEVGQQAVEVWQGLALLSEAVL
SEQ ID NO.119
   APPRLICDSRVLERYLLEAKEAENITTGCAEHCSLNENITVPDTKVNFYAWKRMEVGQQAVEVWQGLALLSEAV
SEQ ID NO.120
   APPRLICDSRVLERYLLEAKEAENITTGCAEHCSLNENITVPDTKVNFYAWKRMEVGQQAVEVWQGLALLSEA
SEQ ID NO.121
   APPRLICDSRVLERYLLEAKEAENITTGCAEHCSLNENITVPDTKVNFYAWKRMEVGQQAVEVWQGLALLSE
SEQ ID NO.122
   APPRLICDSRVLERYLLEAKEAENITTGCAEHCSLNENITVPDTKVNFYAWKRMEVGQQAVEVWQGLALLS
SEQ ID NO.123
   APPRLICDSRVLERYLLEAKEAENITTGCAEHCSLNENITVPDTKVNFYAWKRMEVGQQAVEVWQGLALL
SEQ ID NO.124
   APPRLICDSRVLERYLLEAKEAENITTGCAEHCSLNENITVPDTKVNFYAWKRMEVGQQAVEVWQGLAL
SEQ ID NO.125
   APPRLICDSRVLERYLLEAKEAENITTGCAEHCSLNENITVPDTKVNFYAWKRMEVGQQAVEVWQGLA
SEQ ID NO.126
   APPRLICDSRVLERYLLEAKEAENITTGCAEHCSLNENITVPDTKVNFYAWKRMEVGQQAVEVWQGL
SEQ ID NO.127
   APPRLICDSRVLERYLLEAKEAENITTGCAEHCSLNENITVPDTKVNFYAWKRMEVGQQAVEVWQG
SEQ ID NO.128
   APPRLICDSRVLERYLLEAKEAENITTGCAEHCSLNENITVPDTKVNFYAWKRMEVGQQAVEVWQ
SEQ ID NO.129
   APPRLICDSRVLERYLLEAKEAENITTGCAEHCSLNENITVPDTKVNFYAWKRMEVGQQAVEVW
SEQ ID NO.130
   APPRLICDSRVLERYLLEAKEAENITTGCAEHCSLNENITVPDTKVNFYAWKRMEVGQQAVEV
SEQ ID NO.131
   APPRLICDSRVLERYLLEAKEAENITTGCAEHCSLNENITVPDTKVNFYAWKRMEVGQQAVE
SEQ ID NO.132
   APPRLICDSRVLERYLLEAKEAENITTGCAEHCSLNENITVPDTKVNFYAWKRMEVGQQAV
SEQ ID NO.133
   APPRLICDSRVLERYLLEAKEAENITTGCAEHCSLNENITVPDTKVNFYAWKRMEVGQQA
SEQ ID NO.134
   APPRLICDSRVLERYLLEAKEAENITTGCAEHCSLNENITVPDTKVNFYAWKRMEVGDQ
SEQ ID NO.135
   APPRLICDSRVLERYLLEAKEAENITTGCAEHCSLNENITVPDTKVNFYAWKRMEVGQ
SEQ ID NO.136
   APPRLICDSRVLERYLLEAKEAENITTGCAEHCSLNENITVPDTKVNFYAWKRMEVG
SEQ ID NO.137
   APPRLICDSRVLERYLLEAKEAENITTGCAEHCSLNENITVPDTKVNFYAWKRMEV
SEQ ID NO.138
   APPRLICDSRVLERYLLEAKEAENITTGCAEHCSLNENITVPDTKVNFYAWKRME
SEQ ID NO.139
   APPRLICDSRVLERYLLEAKEAENITTGCAEHCSLNENITVPDTKVNFYAWKRM
SEQ ID NO.140
   APPRLICDSRVLERYLLEAKEAENITTGCAEHCSLNENITVPDTKVNFYAWKR
SEQ ID NO.141
   APPRLICDSRVLERYLLEAKEAENITTGCAEHCSLNENITVPDTKVNFYAWK
SEQ ID NO.142
   APPRLICDSRVLERYLLEAKEAENITTGCAEHCSLNENITVPDTKVNFYAW
SEQ ID NO.143
   APPRLICDSRVLERYLLEAKEAENITTGCAEHCSLNENITVPDTKVNFYA
SEQ ID NO.144
   APPRLICDSRVLERYLLEAKEAENITTGCAEHCSLNENITVPDTKVNFY
SEQ ID NO.145
   APPRLICDSRVLERYLLEAKEAENITTGCAEHCSLNENITVPDTKVNF
SEQ ID NO.146
   APPRLICDSRVLERYLLEAKEAENITTGCAEHCSLNENITVPDTKVN
SEQ ID NO.147
   APPRLICDSRVLERYLLEAKEAENITTGCAEHCSLNENITVPDTKV
SEQ ID NO.148
   APPRLICDSRVLERYLLEAKEAENITTGCAEHCSLNENITVPDTK
SEQ ID NO.149
   APPRLICDSRVLERYLLEAKEAENITTGCAEHCSLNENITVPDT
SEQ ID NO.150 APPRLICDSRVLERYLLEAKEAENITTGCAEHCSLNENITVPD
SEQ ID NO.151
   APPRLICDSRVLERYLLEAKEAENITTGCAEHCSLNENITVP
SEQ ID NO.152
   APPRLICDSRVLERYLLEAKEAENITTGCAEHCSLNENITV
SEQ ID NO.153
   APPRLICDSRVLERYLLEAKEAENITTGCAEHCSLNENIT
SEQ ID NO.154
   APPRLICDSRVLERYLLEAKEAENITTGCAEHCSLNENI
SEQ ID NO.155
   APPRLICDSRVLERYLLEAKEAENITTGCAEHCSLNEN
SEQ ID NO.156
   APPRLICDSRVLERYLLEAKEAENITTGCAEHCSLNE
SEQ ID NO.157
   APPRLICDSRVLERYLLEAKEAENITTGCAEHCSLN
SEQ ID NO.158
   APPRLICDSRVLERYLLEAKEAENITTGCAEHCSL
SEQ ID NO.159
   APPRLICDSRVLERYLLEAKEAENITTGCAEHCS
SEQ ID NO.160
   APPRLICDSRVLERYLLEAKEAENITTGCAEHC
SEQ ID NO.161
   APPRLICDSRVLERYLLEAKEAENITTGCAEH
SEQ ID NO.162
   APPRLICDSRVLERYLLEAKEAENITTGCAE
SEQ ID NO.163
   APPRLICDSRVLERYLLEAKEAENITTGCA
SEQ ID NO.164
   APPRLICDSRVLERYLLEAKEAENITTGC
SEQ ID NO.165
   APPRLICDSRVLERYLLEAKEAENITTG
SEQ ID NO.166
   APPRLICDSRVLERYLLEAKEAENITT
SEQ ID NO.167
   APPRLICDSRVLERYLLEAKEAENIT
SEQ ID NO.168
   APPRLICDSRVLERYLLEAKEAENI
SEQ ID NO.169
   APPRLICDSRVLERYLLEAKEAEN
SEQ ID NO.170
   APPRLICDSRVLERYLLEAKEAE
SEQ ID NO.171
   APPRLICDSRVLERYLLEAKEA
SEQ ID NO.172
   APPRLICDSRVLERYLLEAKE
SEQ ID NO.173
   APPRLICDSRVLERYLLEAK
SEQ ID NO.174
   APPRLICDSRVLERYLLEA
SEQ ID NO.175
   APPRLICDSRVLERYLLE
SEQ ID NO.176
   APPRLICDSRVLERYLL
SEQ ID NO.177
   APPRLICDSRVLERYL
SEQ ID NO.178
   APPRLICDSRVLERY
SEQ ID NO.179
   APPRLICDSRVLER
SEQ ID NO.180
   APPRLICDSRVLE
SEQ ID NO.181
   APPRLICDSRVL
SEQ ID NO.182
   APPRLICDSRV
SEQ ID NO.183
   APPRLICDSR
SEQ ID NO.184
   APPRLICDS
SEQ ID NO.185
   APPRLICD
SEQ ID NO.186
   APPRLIC

Single Amino Acid Mutations (Ala / Conversions) and all combinations/permutations thereof and all glycosylated versions of same. All possible combinations/permutations of mutations contained in Single mutations of SEQ ID NOs. 187-208 and glycosylated versions thereof.
SEQ ID NO.107 
APPRLICASRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.188 
APPRLICRSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.189 
APPRLICDSRVLEAYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.190 
APPRLICDSRVLEEYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.191 
APPRLICDSRVLERYLLEAAEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.192 
APPRLICDSRVLERYLLEAEEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.193 
APPRLICDSRVLERYLLEAKEAENITTGCAEHCSLNENITVPDAKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.194 
APPRLICDSRVLERYLLEAKEAENITTGCAEHCSLNENITVPDTAVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.195 
APPRLICDSRVLERYLLEAKEAENITTGCAEHCSLNENITVPDTEVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.196 
APPRLICDSRVLERYLLEAKEAENITTGCAEHCSLNENITVPDTKANFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.197 
APPRLICDSRVLERYLLEAKEAENITTGCAEHCSLNENITVPDTKVAFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLRGKLK
LYTGEACRTGDR
SEQ ID NO.198 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDAAVSGLRSLTTLLRALGAQKEAISPPDAASAAPLRTITADTFRKLFRVYSNFLRGKLK
LYTGEACRTGDR
SEQ ID NO.199 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDEAVSGLRSLTTLLRALGAQKEAI SPPDAASAAPLRTITADTFRKLFRVYSNFLRGKLK
LYTGEACRTGDR
SEQ ID NO.200 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVAGLRSLTTLLRALGAQKEAISPPDAASAAPLRTITADTFRKLFRVYSNFLRGKLK
LYTGEACRTGDR
SEQ ID NO.201 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLASLTTLLRALGAQKEAI SPPDAASAAPLRTITADTFRKLFRVYSNFLRGKLK
LYTGEACRTGDR
SEQ ID NO.202 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLESLTTLLRALGAQKEAISPPDAASAAPLRTITADTFRKLFRVYSNFLRGKLK
LYTGEACRTGDR
SEQ ID NO.203 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRALTTLLRALGAQKEAISPPDAASAAPLRTITADTFRKLFRVYSNFLRGKLK
LYTGEACRTGDR
SEQ ID NO.204 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFAVYSNFLRGKLK
LYTGEACRTGDR
SEQ NO.205 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFEVYSNFLRGKLK
LYTGEACRTGDR
SEQ ID NO.206 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAI SPPDAASAAPLRTITADTFRKLFRVYSAFLRGKLK
LYTGEACRTGDR
SEQ ID NO.207 
APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLAGKLK
LYTGEACRTGDR
SEQ ID NO.208

APPRLICDSRVLERYLLEAKEAENITTGCAEHCSLNENITVPDTKVNFYAWKRMEVGQQAVEVWQGLALLSEAVLRG
QALLVNSSQPWEPLQLHVDKAVSGLRSLTTLLRALGAQKEAISPPDAASAAPLRTITADTFRKLFRVYSNFLEGKLK
LYTGEACRTGDR

EPO peptides overlapping interaction regions
SEQ ID NO.209
   APPRLICDSRVLERYLLEAKEAENITT
SEQ ID NO.210
   NENITVPDTKVNFYAWKRMEV
SEQ ID NO.211
   NSSQPWEPLQLHVDKAVSGLRSLTTLL
SEQ ID NO.212
   FRKLFRVYSNFLRGKLKL

d) NEPOR-targeting EPO chimera's. Such mutants bind and initiate/enhance the mediation of NEPOR's cytoprotective activity. For example, in a scenario where NEPOR constitutes an Ephrin A1 molecule (either as a homodimer or in heterodimeric association with EPOR), then chimeric proteins involving fusions of part of EPH-B4's Ephrin-ligand-binding domain and part of the EPO molecule may be developed as optimised binding partners. This implies fusing an N-terminal portion of EPO (derived from SEQ IDNO. 213) to a C-terminal portion of EPH-B4's Ephrin ligand binding domain (SEQ IDNO. 214), giving a sequence similar to SEQ IDNO. 215, or fusing an N-terminal portion of EPH-B4's Ephrin ligand binding domain (derived from SEQ ID NO. 214) to a C-terminal portion of EPO (SEQ ID NO. 213), giving a sequence similar to SEQ ID NO. 216.
e) high-affinity peptides which specifically target NEPOR to initiate/enhance the mediation of EPO's cytoprotective activity.
f) Small molecules targeting and enhancing the activity of downstream components of NEPOR.
g) Combination therapies involving one or more of approaches a-f.

SEQ ID NO.213
   >P01588|EPO_HUMAN Erythropoietin - Homo sapiens (Human). 
MGVHECPAWLWLLLSLLSLPLGLPVLGAPPRLICDSRVLERYLLEAKEAENITTGCAEHC
SLNENITVPDTKVNFYAWKRMEVGQQAVEVWQGLALLSEAVLRGQALLVNSSQPWEPLQL
HVDKAVSGLRSLTTLLRALGAQKEAISPPDAASAAPLRTITADTFRKLFRVYSNFLRGKL
KLYTGEACRTGDR
SEQ ID NO.214
   >EPH-B4_ephrin_ligand_binding_domain 
EETLLNTKLETADLKWVTFPQVDGQWEELSGLDEEQHSVRTYEVCDVQRAPGQAHWLRTG
WVPRRGAVHVYATLRFTMLECLSLPRAGRSCKETFTVFYYESDADTATALTPAWMENPYI
KVDTVAAEHLTRKRPGAEATGKVNVKTLRLGPLSKAGFYLAFQDQGACMALLSLHLFYKK
C
SEQ ID NO.215
   >NtermEPO_CtermEPHB4LBD 
APPRLICDSRVLERYLLEAKEAENITTGCAEHCSLNENITVPDTKVNFYAWKRMEVGQQA
VEVWQGLALLSEAVLRGQALLVNSSQPWEPLQLHVDKAVSGLRSLTTLLRALGAQKEAIS
PPDAASALTPAWMENPYIKVDTVAAEHLTRKRPGAEATGKVNVKTLRLGPLSKAGFYLAF
QDQGACMALLSLHLFYKKC
SEQ ID NO.216
   >NtermEPHB4LBD_CtermEPO

EETLLNTKLETADLKWVTFPQVDGQWEELSGLDEEQHSVRTYEVCDVQRAPGQAHWLRTG
WVPRRGAVHVYATLRFTMLECLSLPRAGRSCKETFTVFYYESDADTATALSEAVLRGQAL
LVNSSQPWEPLQLHVDKAVSGLRSLTTLLRALGAQKEAISPPDAASAAPLRTITADTFRK
LFRVYSNFLRGKLKLYTGEACRTGDR

### Compounds in Combination with EPO

Such compounds, in combination with EPO, inhibit EPH-B4's tyrosine kinase activity while permitting EPOR mediated signalling/haematopoiesis. The following 101 compounds, either alone or in combination, inhibit the tyrosine kinase activity of EPH-B4 containing NEPOR dimers. Therefore, the present disclosure provides a combination therapeutic agent of a tyrosine kinase inhibitor in combination with EPO to provide the hematopoietic properties of EPO along with the prevention of NEPOR signalling so as to block the potentially fatal side effect of EPO to promote tumour survival and angiogenesis.
1: CID: 1095868, AKI-STT-00166305; ZINC00818264; BAS 09636496 IUPAC: N-[5-[(3-chlorophenyl)methyl]-1,3-thiazol-2-yl]-2-(4,6-dimethylpyrimidin-2-yl)sulfanylacetamide. MW: 404.93678 | MF: C18H17ClN4OS2. (MW is molecular weight and MF is molecular formula).
2: CID: 1465558, IUPAC: 2-[(3-chlorobenzoyl)amino]-4-methyl-N-pyridin-3-yl-1,3-thiazole-5-carboxamide, MW: 372.82872 | MF: C17H13ClN4O2S.
3: CID: 1468201, IUPAC: N-[5-[(2-chlorophenyl)carbamoyl]-4-methyl-1,3-thiazol-2-yl]pyridine-4-carboxamide, MW: 372.82872 |MF: C17H13ClN4O2S.
4: CID: 3062316, Dasatinib; Sprycel; BMS Dasatinib, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 488.00554 | MF: C22H26ClN7O2S.
5: CID: 3072360, 142287-40-9; Pyrimido(4,5-d)pyrimidin-4(1H)-one, 7-methyl-1-phenyl-2-((3-(4-(2-thiazolyl)-1-piperazinyl)propyl)thio)- IUPAC: 2-methyl-8-phenyl-7-[3-[4-(1,3-thiazol-2-yl)piperazin-1-yl]propylsulfanyl]pyrimido[6,5-d]pyrimidin-5-one, MW: 479.6209 | MF: C23H25N7OS2.
6: CID: 5041467, STK154706; ZINC04687922, IUPAC: [2-[(2-methylphenyl)amino]-1,3-thiazol-4-yl]-(4-pyrimidin-2-ylpiperazin-1-yl)methanone, MW: 380.4667 | MF: C19H20N6OS.
7: CID: 9822929, IUPAC: N-(2-chloro-6-methylphenyl)-2-[(6-imidazol-1-ylpyridazin-3-yl)amino]-1,3-thiazole-5-carboxamide, MW: 411.869 | MF: C18H14ClN7OS.
8: CID: 9927718, IUPAC: N-(2-chloro-6-methylphenyl)-2-(cyclopropanecarbonylamino)-1,3-thiazole-5-carboxamide, MW: 335.809 | MF: C15H14ClN3O2S.
9: CID: 10006113, IUPAC: N-[4-chloro-2-[(5-chloropyridin-2-yl)carbamoyl]phenyl]-5-methyl-6,7-dihydro-4H-[1,3]thiazolo[5,4-c]pyridine-2-carboxamide hydrochloride, MW: 498.81322 | MF: C20H18Cl3N5O2S.
10: CID: 10006114, IUPAC: N-[4-chloro-2-[(5-chloropyridin-2-yl)carbamoyl]phenyl]-5-methyl-6,7-dihydro-4H-[1,3]thiazolo[5,4-c]pyridine-2-carboxamide, MW: 462.35228 | MF: C20H17Cl2N5O2S.
11: CID: 10052635, IUPAC: 2-[[2-methyl-5-[[6-[(4-methylpiperazin-1-yl)methyl]pyridin-2-yl]amino]phenyl]amino]-N-(2-methylphenyl)-1,3-thiazole-5-carboxamide, MW: 527.68362 | MF: C29H33N7OS.
12: CID: 10195898, IUPAC: N-[(4-chlorophenyl)methyl]-2-[[[(2S)-2-hydroxy-2-pyrimidin-2-ylethyl]-methylamino]methyl]-4-methyl-7-oxothieno[2,3-e]pyridine-6-carboxamide, MW: 497.99706 | MF: C24H24ClN5O3S.
13: CID: 10206276, IUPAC: N-[4-[(5-chloropyridin-2-yl)carbamoyl]-2-phenyl-1,3-thiazol-5-yl]-1-propan-2-ylpiperidine-4-carboxamide, MW: 484.01354 | MF: C24H26ClN5O2S.
14: CID: 10252208, IUPAC: 2-[4-(5-amino-1,3-thiazol-2-yl)phenyl]-3-(5-chloropyridin-2-yl)quinazolin-4-one, MW: 431.89746 MF: C22H14ClN5OS.
15: CID: 10253695, IUPAC: 2-[4-[3-(5-chloropyridin-2-yl)-4-oxoquinazolin-2-yl]phenyl]-1,3-thiazole-5-carboxamide, MW: 459.90756 | MF: C23H14ClN5O2S.
16: CID: 10301604, IUPAC: N-[4-[(5-chloropyridin-2-yl)carbamoyl]-2-(3,4-difluorophenyl)-1,3-thiazol-5-yl]-1-propan-2-ylpiperidine-4-carboxamide, MW: 519.994466 | MF: C24H24ClF2N5O2S.
17: CID: 10344807, IUPAC: N-[2-[4-[3-(5-chloropyridin-2-yl)-4-oxoquinazolin-2-yl]phenyl]-1,3-thiazol-4-yl]acetamide, MW: 473.93414 | MF: C24H16ClN5O2S.
18: CID: 10368624, IUPAC: N-[(4-chlorophenyl)methyl]-2-[[(2-hydroxy-2-pyrimidin-2-ylethyl)-methylamino]methyl]-7-methyl-4-oxothieno[3,2-e]pyridine-5-carboxamide, MW: 497.99706 | MF: C24H24ClN5O3S.
19: CID: 10370949, IUPAC: (3Z)-4-[[(2S)-2-(3-chlorophenyl)-2-hydroxyethyl]amino]-3-[6-methyl-2-[4-(1,3-thiazol-2-ylmethyl)piperazin-1-yl]-7,9-dihydropurin-8-ylidene]pyridin-2-one, MW: 578.08832 | MF: C27H28ClN9O2S.
20: CID: 10412586, IUPAC: N-[2-[4-[3-(5-chloropyridin-2-yl)-4-oxoquinazolin-2-yl]phenyl]-1,3-thiazol-5-yl]acetamide, MW: 473.93414 | MF: C24H16ClN5O2S.
21: CID: 10413555, IUPAC: N-[(4-chlorophenyl)methyl]-2-[[[(2R)-2-hydroxy-2-pyrimidin-2-ylethyl]-methylamino]methyl]-7-methyl-4-oxothieno[3,2-e]pyridine-5-carboxamide, MW: 497.99706 | MF: C24H24ClN5O3S.
22: CID: 10456156, IUPAC: 4-[(3-chlorothiophen-2-yl)methylamino]-2-[(4-morpholin-4-ylphenyl)amino]pyrimidine-5-carboxamide, MW: 444.93774 | MF: C20H21ClN6O2S.
23: CID: 10458706, IUPAC: N-[5-[2-[(4-chlorophenyl)amino]pyrimidin-4-yl]-4-methyl-1,3-thiazol-2-yl]-3-(2-morpholin-4-ylethylamino)propanamide, MW: 502.03212 | MF: C23H28ClN7O2S.
24: CID: 11153014, IUPAC: N-(2-chloro-6-methylphenyl)-2-[(2,6-dimethylpyrimidin-4-yl)amino]-1,3-thiazole-5-carboxamide, MW: 373.85984 | MF: C17H16ClN5OS.
25: CID: 11167695, IUPAC: N-(2-chloro-6-methylphenyl)-2-[[2-methyl-6-(2-morpholin-4-ylethylamino)pyrimidin-4-yl]amino]-1,3-thiazole-5-carboxamide, MW: 488.00554 | MF: C22H26ClN7O2S.
26: CID: 11168231, IUPAC: N-(2-chloro-6-methylphenyl)-2-[(6-chloro-2-methylpyrimidin-4-yl)amino]-N-[(4-methoxyphenyl)methyl]-1,3-thiazole-5-carboxamide, MW: 514.42684 | MF: C24H21Cl2N5O2S.
27: CID: 11200510, IUPAC: N-(2-chloro-6-methylphenyl)-2-[[6-(2-hydroxyethylamino)pyridin-2-yl]amino]-1,3-thiazole-5-carboxamide, MW: 403.88582 | MF: C18H18ClN5O2S.
28: CID: 11247793, IUPAC: N-(2-chloro-6-methylphenyl)-2-[[6-(methyl-(3-methylaminopropyl)amino)pyridin-2-yl]amino]-1,3-thiazole-5-carboxamide, MW: 444.9808 | MF: C21H25ClN6OS.
29: CID: 11260009, IUPAC: N-(2-chloro-6-methylphenyl)-2-[[6-[4-(hydroxymethyl)piperidin-1-yl]-2-methylpyrimidin-4-yl]amino]-1,3-thiazole-5-carboxamide, MW: 472.9909 | MF: C22H25ClN6O2S.
30: CID: 11269410, IUPAC: N-(2-chloro-6-methylphenyl)-2-[(6-chloro-2-methylpyrimidin-4-yl)amino]-1,3-thiazole-5-carboxamide, MW: 394.27832 | MF: C16H13Cl2N5OS.
31: CID: 11282881, IUPAC: N-(2-chloro-6-methylphenyl)-2-[[6-(2-morpholin-4-ylethylamino)pyrimidin-4-yl]amino]-1,3-thiazole-5-carboxamide, MW: 473.97896 | MF: C21H24ClN7O2S.
32: CID: 11283174, IUPAC: N-(2-chloro-6-methylphenyl)-2-[[6-(3-morpholin-4-ylpropylamino)pyridin-2-yl]amino]-1,3-thiazole-5-carboxamide, MW: 487.01748 | MF: C23H27ClN6O2S.
33: CID: 11328827, IUPAC: N-(2-chloro-6-methylphenyl)-2-[[6-(3-imidazol-1-ylpropylamino)pyridin-2-yl]amino]-1,3-thiazole-5-carboxamide, MW: 467.97438 | MF: C22H22ClN7OS.
34: CID: 11407465, IUPAC: N-(2-chloro-6-methylphenyl)-2-[[6-(2-hydroxyethylamino)-2-methylpyrimidin-4-yl]amino]-1,3-thiazole-5-carboxamide, MW: 418.90046 | MF: C18H19ClN6O2S.
35: CID: 11466196, IUPAC: N-(2-chloro-6-methylphenyl)-2-[[2-methyl-6-(3-morpholin-4-ylpropylamino)pyrimidin-4-yl]amino]-1,3-thiazole-5-carboxamide. MW: 502.03212 | MF: C23H28ClN7O2S.
36: CID: 11466607, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrochloride, MW: 524.46648 | MF: C22H27Cl2N7O2S.
37: CID: 11487256, IUPAC: N-(2-chloro-6-methylphenyl)-2-[(6-morpholin-4-ylpyrimidin-4-yl)amino]-1,3-thiazole-5-carboxamide, MW: 430.91116 | MF: C19H19ClN6O2S.
38: CID: 11505502, IUPAC: 2-[[6-[4-(2-hydroxyethyl)piperazin-1-yl]pyrimidin-4-yl]amino]-N-[2-methyl-5-[[3-(trifluoromethyl)benzoyl]amino]phenyl]-1,3-thiazole-5-carboxamide. MW: 626.65257 | MF: C29H29F3N803S.
39: CID: 11512538, IUPAC: 2-[4-[6-[[5-[(2-chloro-6-methylphenyl)carbamoyl]-1,3-thiazol,2-yl]amino]-2-methylpyrimidin-4-yl]piperazin-1-yl]ethyl 2,2-dimethylpropanoate, MW: 572.12196 | MF: C27H34ClN7O3S.
40: CID: 11539665, IUPAC: (3-chloro-2-fluorophenyl)-[4-[[6-[(5-fluoro-1,3-thiazol-2-yl)amino]pyridin-2-yl]methyl]piperazin-1-yl]methanone, MW: 449.904626 | MF: C20H18ClF2N5OS.
41: CID: 11540687, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrate, MW: 506.02082 | MF: C22H28ClN7O3S.
42: CID: 11569328, IUPAC: N-(2-chloro-6-methylphenyl)-2-[[3-[4-(2-hydroxyethyl)piperazin-1-yl]-5-methylphenyl]amino]-1,3-thiazole-5-carboxamide, MW: 486.02942 | MF: C24H28ClN5O2S.
43: CID: 11570976, IUPAC: 2-[[6-[4-(2-hydroxyethyl)piperazin-1-yl]-2-methylpyrimidin-4-yl]amino]-N-[2-methyl-5-[[3-(trifluoromethyl)phenyl]carbamoyl]phenyl]-1,3-thiazole-5-carboxamide, MW: 640.67915 | MF: C30H31F3N8O3S.
44: CID: 11577776, IUPAC: 2-[[6-(2-hydroxyethylamino)-2-methylpyrimidin-4-yl]amino]-N-[2-methyl-5-[[3-(trifluoromethyl)benzoyl]amino]phenyl]-1,3-thiazole-5-carboxamide, MW: 571.57407 | MF: C26H24F3N7O3S.
45: CID: 11590089, IUPAC: (3-chloro-2-fluorophenyl)-[4-[5-methyl-6-(1,3-thiazol-2-ylamino)pyridin-2-yl]piperazin-1-yl]methanone, MW: 431.914163 | MF: C20H19ClFN5OS.
46: CID: 11606973, IUPAC: N-[5-[[3-[4-(2-hydroxyethyl)piperazin-1-yl]-5-(trifluoromethyl)benzoyl]amino]-2-methylphenyl]-2-(pyridin-2-ylamino)-1,3-thiazole-5-carboxamide, MW: 625.66451 | MF: C30H30F3N7O3S.
47: CID: 11650098, IUPAC: -2-[[6-(4-methylpiperazin-1-yl)pyrimidin-4-yl]amino]-N-[2-methyl 5-[[3-(trifluoromethyl)benzoyl]amino]phenyl]-1,3-thiazole-5-carboxamide,MW: 596.62659 | MF: C28H27F3N8O2S.
48: CID: 11650132, IUPAC: pentyl N-[5-[(2-chloro-6-methylphenyl)carbamoyl]-1,3-thiazol-2-yl]-N-[6-[4-(2-hydroxyethyl)piperazin-1-yl]-2-methylpyrimidin-4-yl]carbamate, MW: 602.14794 | MF: C28H36ClN7O4S.
49: CID: 11650511, IUPAC: N-[5-[[3-(4-ethylpiperazin-1-yl)-5-(trifluoromethyl)benzoyl]amino]-2-methylphenyl]-2-[[6-(2-hydroxyethylamino)-2-methylpyrimidin-4-yl]amino]-1,3-thiazole-5-carboxamide, MW: 683.74695 | MF: C32H36F3N9O3S.
50: CID: 11664355, IUPAC: 2-[(2-methyl-6-morpholin-4-ylpyrimidin-4-yl)amino]-N-[2-methyl-5-[[3-(trifluoromethyl)benzoyl]amino]phenyl]-1,3-thiazole-5-carboxamide, MW: 597.61135 | MF: C28H26F3N7O3S.
51: CID: 11664511, IUPAC: 2-[[4-[4-(2-hydroxyethyl)piperazin-1-yl]pyridin-2-yl]amino]-N-[2-methyl-5-[[3-(trifluoromethyl)benzoyl]amino]phenyl]-1,3-thiazole-5-carboxamide, MW: 625.66451 | MF: C30H30F3N7O3S.
52: CID: 11669430, IUPAC: N-(2-chloro-6-methylphenyl)-2-[(2-methyl-6-piperazin-1-ylpyrimidin-4-yl)amino]-1,3-thiazole-5-carboxamide, MW: 443.95298 | MF: C20H22ClN7OS.
53: CID: 11676373, IUPAC: (3-chloro-2-fluorophenyl)-[4-[[6-(1,3-thiazol-2-ylamino)pyridin-2-yl]methyl]piperazin-1-yl]methanone, MW: 431.914163 | MF: C20H19ClFN5OS.
54: CID: 11684148, IUPAC: (3-chloro-2-fluorophenyl)-[4-[[6-[(5-chloro-1,3-thiazol-2-yl)amino]pyridin-2-yl]methyl]piperazin-1-yl]methanone, MW: 466.359223 | MF: C20H18Cl2FN5OS.
55: CID: 11700117, IUPAC: 2-[[6-(4-ethylpiperazin-1-yl)-2-methylpyrimidin-4-yl]amino]-N-[2-methyl-5-[[3-(trifluoromethyl)benzoyl]amino]phenyl]-1,3-thiazole-5-carboxamide, MW: 624.67975 | MF: C30H31F3N8O2S.
56: CID: 11707091, IUPAC: 2-[[2-methyl-6-(4-methylpiperazin-1-yl)pyrimidin-4-yl]amino]-N-[2-methyl-5-[[3-(trifluoromethyl)benzoyl]amino]phenyl]-1,3-thiazole-5-carboxamide, MW: 610.65317 | MF: C29H29F3N8O2S.
57: CID: 11714286, IUPAC: 2-[[5-[4-(2-hydroxyethyl)piperazin-1-yl]pyridin-2-yl]amino]-N-[2-methyl-5-[[3-(trifluoromethyl)benzoyl]amino]phenyl]-1,3-thiazole-5-carboxamide, MW: 625.66451 | MF: C30H30F3N7O3S.
58: CID: 11714353, IUPAC: 2-[[6-[4-(2-hydroxyethyl)piperazin-1-yl]-2-methylpyrimidin-4-yl]amino]-N-[2-methyl-5-[[3-(trifluoromethyl)benzoyl]amino]phenyl]-1,3-thiazole-5-carboxamide, MW: 640.67915 MF: C30H31F3N8O3S.
59: CID: 11752136, IUPAC: N-(2-chloro-6-methylphenyl)-2-[[5-[4-(2-hydroxyethyl)piperazin-1-yl]-2-methylpyrimidin-4-yl]amino]-1,3-thiazole-5-carboxamide, MW: 488.00554 | MF: C22H26ClN7O2S.
60: CID: 11772766, IUPAC: 4-[2-(3-chlorophenyl)ethylamino]-2-pyridin-4-yl-1,3-thiazole-5-carboxamide, MW: 358.8452 | MF: C17H15ClN4OS.
61: CID: 11775143, IUPAC: N-(2-chloro-6-methylphenyl)-2-[(2-methyl-6-morpholin-4-ylpyrimidin-4-yl)amino]-1,3-thiazole-5-carboxamide, MW: 444.93774 | MF: C20H21ClN6O2S.
62: CID: 11854012, IUPAC: 2-[4-[6-[[5-[(2-chloro-6-methylphenyl)carbamoyl]-1,3-thiazol-2-yl]amino]-2-methylpyrimidin-4-yl]piperazin-1-yl]acetic acid, MW: 501.98906 | MF: C22H24ClN7O3S.
63: CID: 11854269, IUPAC: 2-[4-[6-[[5-[(2-chloro-6-methylphenyl)carbamoyl]-1,3-thiazol-2-yl]amino]-2-methylpyrimidin-4-yl]piperazin-1-yl]ethyl hydrogen sulfate, MW: 568.06874 | MF: C22H26ClN7O5S2.
64: CID: 11854270, IUPAC: N-(2-chloro-6-methylphenyl)-2-[[6-[2-(2-hydroxyethylamino)ethylanimo]-2-methylpyrimidin-4-yl]amino]-1,3-thiazole-5-carboxamide, MW: 461.96826 MF: C20H24ClN7O2S
65: CID: 11854271, IUPAC: 2-[[6-(2-aminoethylamino)-2-methylpyrimidin-4-yl]amino]-N-(2-chloro-6-methylphenyl)-1,3-thiazole-5-carboxamide, MW: 417.9157 | MF: C18H20ClN7OS.
66: CID: 11854272, IUPAC: 2-[[2-[4-[6-[[5-[(2-chloro-6-methylphenyl)carbamoyl]-1,3-thiazol-2-yl]amino]-2-methylpyrimidin-4-yl]piperazin-1-yl]acetyl]amino]ethanesulfonic acid, MW: 609.12066 MF: C24H29ClN8O5S2.
67: CID: 11854533, IUPAC: N-(2-chloro-4-hydroxy-6-methylphenyl)-2-[[6-[4-(2-hydroxyethyl)piperazin-1-yl]-2-methylpyrimidin-4-yl]amino]-1,3-thiazole-5-carboxamide, MW: 504.00494 | MF: C22H26ClN7O3S.
68: CID: 11854534, IUPAC: N-[2-chloro-6-(hydroxymethyl)phenyl]-2-[[6-[4-(2-hydroxyethyl)piperazin-1-yl]-2-methylpyrimidin-4-yl]amino]-1,3-thiazole-5-carboxamide, MW: 504.00494 | MF: C22H26ClN7O3S.
69: CID: 11854535, IUPAC: N-(2-chloro-6-methylphenyl)-2-[[6-[4-(2-hydroxyethyl)-4-oxidopiperazin-4-ium-1-yl]-2-methylpyrimidin-4-yl]amino]-1,3-thiazole-5-carboxamide, MW: 504.00494 | MF: C22H26ClN7O3S.
70: CID: 11854536, IUPAC: 2-[4-[6-[[5-[(2-chloro-6-methylphenyl)carbamoyl]-1,3-thiazol-2-yl]amino]-2-methylpyrimidin-4-yl]-1-oxidopiperazin-1-ium-1-yl]acetic acid, MW: 517.98846 | MF: C22H24ClN7O4S.
71: CID: 11949914, IUPAC: 4-[[2-(5-chloro-2-fluorophenyl)-5-dimethylaminopyrimidin-4-yl]amino]-N-[2-(2-hydroxyethylamino)ethyl]pyridine-3-carboxamide, MW: 473.931003 | MF: C22H25ClFN7O2.
72: CID: 11951866, IUPAC: 4-[[2-(5-chloro-2-fluorophenyl)-5-pyrrolidin-1-ylpyrimidin-4-yl]amino]-N-(2-hydroxyethyl)pyridine-3-carboxamide, MW: 456.900483 | MF: C22H22ClFN6O2.
73: CID: 11952045, IUPAC: 4-[[2-(5-chloro-2-fluorophenyl)-5-pyrrolidin-1-ylpyrimidin-4-yl]amino]-N-[(2S)-2-hydroxypropyl]pyridine-3-carboxamide, MW: 470.927063 | MF: C23H24ClFN6O2.
74: CID: 15979866, IUPAC: 5-[2-[[4-(4-acetylpiperazin-1-yl)pyridin-2-yl]amino]-1,3-thiazol-5-yl]-N-methylpyridine-3-carboxamide, MW: 437.51802 | MF: C21H23N7O2S.
75: CID: 15980109, IUPAC: N-(2-aminoethyl)-5-[2-[(4-morpholin-4-ylpyridin-2-yl)amino]-1,3-thiazol-5-yl]pyridine-3-carboxamide, MW: 425.50732 | MF: C20H23N702S
76: CID: 15980233, IUPAC: N-(2-hydroxyethyl)-5-[2-[(4-morpholin-4-ylpyridin-2-yl)amino]-1,3-thiazol-5-yl]pyridine-3-carboxamide, MW: 426.49208 MF: C20H22N6O3S.
77: CID: 15980347, IUPAC: N-(2-methylaminoethyl)-5-[2-[(4-morpholin-4-ylpyridin-2-yl)amino]-1,3-thiazol-5-yl]pyridine-3-carboxamide, MW: 439.5339 | MF: C21H25N7O2S.
78: CID: 15980351, IUPAC: 5-[2-[[4-[4-(2-hydroxyacetyl)piperazin-1-yl]pyridin-2-yl]amino]-1,3-thiazol-5-yl]-N-(2,2,2-trifluoroethyl)pyridine-3-carboxamide, MW: 521.51539 | MF: C22H22F3N703S.
79: CID: 15982537, IUPAC: (3-chloro-2-fluorophenyl)-[4-[6-[(5-fluoro-1,3-thiazol-2-yl)amino]-5-methylpyridin-2-yl]piperazin-1-yl]methanone, MW: 449.904626 | MF: C20H18ClF2N5OS.
80: CID: 16034848, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 2,3-dihydroxybutanedioic acid, MW: 638.09238 | MF: C26H32ClN7O8S.
81: CID: 16037977, IUPAC: N-(2-chloro-6-methylphenyl)-2-[[6-[4-(2-hydroxyethyl)piperazin-1-yl]-5-methylpyrimidin-4-yl]amino]-1,3-thiazole-5-carboxamide, MW: 488.00554 | MF: C22H26ClN7O2S.
82: CID: 16061431, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 4-[(4-methylpiperazin-1-yl)methyl]-N-[4-methyl-3-[(4-pyridin-3-ylpyrimidin-2-yl)amino]phenyl]benzamide, MW: 981.60828 | MF: C51H57ClN14O3S.
83: CID: 16223227, IUPAC: but-2-enedioic acid; N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 604.0777 | MF: C26H30ClN7O6S.
84: CID: 16223228, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrobromide, MW: 568.9174748 | MF: C22H27BrClN7O2S.
85: CID: 16223229, IUPAC: but-2-enedioic acid; N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 604.0777 | MF: C26H30ClN7O6S.
86: CID: 16223316, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; methanesulfonic acid, MW: 584.1112 | MF: C23H30ClN7O5S2.
87: CID: 16223317, IUPAC: N-(2-chloro-6-methylphonyl)-2-[[6-[4-(2-hydroxyethyl)piperazin-1-yl]-2-methylpyrimidin-4-yl]amino]-1,3-thiazole-5-carboxamide; phosphoric acid, MW: 586.000721 | MF: C22H29ClN7O6PS.
88: CID: 16223318, IUPAC: N-(2-chloro-6-methylphenyl)-2-[[6-[4-(2-hydroxyethyl)piperazin--yl]-2-methylpyrimidin-4-yl]amino]-1,3-thiazole-5-carboxamide; 2-hydroxybenzoic acid, MW: 626.12628 | MF: C29H32ClN7O5S.
89: CID: 16223319, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; sulfuric acid, MW: 586.08402 | MF: C22H28ClN7O6S2.
90: CID: 16223320, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 4-methylbenzenesulfonic acid, MW: 660.20716 | MF: C29H34ClN7O5S2.
91: CID: 16584134, AKE-PB223730486, IUPAC: N-(4-chlorophenyl)-2-[(4,5-dimethyl-1,3-thiazol-2-yl)amino]-4-methylpyrimidine-5-carboxamide, MW: 373.85984 | MF: C17H16ClN5OS.
92: CID: 16584137, AKE-PB223730492, IUPAC: N-(3-chlorophenyl)-2-[(4,5-dimethyl-1,3-thiazol-2-yl)amino]-4-methylpyrimidine-5-carboxamide, MW: 373.85984 I MF: C17H16ClN5OS.
93: CID: 16584139, AKE-PB223730496, IUPAC: 2-[(4,5-dimethyl-1,3-thiazol-2-yl)amino]-4-methyl-N-(2-methylphenyl)pyrimidine-5-carboxamide, MW: 353.44136 | MF: C18H19N5OS.
94: CID: 16655683, IUPAC: 2-[(6-chloro-2-methylpyrimidin-4-yl)amino]-N-(2,6-dichlorophenyl)-1,3-thiazole-5-carboxamide, MW: 414.6968 | MF: C15H10Cl3N5OS.
95: CID: 16655839, IUPAC: N-(2,6-dichlorophenyl)-2-[[6-[4-(2-hydroxyethyl)piperazin-1-yl]-2-methylpyrimidin-4-yl]amino]-1,3-thiazole-5-carboxamide, MW: 508.42402 | MF: C21H23Cl2N7O2S.
96: CID: 16660745, IUPAC: N-(4-fluorophenyl)-4-(2-hydroxyethylamino)-6-methylsulfanyl-2-pyridin-4-ylpyrimidine-5-carboxamide, MW: 399.441923 | MF: C19H18FN502S.
97: CID: 16660747, IUPAC: N-(4-ethylphenyl)-4-(2-hydroxyethylamino)-6-methylsulfanyl-2-pyridin-4-ylpyrimidine-5-carboxamide, MW: 409.50462 | MF: C21H23N5O2S.
98: CID: 16660907, IUPAC: 4-(2-hydroxyethylamino)-N-(4-methylphenyl)-6-methylsulfanyl-2-pyridin-4-ylpyrimidine-5-carboxamide, MW: 395.47804 | MF: C20H21N5O2S.
99: CID: 16661063, IUPAC: N-(4-chlorophenyl)-4-(2-hydroxyethylamino)-6-methylsulfanyl-2-pyridin-4-ylpyrimidine-5-carboxamide, MW: 415.89652 | MF: C19H18ClN5O2S.
100: CID: 16661212, IUPAC: N-(2,4-dimethylphenyl)-4-(2-hydroxyethylamino)-6-methylsulfanyl-2-pyridin-4-ylpyrimidine-5-carboxamide, MW: 409.50462 | MF: C21H23N502S.
101: CID: 16661214, IUPAC: 4-(1-hydroxybutan-2-ylamino)-N-(4-methylphenyl)-6-methylsulfanyl-2-pyridin-4-ylpyrimidine-5-carboxamide, MW: 423.5312 | MF: C22H25N502S.

### NEPOR: Combined Prognostic and Therapeutic Value in Cancer Treatment.

Without being bound by theory, the observation that EPO treated patients often have poorer survival outcomes (at least in some cancers) means that treatment of these patients with a NEPOR targeted therapy provides a pharmacogenetic approach to targeted cancer treatment providing tumour tissue can be assessed for expression of NEPOR. Such a therapeutic perspective changes the balance in favour of performing biopsies under all suitable circumstances - meaning for cancers where EPOR, EPH-B4 and/or EphrinA1 are typically expressed.

The present disclosure further provides a method for imaging tumour tissue that is susceptible to enhanced survival in response to EPO treatment, comprising administering an anti-NEPOR antibody or NEPOR binding peptide linked to a radio-ligand or other imaging agent, and measuring for tissue distribution and location of the radio-ligand or other imaging agent.

If a tumour is NEPOR positive, then EPO is contraindicated and a NEPOR targeted therapy is administered. If NEPOR is not present, then it is safe to administer EPO. Both outcomes stand to benefit patient outcome, regardless of whether a patient is NEPOR positive or negative. Again, this shifts the balance in favour of performing routine biopsies.

In one embodiment the disclosure relates to an siRNA molecule specific to EPH-B4 and/or Ephrin A1 for use in treating a cancer patient that is or will receive EPO. EPH-B4 siRNAs and Antisense Oligodeoxynucleotides

Various EphB4-specific anti-sense phosphorothioate-modified oligodeoxynucleotides (ODNs) and siRNA may be synthesized from (e.g. by Qiagen. The most active antisense ODN and siRNA that knocks down EphB4 expression in the transiently transfected 293T cell line is chosen. The antisense ODN that may be used is AS-10 which spans nucleotides 1980 to 1999 with a sequence 5'-ATG GAG GCC TCG CTC AGA AA-3' (SEQ ID NO. 217). To eliminate cytokine responses, the cytosine at the CpG site may be methylated (AS-10M) without any loss in EphB4 knockdown efficiency (data not shown). Scrambled ODNs containing random nucleotide sequence and a similar CpG site, 5'-TAC CTG AAG GTC AGG CGA AC-3' (SEQ ID NO. 218), may be used as control. siRNA 465 corresponding to the sequences 5'-GGU GAA UGU CAA GAC GCU GUU-3' (SEQ ID NO. 219) and 3'-UUC CAC UUA CAG UUC UGC GAC-5' (SEQ ID NO. 220) may be used for RNA interference. Control siRNA may be generated by mutating three bases in this sequence to effectively abrogate EphB4 knockdown. This mutated siRNA (siRNAΔ) had the sequences 5'-AGU UAA UAU CAA GAC GCU GUU-3' (SEQ ID NO. 221) and 3'-UUU CAA UUA UAG UUC UGA GAC-5' (SEQ ID NO. 222). Additionally, siRNA directed against green fluorescent protein with sequences 5'-CGC UGA CCC UGA AGU UCA TUU-3' (SEQ ID NO. 223) and 3'-UUG CGA CUG GGA CUU CAA GUA-5' (SEQ ID NO. 224) may be used as a negative control.

siRNA may be obtained from numerous companies in particular Sigma Aldrich: The siRNA's from the sigma catalogue predicted using Rosetta siRNA design algorithm
**EPHRIN A1**

| siRNA_ID | entrezgene_ID | approx_start_nucleotide |
|---|---|---|
| SASI_Hs01_00211016 | NM_004428 | 247 |
| SASI_Hs01_00211017 | NM_004428 | 223 |
| SASI_Hs01_00211018 | NM_004428 | 248 |
| SASI_Hs01_00211019 | NM_004428 | 1071 |
| SASI_Hs01_00211020 | NM_004428 | 256 |
| SASI_Hs01_00211021 | NM_004428 | 208 |
| SASI_Hs01_00211022 | NM_004428 | 829 |
| SASI_Hs01_00211023 | NM_004428 | 1015 |
| SASI_Hs01_00211024 | NM_004428 | 846 |
| SASI_Hs01_00211025 | NM_004428 | 225 |
| SASI_Hs01_00071683 | NM_182685 | 248 |
| SASI_Hs01_00071684 | NM_182685 | 214 |
| SASI_Hs01_00071685 | NM_182685 | 242 |
| SASI_Hs01_00071686 | NM_182685 | 1000 |
| SASI_Hs01_00071687 | NM_182685 | 263 |
| SASI_Hs01_00071688 | NM_182685 | 203 |
| SASI_Hs01_00071689 | NM_182685 | 769 |
| SASI_Hs01_00071690 | NM_182685 | 948 |
| SASI_Hs01_00071691 | NM_182685 | 778 |
| SASI_Hs01_00071692 | NM_182685 | 227 |

**EPHB4**

| siRNA_ID | entrezgene_ID | approx_start_nucleotide |
|---|---|---|
| SASI_Hs01_00039855 | NM_004444 | 1756 |
| SASI_Hs01_00039856 | NM_004444 | 577 |
| SASI_Hs01_00039857 | NM_004444 | 1373 |
| SASI_Hs01_00039858 | NM_004444 | 2290 |
| SASI_Hs01_00039859 | NM_004444 | 2318 |
| SASI_Hs01_00039860 | NM_004444 | 2353 |
| SASI_Hs01_00039861 | NM_004444 | 2898 |
| SASI_Hs01_00039862 | NM_004444 | 2245 |
| SASI_Hs01_00039863 | NM_004444 | 1679 |
| SASI_Hs01_00039864 | NM_004444 | 1416 |

### Antibodies to NEPOR

The present disclosure includes several antibodies that bind to NEPOR components. The following Table 6 provides a list of such antibodies and their availability.

**Table 6**

| **Table 6** | | | | | |
|---|---|---|---|---|---|
| ***Company*** | ***Item*** | ***Antigen*** | ***Catalog Number*** | ***Applications*** | ***Type*** |
| | ***EPOR*** | | | | |
| Abcam | Goat Anti-EPO Receptor Polyclonal Antibody, Unconjugated | EPOR | ab10653 | ELISA, WB | polyclonal |
| ABR-Affinity BioReagents | Mouse Anti-Human EPOR Monoclonal Antibody, Unconjugated, Clone 3D10 | EPOR | MA1-51823 | WB, ELISA | Monoclonal |
| Abnova Corporation | Mouse Anti-Human EPOR Monoclonal Antibody, Unconjugated, Clone 3D10 | EPOR | H00002057-M01 | WB, Capture ELISA | Monoclonal |
| Abcam | Goat Anti-Human EPO Receptor Polyclonal Antibody, Unconjugated | EPOR | ab27497 | ELISA, WB | Polyclonal |
| Abcam | Mouse Anti-Human EPO Receptor Monoclonal Antibody, Unconjugated, Clone MM-0031-6G7 | EPOR | ab56310 | WB | Monoclonal |
| ABR-Affinity BioReagents | Mouse Anti-Human EPOR Polyclonal Antibody, Unconjugated | EPOR | PA1-51822 | WB | Polyclonal |
| IMGENEX | Goat Anti-Human EPOR Polyclonal Antibody, Unconjugated | EPOR | IMG-3771 | WB, ELISA | Polyclonal |
| Lifespan Biosciences | Rabbit Anti-Human Erythropoietin Receptor (EPOR) Polyclonal Antibody, Unconjugated | EPOR | LS-C6720 | ELISA | Polyclonal |
| GeneTex | Mouse Anti-Human EPOR Monoclonal Antibody, Unconjugated, Clone 3D10 | EPOR | GTX91710 | ELISA, WB | Monoclonal |
| Lifespan Biosciences | Rabbit Anti-Human EPOR Polyclonal Antibody, Unconjugated | EPOR | LS-C6719-100 | ELISA | Polyclonal |
| Novus Biologicals | Mouse Anti-Human EPOR Polyclonal Antibody, Unconjugated | EPOR | H00002057-A01 | | Polyclonal |
| Novus Biologicals | Mouse Anti-Human EPOR Monoclonal Antibody, Unconjugated, Clone 3D10 | EPOR | H00002057-M01 | ELISA, WB | Monoclonal |
| Lifespan Biosciences | Sheep Anti-Human Erythropoietin Receptor (EPOR) Polyclonal Antibody, Unconjugated | EPOR | LS-C6718 | ELISA, WB | Polyclonal |
| Lifespan Biosciences | Sheep Anti-Human Erythropoletin Receptor (EPOR) Polyclonal Antibody, Unconjugated | EPOR | LS-C6716 | | Polyclonal |
| Lifespan Biosciences | Sheep Anti-Human EPOR Polyclonal Antibody, Unconjugated | EPOR | LS-C6717-50 | ELISA | Polyclonal |
| Santa Cruz Biotechnology, Inc. | Rabbit Anti-Human EpoR (C-20) Polyclonal Antibody, Unconjugated | EpoR (C-20) | sc-695 | WB, IP, IF, ICH. | Polyclonal |
| Santa Cruz Biotechnology, Inc. | Rabbit Anti-EpoR (M-20) Polyclonal Antibody, Unconjugated | EpoR (M-20) | sc-697 | WB, IP, IF, ICH. | Polyclonal |
| Santa Cruz Biotechnology, Inc. | Rabbit Anti-EpoR Polyclonal Antibody, Unconjugated | EpoR | sc-5624 | WB, IP, IF, ICH. | Polyclonal |

| | ***EPH-B4*** | | | | |
|---|---|---|---|---|---|
| Abgent | Rabbit Anti-EPH-B4 C-term RB1659-1660 Polyclonal Antibody, Unconjugated | EPH-B4 C-term | AP7625a | ELISA; IHC. | Polyclonal |
| ABR-Affinity BioReagents | Rabbit Anti-Human EPH-B4 Polyclonal Antibody, Unconjugated | EPH-B4 | PA1-24241 | WB | Polyclonal |
| ABR-Affinity BioReagents | Mouse Anti-Human EPH-B4 Monoclonal Antibody, Unconjugated, Clone 1D1 | EPH-B4 | MA1-51815 | ELISA | Monoclonal |
| AbD Serotec | Human Anti-Human EPH-B4 Monoclonal Antibody, Unconjugated, Clone 1327 | EPH-B4 | HCA001 | IHC, WB, ELISA | Monoclonal |
| AbD Serotec | Human Anti-Human EPH-B4 Monoclonal Antibody, Unconjugated, Clone 3934 | EPH-B4 | HCA025 | IHC, WB, ELISA | Monoclonal |
| Invitrogen | Anti-EPH-B4 receptor Monoclonal Antibody, Unconjugated, Clone 3D7F8 | EPH-B4 | 35-2900 | WB, ELISA | Monoclonal |
| GeneTex | Rabbit Anti-EPH-B4 Polyclonal Antibody, Unconjugated | EPH-B4 | GTX77656 | WB | Polyclonal |
| Invitrogen | Mouse Anti-EPH-B4 Receptor Monoclonal Antibody" Clone 3D7G8 | EPH-B4 | 182394 | IHC(FFPE) | Monoclonal |
| Invitrogen | Anti-Eph Receptor Sampler Pack Antibody, | EPH-B4 | 901100 | | |
| GeneTex | Mouse Anti-Human EPH-B4 Monoclonal Antibody, Unconjugated, Clone 1D1 | EPH-B4 | GTX91629 | ELISA | Monoclonal |
| Invitrogen | Mouse Anti-Human EPH-B4 Receptor Monoclonal Antibody" Clone 3D7G8 | EPH-B4 | 371800 | WB ELISA IP, IHC | Monoclonal |
| Novus Biologicals | Mouse Anti-Human EPH-B4 Monoclonal Antibody, Unconjugated, Clone 1D1 | EPH-B4 | H00002050-M01 | | Monoclonal |
| R&D Systems | Goat Anti-Human EPH-B4 Polyclonal Antibody, Unconjugated | EPH-B4 | AF3038 | FC, IHC, WB | Polyclonal |
| Raybiotech, Inc. | Human Anti-Human EPH-B4, (packaged with HRP-Conjugated Secondary Antibody); Monoclonal Antibody, Unconjugated | EPH-B4 | DS-MB-01224 | | Monoclonal |
| R&D Systems | Rat Anti-Human EPH-B4 Monoclonal Antibody, Unconjugated, Clone 395810 | EPH-B4 | MAB3038 | FC, IHC, WB | Monoclonal |
| Santa Cruz Biotechnology, Inc. | Goat Anti-EPH-B4 Polyclonal Antibody, Unconjugated | EPH-B4 | sc-7284 | WB, IF | Polyclonal |
| Santa Cruz | Goat Anti-EPH-B4 Polyclonal Antibody, | EPH-B4 | sc-7285 | WB, IF | Polyclonal |
| Biotechnology, Inc. Santa Cruz Biotechnology, Inc. | Unconjugated Rabbit Anti-EPH-B4 Polyclonal Antibody, Unconjugated | EPH-B4 | sc-5536 | WB, IF | Polyclonal |
| Raybiotech, Inc. | Human Anti-Human EPH-B4, (packaged with HRP-Conjugated Secondary Antibody); Monoclonal Antibody, Unconjugated | EPH-B4 | DS-MB-01225 | | Monoclonal |

| | ***EFNA1*** | | | | |
|---|---|---|---|---|---|
| Invitrogen | Anti-Ephrin A1 Polyclonal Antibody, Unconjugated, Clone ZMD.39 | EFNA1 | 34-3300 | | Polyclonal |
| Novus Biologicals | Mouse Anti-Human EFNA1 Monoclonal Antibody, Unconjugated, Clone 3C6 | EFNA1 | H00001942-M01 | | Monoclonal |
| Santa Cruz Biotechnology, Inc. | Rabbit Anti-ephrln-A1 (V-18) Polyclonal Antibody, Unconjugated | EFNA1 (V-18) | sc-911 | WB, IF | Polyclonal |
| Santa Cruz Biotechnology, Inc. | Rabbit Anti-ephrin-A1 Polyclonal Antibody, Unconjugated | EFNA1 | sc-20719 | WB, IP, IF | Polyclonal |
| Abcam | Rabbit Anti-Human Ephrin A1 Receptor Polyclonal Antibody, Unconjugated | EFNA1 | ab37857 | ELISA, IHC, WB | Polyclonal |
| GeneTex | Mouse Anti-Human EFNA1 Monoclonal Antibody, Unconjugated, Clone 3C7 | EFNA1 | GTX91614 | | Monoclonal |

| | ***EFNB2*** | | | | |
|---|---|---|---|---|---|
| Novus Biologicals | Mouse Anti-Human EFNB2 Polyclonal Antibody, Unconjugated | EFNB2 | H00001948-A01 | | Polyclonal |
| Santa Cruz Biotechnology, Inc. | Rabbit Anti-ephrin-B2 (P-20) Polyclonal Antibody, Unconjugated | EFNB2 (P-20) | sc-1010 | WB, IF | Polyclonal |
| Santa Cruz Biotechnology, Inc. | Goat Anti-ephrin-B2 Polyclonal Antibody, Unconjugated | EFNB2 | sc-19227 | WB, IF, IP | Polyclonal |
| Santa Cruz Biotechnology, Inc. | Rabbit Anti-ephrin-B2 Polyclonal Antibody, Unconjugated | EFNB2 | sc-15397 | WB, IF, IP | Polyclonal |

### EXAMPLES

### Example 1

A variety of sequence analysis approaches were pursued, including the search for homologues of the EPO binding domain from EPOR, a domain analysis based method combined with text-mining, and EPO homology analysis followed by text-mining of resultant hits. Only that part of the human proteome exposed to the extracellular environment was investigated. This allowed a focus on homologies that were significant, though possibly overlooked within the context of a complete proteome analysis. This formed the XtraCell database. The XtraCell database performed a signal peptide and transmembrane prediction for the entire human proteome. All proteins possessing at least one of these features were stored in a first version of the extracellular database. Given that not all extracellular proteins actually possess either of these features, there was extracted a list of protein domains specific to the extracellular environment from a SMART (Simple Modular Architecture Research Tool -.SMART is a well-known protein domain database with a strong bias towards domains contained in signalling proteins.) These were then screened against the human proteome using the HMMER algorithm. HMMER is a freely distributable implementation of profile HMM software for protein sequence analysis - Profile hidden Markov models (profile HMMs) can be used to do sensitive database searching using statistical descriptions of a sequence family's consensus. All hits were added to the XtraCell database and the dataset made non-redundant. A final version of the XtraCell database was established for the purpose of these EPO specific analyses (Figure 3).

### Example 2

This example illustrates a domain-based approach coupled with a text-mining and genome-wide analysis. The operating theory was that any novel EPO receptor involved in mediating EPO's neuroprotective effect might also possess the two membrane proximal fibronectin 3 (FN3) domains (as found in EPOR), whilst at the same time being hypoxia inducible. Such conserved domain architecture is compatible with both a heterodimeric complex containing EPOR and/or an independent hypoxia inducible homodimeric receptor. All proteins containing two membrane proximal FN3 domains from the human proteome (84 in all) were extracted and asked whether there was any evidence for their role in response to low oxygen conditions/ischaemia. The latter analysis was performed using a text-mining approach that encompasses the use of comprehensive protein synonyms, and concepts such as hypoxia and ischaemia. Of the 84 proteins containing the 2FN3-TM domain composition, only four showed evidence for mediating response to low oxygen conditions: EPH-B4, IL6RB, TIE1 and GM-CSF. Apart from EPH-B4, the cellular role of each of these proteins has been studied and an important role in response to hypoxia established.

Direct examination of the EPH-B4 locus revealed that it directly juxtaposes the EPO locus, albeit on the opposite strand. This close genomic association was conserved in all vertebrate genomes examined. The need for immediate response of cells to low oxygen conditions and thus the need to co-transcribe/-translate key effector molecules was seen. Moreover, such genomic co-localisation of functionally associated molecules is seen for other receptor:ligand partners (*e.g*. MST1 and its receptor MST1R: see http://www.ensembl.org/Homo_sapiens/contigview?gene=OTTHUMG00000136237;db=vega ); (see also Figure 4).

To examine this possibility in greater detail, we analysed the promoter, 5' UTR and 3' UTR regions of EPO, EPHB4 and EPOR in search of hypoxia inducible factor binding sites. Here we utilised the 'match' algorithm from Genomatix, searching for strict conservation of the core binding site residues and at least 90% conservation of non-core residues. We found that the EPO and EPH-B4 loci possessed numerous hypoxia-inducible transcription factor binding sites. In contrast, the EPOR gene regulatory regions were found to be complete devoid of such HIF-1 binding sites, again hinting at a possible role for EPHB4 as a hypoxia inducible EPO receptor (see Figure 4).

### Example 3

This example shows the homology-based approach using human extra-cellular database. Here we sought to directly identify regions of EPO binding activity in other proteins, by direct comparison to the EPO binding domain of EPOR. The region of EPOR responsible for EPO binding was thus extracted and used to identify homologies with proteins of the XtraCellDB. This specially developed database holds distinct advantages in that all homologies identified are to human extracellular proteins, thus avoiding the need to assess spurious homologies to irrelevant intracellular species. Analysis of resultant homologues revealed a striking homology to the Ephrin A1 protein, within the top four hits. Given what we had learned about EPH-B4's possible role in EPO signalling we decided to assess this homology in greater detail using the Swiss-model protein structure package. Here we employed information derived from the co-crystal structure of Ephrin A5 in association with EphB2 and compared it to EPO:EPOR co-crystal information. Conservation of key residues in structurally aligned positions allowed us to conclude a firm structural basis for association between Ephrin A1 and EPO. Moreover, the realisation that both EphrinA1 and EPHB4 possess a putative affinity for EPO, suggests a more exciting functional context for eprhin biology than heretofore recognised (See Figure 6).

### Example 4

This example provides wet lab or *in vivo* data that validates the bioinformatics analysis provide in Examples 1-3 herein. *In vivo* validation of EPH-B4's role in EPO signalling has focussed on the neuroprotective aspect of EPO's function, with a bias towards the hypothesis that EPH-B4 and EPOR are heterodimeric partners. The following table lists the validation experiments for which data are available (see Table 7).

### LAB-BASED validation experiments

| **Method** | **Goal** | **Result** |
|---|---|---|
| **Immunohistochemistry** | To assess the expression of EPHB4 protein in brain and how it relates to EPOR expression. | **Precipitation stainings on adult rodent brain showed that EPHB4 was expressed in adult neurons in the same pattern as EPO receptor. Staining in hippocampus showed co-expression of EPOR and EPHB4 (see** **figure 6****). Strikingly, the staining was restricted to particular cells within the field of tissue.** |
| Co-IP | **Exogenous expression of EPOR/EPHB4 in COS cells. Co-ip with EpoR-and EphB4-antibodies => WB analysis.** | **Positive. Use of EPOR antibody successfully Co-IP's EPHB4 protein.** |

Immunohistochemistry. For immunofluorescence, sections of paraffin-embedded rat brain tissues (2 µm) were deparaffinated and microwaved (citrate buffer at 600W for 15 min). Afterwards, sections were incubated simultaneously with the EpoR antiserum (1:200; sc-697, Santa Cruz Biotechnology) and the EphB4 antibody (1:100; AF446, R&D Systems) at 4°C over night. After adding a biotinylated anti-goat secondary antibody (1:200; Dianova), sections were incubated with Streptavidin-coupled Alexa Fluor 555 (1:200; Invitrogen, Karlsruhe, Germany) and a FITC-coupled anti-rabbit secondary antibody (1:200; Dianova). The nuclei were counterstained with Hoechst 33342 (1:10,000; Molecular Probes). Controls for the stainings included omission of primary antibodies, fluorophor swapping, and single-fluorescence stainings. Images were obtained with an Olympus IX-81 microscope with narrow-bandwidth monochromator excitation (Polychrome IV, Till Photonics, Gräfelfing, Germany) and appropriate filters.

Both EPHB4 and EPOR displayed a striking co-localisation when assessed in rat brain tissue sections. Without being bound by theory, this co-expression suggests functional coupling of both receptors.

Co-immunoprecipitation. The principle of an immunoprecipitation is an antibody (monoclonal or polyclonal) against a specific target antigen is allowed to form an immune complex with that target in a sample, such as a cell lysate. The immune complex is then captured on a solid support to which either Protein A or Protein G has been immobilized (Protein A or G binds to the antibody, which is bound to its antigen). The process of capturing this complex from the solution is referred to as precipitation. Any proteins not "precipitated" by the immobilized Protein A or G support are washed away. Finally, components of the bound immune complex (both antigen and antibody) are eluted from the support and analyzed by SDS-PAGE (gel electrophoresis), often followed by Western blot detection to verify the identity of the antigen.

Traditional immunoprecipitation involves the following steps:
1. Form the antigen-antibody complex (immune complex) by incubating specific antibody with the antigen-containing sample for 1 hour to several hours.
2. Capture the immune complex on an immobilized Protein A or Protein G agarose gel support by incubation for 0.5-2 hours.
3. Remove any non-bound protein (non-immune complex sample components) from the precipitated complex by washing gel support with additional sample buffer.
4. Boil gel support in reducing SDS-PAGE sample loading buffer.
5. Recover sample eluted in loading buffer from gel support and analyze by SDS-PAGE.
6. Perform Western blot analysis, probing with antigen-specific antibody.

In a co-immunoprecipitation the target antigen precipitated by the antibody "co-precipitates" a binding partner/protein complex from a lysate, that is, the interacting protein is bound to the target antigen, which becomes bound by the antibody that becomes captured on the Protein A or G gel support. The assumption that is usually made when associated proteins are coprecipitated is that these proteins are related to the function of the target antigen at the cellular level.

Assessment of a putative EPHB4:EPOR association using co-immmunoprecipitation showed that both proteins were physically associated. Here, FLAG-tagged EPOR was co-expressed with EPH-B4 in COS cells and then immunoprecipitated using an a-FLAG antibody. As can be seen from Figure 8, EPHB4 was shown to co-immunoprecipitate in these experiments.

Human Fc Antibody Constructs. The Fc conjugate approach is most appropriate when dealing with dimeric cell surface receptors. Here the extracellular portion of EPHB4/EPOR was fused to an Fc fragment. This method has advantages due to its *in vivo* (therapeutic) viability and the fact that it optimally mimics the dimerised receptor state. Figure 9 highlights the Human constructs that are used to show EPHB4's/EphrinA1's affinity for EPO.

One of two alternatives can assay the interaction of the Fc constructs with EPO, including, for example, a protein array approach or a surface plasmon resonance analysis.

### Example 5

### Further in vitro and in vivo validation of NEPORs role in mediating EPO function.

In these experiments we sought to determine the response to erythropoietin (EPO) treatment in a panel of ovarian cancer cell lines. This would be mediated by the expression of erythropoietin receptor (EPO as well as two receptors that potentially may be able to activate signaling pathways in response to EPO binding, EPH-B4 and Ephrin A1. It was first necessary to characterize the expression of these receptors in a panel of ovarian cancer cell lines. First we collected RNA from each cell line and reverse transcribed them into cDNA. Using specific primers for each receptor we analyzed their RNA expression. As evident in the figures the expression of EPOR and EPH-B4 RNA is different in different cell lines suggesting changes in transcriptional regulation during tumorigenesis no significant changes were seen in the EphrinA1. It was then necessary to determine protein expression of these receptors in the panel. Again we see significant differences in the expression of the EPOR and EPH-B4 receptors though they do not coincide with the RNA expression suggesting there is changes in post transcriptional regulation of these receptors in the cell lines. We then categorized these expression changes particularly with regard to the EPOR and EPH-B4 to then analyze the response to EPO treatment. We analyzed their response to chemotherapy in conjunction with EPO. We found that particularly in the HeyA8 ovarian cancer cell line that EPO was able to abrogate the apoptosis induced by docetaxei. It was then necessary to analyze the activation of signaling pathways known to be activated by these receptors in response to EPO treatment. Three cell lines were starved for two hours to isolate their response to EPO. Cell lines with higher expression (HeyA8 and HeyA8 MDR) of the EPOR demonstrated activation of the MAPK/ERK pathway while cell lines that expressed higher EPH-B4 (SKOV3ip1) demonstrated increased activation of the AKT and STAT5b signaling pathways. We then sought to determine a EPO dose that optimized its tumor promoting effect *in vivo.* Female nude mice were injected i.p. with HeyA8 MDR (positive for both EPOR and EPH-B4). At day eight the mice were treated with increasing doses of EPO (10, 50, 100 U) every two days. Treatment continued until tumors became evident, the mice were then sacrificed and the tumor weight was determined. We saw an increase in tumor weight as compared to control in the mice treated with 10 and 50 U EPO. The differential expression of EphB4 in cell lines as well as the activation of particular signaling pathways suggested that it would also mediate the tumor promoting effect *in vivo.* To determine this we again injected mice with HeyA8 MDR cell lines i.p. At day eight we began treatment with EPO (50 U 3x week) in conjunction with siRNA specific to EPH-B4. As previously described EphB4 siRNA was able decrease tumor growth alone. Moreover, EPH-B4 siRNA also completely abrogated the EPO induced tumor growth.

The following are specific embodiments of this disclosure:
1. A method for assessing tumour tissue for expression of EPH-B4 and/or Ephrin A1, comprising:
   (a) isolating a tissue sample from an individual who is receiving or shall receive erythropoietin,
   (b) determining the level of expression of the EPH-B4 and/or Ephrin A1
   (c) correlating the presence of these component gene expression products to a negative physiological response to the treatment with erythropoietin.
2. The method according to 1, wherein the level of expression of the component genes (mRNA) is determined by a molecular biological technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF).
3. The method according to 1 or 2, wherein the individual is a cancer patient who is to be treated with erythropoietin or is being treated with erythropoietin.
4. The method according to any one of 1 to 3, wherein the presence of EPH-B4 and/or Ephrin A1 gene expression products is indicative of poorer loco-regional tumor control and poorer patient survival upon treatment with erythropoietin.
5. The method according to any one of 1 to 3, wherein the presence of a higher level of EPH-B4 and/or Ephrin A1 gene expression products is indicative of poorer loco-regional tumour control and poorer patient survival upon treatment with erythropoietin.
6. The method according to any one of 1 or 3 to 5, wherein the means for testing for the presence of the gene expression products are a protein array or binding to a mass microbalance instrument.
7. The method according to any one of 1 or 3 to 5, wherein the determination of the presence of the EPH-B4 and/or Ephrin A1 gene products is done by detecting the respective proteins with an immunoassay procedure, where the immunoassay procedure is selected from the group of immunoprecipitation, enzyme immunoassay (EIA), radioimmunoassay (RIA) or fluorescent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay.
8. The method according to 7, wherein the immunoassay is an ELISA.
9. The method according to any one of 1 to 8, wherein the tissue sample may be selected from the cancerous tissue or circulating cells derived from same or from a group of biological tissues and fluids such as blood, lymph, urine, cerebral fluid.
10. A prognostic method to stratify patients having a tumour as suitable (EPH-B4 and/or Ephrin A1) or non-suitable (EPH-B4 and/or Ephrin A1) for EPO treatment, comprising:
   (a) isolating a tissue sample from an individual who is receiving or shall receive erythropoietin;
   (b) determining the level of expression of the EPH-B4 and/or Ephrin A1 gene(s) component, a EPH-B4 and/or Ephrin A1 from the isolated tissue; and
   (c) correlating the presence of EPH-B4 and/or Ephrin A1 component gene expression products to a negative physiological response to the treatment with erythropoietin.
11. The prognostic method according to 10, wherein the level of expression of EPH-B4 and/or Ephrin A1 component genes is determined by a molecular biological technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF).
12. The prognostic method according to 10, wherein the determination of the presence of the EPH-B4 and/or Ephrin A1 gene products is done by detecting the respective protein with an immunoassay procedure, where the immunoassay procedure is selected from the group of ELISA, immunoprecipitation, enzyme immunoassay (EIA), radioimmunoassay (RIA) or fluorescent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a luciferase-assay.
13. The prognostic method according to any one of 10 to 12, wherein the tissue sample is selected from the cancerous tissue or circulating cells derived from same, or from a group of biological tissues and fluids such as blood, lymph, urine, cerebral fluid.
14. A method for imaging tumour tissue that is susceptible to enhanced survival in response to EPO treatment, comprising administering an anti-EPH-B4 and/or anti-Ephrin A1 antibody or EPH-B4 and/or Ephrin A1 binding peptide linked to a radio-ligand or other imaging agent, and measuring for tissue distribution and location of the radio-ligand or other imaging agent.
15. The method according to 14, wherein the anti-EPH-B4 and/or anti-Ephrin A1 antibody is a monoclonal or polyclonal antibody selected from the group of antibodies listed in Table 6.
16. A method for designing a compound which interferes with NEPOR's survival promoting activity, comprising:
   (a) providing the molecular makeup of the NEPOR species and providing amino acid sequences of a component NEPOR polypeptides;
   (b) using software comprised by the digital computer to design a chemical compound/protein construct which is predicted to bind to NEPOR; and
   (c) optionally designing protein constructs which mimic NEPOR in its dimerised/multimerised state (*e.g*. Fc constructs).
17. A method for identifying compounds that modulate NEPOR's tissue protective signalling activity, comprising (a) contacting a test compound with the NEPOR receptor complex; (b) measuring the level of tissue protective activity initiated by NEPOR activation; (c) identifying a test compound which increases or decreases the level of tissue protective NEPOR complex activity; (d) assaying the identified therapeutics for tissue protective activity mediated via NEPOR; and (e) assaying the identified therapeutics for NEPOR inhibitory activity.
18. The method of 17 wherein the tissue protective NEPOR receptor complex activity is measured by measuring the binding of the test compound to the NEPOR receptor complex.
19. The method of 18, wherein the test compound is labelled and binding of the labelled test compound to the tissue protective NEPOR receptor complex is measured by detecting the label attached to the test compound.
20. The method of 18 wherein the tissue protective NEPOR receptor complex activity is measured by measuring the binding of the test compound to the tissue protective NEPOR receptor complex.
21. A method for identifying compounds that modulate NEPOR's tissue protective signalling activity, comprising
   (a) contacting a test compound with the NEPOR receptor complex expressing cell;
   (b) measuring the level of tissue protective activity initiated by NEPOR activation in the cell;
   (c) identifying a test compound which increases or decreases the level of tissue protective NEPOR complex activity in a cell;
   (d) assaying the identified compounds for tissue protective activity mediated via NEPOR; and
   (e) assaying the identified therapeutics for NEPOR inhibitory activity.
22. The method of . 21, wherein assay in step (d) is a tissue protective NEPOR receptor complex activity is measured by a cell proliferation/differentiation assay.
23. The method of 22, wherein the cells in the cell proliferentiation/differentiation assay are recombinantly engineered to express EPH-B4, and/or EPOR, and/or Ephrin A1.
24. The method of 23, wherein the cells endogenously expresses an EPO receptor and are transformed with a nucleic acid comprising a nucleotide sequence that
   (i) is operably linked to a promoter, and
   (ii) encodes either EPH-B4 and/or Ephrin A1.
25. The method of 23, wherein the cells endogenously expresses EPH-B4 and/or Ephrin A1 and are transformed with a nucleic acid comprising a nucleotide sequence that (i) is operably linked to a promoter, and (ii) encodes an EPO receptor polypeptide.
26. A method for identifying a compound that modulates the interaction between a tissue protective NEPOR receptor complex and a tissue protective NEPOR receptor complex ligand, comprising:
   (a) contacting a tissue protective NEPOR receptor complex with one or more test compounds; and
   (b) measuring the tissue protective NEPOR receptor complex activity, whereby if the activity measured in (b) differs from the tissue protective NEPOR receptor complex activity in the absence of the one or more test compounds, then a compound that modulates the interaction between the tissue protective NEPOR receptor complex and the tissue protective NEPOR receptor complex ligand is identified.
27. The method of 17, 21 or 26, wherein the tissue protective NEPOR receptor complex activity is measured by cell proliferation or cell differentiation.
28. The method of 17, 21 or 26, wherein the tissue protective NEPOR receptor complex activity measured is the ability of the tissue protective NEPOR receptor complex to interact with a tissue protective NEPOR receptor complex ligand.
29. The method of 17, 21 or 26, wherein the step of assaying the identified compound for tissue protective activity comprises detecting the presence of nucleolin in the cell.
30. The method of 17, 21 or 26, wherein the step of assaying the identified compound for tissue protective activity comprises detecting or measuring an increased level of activity of neuroglobin or cytoglobin in a cell.
31. The method of 17, 21 or 26 wherein the tissue protective NEPOR receptor complex is in solution.
32. The method of claims 17, 21 or 26, wherein the tissue protective NEPOR receptor complex is in a cell.
33. The method of 17, 21 or 26, wherein the compound inhibits the binding of a tissue protective NEPOR receptor complex ligand to a tissue protective NEPOR receptor complex.
34. The method of 17, 21 or 26, wherein the compound enhances the binding of a tissue protective NEPOR receptor complex ligand to a tissue protective NEPOR receptor complex.
35. The method of 17, 21 or 26, wherein the tissue protective NEPOR receptor complex contacted in step (a) is on a cell surface.
36. The method of 17, 21 or 26, wherein the tissue protective NEPOR receptor complex is on an isolated cell membrane.
37. The method of 17, 21 or 26, wherein the tissue protective NEPOR receptor complex activity is compared to EPOR receptor activation to identify NEPOR specific compounds.
38. The method of 17, 21 or 26, wherein the tissue protective NEPOR receptor complex is immobilized to a solid surface.
39. The method of 38, wherein the solid surface is a microtiter dish.
40. The method of 38, wherein the solid surface is a chip.
41. A method for identifying a compound that binds a tissue protective NEPOR receptor complex, comprising:
   (a) contacting a test compound with a ligand-binding tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support; and
   (b) contacting a test compound with a ligand-binding EPOR receptor complex fragment comprising at least two EPO receptor extracellular domains fused to an Fc fragment attached to a solid support
   (c) removing unbound test compounds from the solid supports;
   (d) identifying the compound attached to the tissue protective NEPOR receptor complex fragment, but not the EPOR receptor complex (and vice versa), whereby a compound bound to the solid support is identified as a compound that binds specifically to a tissue protective NEPOR receptor complex or a compound that binds specifically to an EPOR receptor complex.
42. A method for identifying a compound that binds a tissue protective NEPOR receptor complex, comprising:
   (a) contacting a test compound with a ligand-binding tissue protective NEPOR receptor complex fragment comprising at least one EPO receptor or EPH-B4 receptor or Ephrin A1 receptor, extracellular domain fused to an Fc fragment attached to a solid support;
   (b) removing unbound test compounds from the solid supports;
   (c) identifying the compound attached to the tissue protective NEPOR receptor complex fragment, whereby a compound bound to the solid support is identified as a compound that binds specifically to a tissue protective NEPOR receptor complex.
43. A method for identifying a compound that binds to a tissue protective NEPOR receptor complex, comprising:
   (a) contacting a tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support with
      (i) a tissue protective NEPOR receptor complex ligand attached to a first label and
      (ii) an equivalent amount of a test compound attached to a second label under conditions conducive to binding;
   (b) removing unbound material from the tissue protective NEPOR receptor complex; and
   (c) detecting the level of the first and second labels wherein if the second label is present the compound binds the complex and if the level of the first label decreases relative to the level of the first label where the labelled ligand is contacted with a tissue protective NEPOR receptor complex under conditions conducive to binding in the absence of a test compound after removal of unbound material, then a compound that binds to a tissue protective NEPOR receptor complex is identified.
44. A method for identifying a compound that modulates the binding of a tissue protective NEPOR receptor complex ligand to a tissue protective NEPOR receptor complex, comprising:
   (a) contacting a tissue protective NEPOR receptor complex ligand with a tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support; in the presence of one or more test compounds under conditions conducive to binding; and
   (b) measuring the amount of tissue protective NEPOR receptor complex ligated bound to the tissue protective NEPOR receptor complex; whereby if the amount of bound tissue protective NEPOR receptor complex ligand measured in (b) differs from the amount of bound tissue protective NEPOR receptor complex ligand measured in the absence of the one or more test compounds, then a compound that modulates the binding of a tissue protective NEPOR receptor complex ligand to the tissue protective NEPOR receptor complex is identified.
45. The method of - 26 or 44, wherein the amount of bound tissue protective NEPOR receptor complex ligand is measured using a tissue protective NEPOR receptor complex ligand-specific antibody.
46. The method of 26 or 44 wherein the tissue protective NEPOR receptor complex ligand is labelled and binding of the tissue protective NEPOR receptor complex ligand to the tissue protective NEPOR receptor complex is measured by detecting the label attached to the tissue protective NEPOR receptor complex ligand.
47. The method of 26 or 44 wherein the tissue protective NEPOR receptor complex ligand is labelled and binding of the labelled ligand to the tissue protective NEPOR receptor complex is measured by detecting the label attached to the tissue protective NEPOR receptor complex ligand.
48. The method of 46, wherein the label is fluorescent.
49. The method of 16, 17, 21, 26, or 41 to 46 wherein the test compound is an antibody specific for the tissue protective NEPOR receptor complex.
50. The method of 16, 17, 21, 26, or 41 to 46 wherein the test compound is a small molecule.
51. The method of 16, 17, 21, 26, or 41 to 46 wherein the test compound is a peptide.
52. The method of 16, 17, 21, 26, or 41 to 46 wherein the test compound is a member of a library.
53. The method of 26 or 44, wherein the tissue protective NEPOR receptor complex ligand is EPO, or derivatives thereof.
54. The method of 16, 17, 21, 41, 42, or 43 wherein the compound binds the tissue protective NEPOR receptor complex.
55. The method of 26 or 44, wherein the compound binds the tissue protective NEPOR receptor complex ligand.
56. The method of 16,17,21, 26, or 41 to 46 wherein the tissue protective NEPOR receptor complex activity is compared to EPOR receptor activation to identify NEPOR specific compounds.
57. A method for identifying a compound that modulates a tissue protective activity in a mammal, comprising:
   (a) administering the compound to a first animal immediately following infliction of an injury, wherein the first animal endogenously expresses a tissue protective NEPOR receptor complex; and
   (b) administering the compound to a second animal immediately following infliction of the same injury as in step (a), wherein the second animal is deficient in expression of a tissue protective NEPOR receptor complex or components thereof; such that if recovery from the injury differs in the animal of step (a) as compared to the animal of step (b), a compound that modulates a tissue protective activity is identified.
58. A method for treating the negative patient outcomes associated with EPO stimulated NEPOR function, involving the co-administration of EPO with an inhibitor of NEPOR activity.
59. The method for treating EPO-induced tumour cell survival in NEPOR positive tumour tissue of 58, comprising administering an effective amount of anti-NEPOR antibody from claim 1, in combination with EPO, whereby such combinations permits haematopoietic signalling whilst switching off NEPOR signalling and thus EPO mediated cell survival signals on tumour cells.
60. The method for treating EPO-induced tumour cell survival in NEPOR positive tumour tissue of 58, further comprising administering an effective amount of EPHB4 tyrosine kinase inhibitor in combination with EPO, whereby such combinations permits haematopoietic signalling whilst switching off NEPOR signalling and thus EPO mediated cell survival signals on tumour cells.
61. The method for treating EPO-induced tumour cell survival in NEPOR positive tumour tissue of 58, further comprising administering an effective amount of anti-NEPOR siRNA's, in combination with EPO, whereby such combinations permits haematopoietic signalling whilst switching off NEPOR signalling and thus EPO mediated cell survival signals on tumour cells.
62. A method for decreasing the survival of tumour cells or tissues in a human comprising administering a therapeutically effective amount of a compound that modulates the activity of a tissue protective NEPOR receptor complex to a human in need thereof, wherein said decreased survival of cancer cells/tissues results in the decrease of tumour growth and/or an increase in patient survival, with the proviso that the compound is an EPO derivative and not a wild-type EPO.
63. The method of 58, 59, 60, 61, 62 wherein the compound is identified by the method of any one of claims 16, 17, 21, 26, or 41 to 46.
64. A method for modulating cell survival in NEPOR positive tissue comprising administering an EPO mutants and peptides selected from the group consisting of peptides from each of SEQ ID NO. 17 through SEQ ID NO. 212.
65. A method for modulating cell survival in NEPOR positive tissue comprising administering an effective amount of an EPO chimera's, comprising an ephrin receptor ligand binding domain selected from the group consisting of SEQ ID NO.215, and SEQ ID NO.216.
66. The method of 58, 59, 60, 61, 62 wherein the compound is an antibody specific for the tissue protective NEPOR receptor complex.
67. The method of 58, 59, 60, 61, 62 wherein the compound is an antibody is specific for a tissue protective NEPOR receptor complex ligand.
68. The method of 58, 59, 60, 61, 62 wherein the compound is a small molecule, peptide, or a member of a library.
69. The method of 58, 59, 60, 61, 62 wherein the compound binds to the tissue protective NEPOR receptor complex.
70. The method of 58, 59, 60, 61, 62 wherein the compound decreases the activity of the tissue protective NEPOR receptor complex.
71. The method of 58, 59, 60, 61, 62 wherein the compound is administered in conjunction with an EPO.
72. The method of 58, 59, 60, 61, 62 wherein the disease or disorder is a cancer including, head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer.
73. The method of 58, 59, 60, 61, 62 wherein the cancer comprises cancer cells expressing the tissue protective NEPOR receptor complex.
74. The method of 58, 59, 60, 61, 62, wherein the cancer is metastatic cancer.
75. The method of 58, 59, 60, 61, 62 wherein the cancer is an angiogenesis-dependent cancer.
76. A method for treating a patient suffering from an angiogenesis-associated disease, comprising administering to the patient a compound identified by the method of any one of 16, 17, 21, 26, or 41 to 46.
77. siRNA which is specific for EPH-B4 for use in treating a cancer and/or tumor patient that is receiving or will receive Erythropoietin.

## Claims

1. A method of determining whether a cancer patient is suitable for erythropoietin (EPO) therapy, comprising
(A) determining the level of expression of EPH-B4 in a tissue sample isolated from said patient; and
(B) correlating a presence of EPH-B4 expression to a negative physiological response to EPO therapy.

2. The method of claim 1, wherein the level of expression is determined by a technique selected from the group consisting of immunoassay, chemiluminescent assay, nephelometric assay, turbidimetric assay, bioassay and reporter-assay.

3. The method of claim 1, wherein the level of expression is determined by an immunoassay selected from the group consisting of enzyme-linked immunosorbent assay (ELISA), immunoprecipitation, enzyme immunoassay (EIA), radioimmunoassay (RIA), fluorescent immunoassay, Western blot, competitive immunoassay, noncompetitive immunoassay, homogeneous immunoassay and heterogeneous immunoassay.

4. The method of claim 1, wherein the level of expression is determined by ELISA.

5. The method of claim 1, wherein said level of expression is determined by immunohistochemistry.

6. The method of claim 1, wherein the level of expression is determined by measuring the amount of EPH-B4 mRNA in said sample, in particular wherein the level of expression is determined by a technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF), and RT-PCR.

7. The method of claim 6, wherein the level of expression is determined by reverse transcription and amplification.

8. The method of claim 1, wherein the tissue sample is selected from cancerous tissue or circulating cells derived from same, or wherein the tissue sample is selected from blood, lymph, urine or cerebral fluid.

9. The method of claim 1, further comprising determining the level of expression in said sample of at least one of Ephrin A1 or EPOR.

10. The method of claim 1, wherein said negative physiological response includes increased tumor progression, poorer patient survival or enhanced tumor cell survival.

## Patentansprüche

1. Verfahren zum Bestimmen, ob ein Krebspatient für Erythropoietin (EPO)-Therapie geeignet ist, umfassend:
(A) Bestimmen des Expressionslevels von EPH-B4 in einer Gewebeprobe, isoliert von dem Patienten; und
(B) Korrelieren eines Vorhandenseins von EPH-B4-Expression mit einer negativen physiologischen Reaktion auf EPO-Therapie.

2. Verfahren nach Anspruch 1, wobei der Expressionslevel mittels einer Technik bestimmt wird, die ausgewählt ist aus der Gruppe, bestehend aus Immunoassay, Chemilumineszenz-Assay, nephelometrischem Assay, turbidimetrischem Assay, Bioassay und Reporter-Assay.

3. Verfahren nach Anspruch 1, wobei der Expressionslevel mittels eines Immunoassays bestimmt wird, der ausgewählt ist aus der Gruppe, bestehend aus enzymgekoppelter Immunadsorbtionsbestimmung (ELISA), Immunopräzipitation, Enzym-Immunoassay (EIA), Radioimmunoassay (RIA), Fluoreszenz-Immunoassay, Western-Blot, kompetitivem Immunoassay, nicht-kompetitivem Immunoassay, homogenem Immunoassay und heterogenem Immunoassay.

4. Verfahren nach Anspruch 1, wobei der Expressionslevel mittels ELISA bestimmt wird.

5. Verfahren nach Anspruch 1, wobei der Expressionslevel mittels Immunohistochemie bestimmt wird.

6. Verfahren nach Anspruch 1, wobei der Expressionslevel mittels Messen der Menge an EPH-B4-mRNA in der Probe bestimmt wird, insbesondere wobei der Expressionslevel mittels einer Technik bestimmt wird, die ausgewählt wird aus der Gruppe, bestehend aus PCR, QPCR, R-PCR, Microarray-Genexpressionsanalyse, Northern-Blot-Analyse, reverser Transkription und Amplifikation, Zymographie, Ligase-Kettenreaktion, NASBA, RNase-Schutz-Assay (RPA), Kapillarelektrophorese mit Laser-induzierter Fluoreszenz (CE-LIF) und RT-PCR.

7. Verfahren nach Anspruch 6, wobei der Expressionslevel mittels reverser Transkription und Amplifikation bestimmt wird.

8. Verfahren nach Anspruch 1, wobei die Gewebeprobe ausgewählt ist aus Krebsgewebe oder davon abgeleiteten zirkulierenden Zellen, oder wobei die Gewebeprobe ausgewählt ist aus Blut, Lymphe, Urin oder Zerebralflüssigkeit.

9. Verfahren nach Anspruch 1, weiterhin umfassend Bestimmen des Expressionslevels in der Probe von mindestens einem von Ephrin A1 oder EPOR.

10. Verfahren nach Anspruch 1, wobei die negative physiologische Reaktion erhöhte Tumorprogression, schlechteres Patientenüberleben oder erhöhtes Tumorzellüberleben beinhaltet.

## Revendications

1. Procédé de détermination si un patient cancéreux est approprié pour un traitement à l'érythropoïétine (EPO), comprenant
(A) la détermination du taux d'expression de EPH-B4 dans un échantillon tissulaire isolé dudit patient ; et
(B) la corrélation de la présence d'expression de EPH-B4 à une réponse physiologique négative au traitement à l'EPO.

2. Procédé selon la revendication 1, dans lequel le taux d'expression est déterminé par une technique choisie dans le groupe constitué d'un test immunologique, un test de chimiluminescence, un test néphélométrique, un test turbidimétrique, un test biologique et un test utilisant un rapporteur.

3. Procédé selon la revendication 1, dans lequel le taux d'expression est déterminé par un test immunologique choisi dans le groupe constitué d'un test ELISA (enzyme-linked immunosorbent assay), une immunoprécipitation, un test immunoenzymatique (EIA), un test radioimmunologique (RIA), un test d'immunofluorescence, une analyse Western blot, un test immunologique compétitif, un test immunologique non compétitif, un test immunologique homogène et un test immunologique hétérogène.

4. Procédé selon la revendication 1, dans lequel le taux d'expression est déterminé par un test ELISA.

5. Procédé selon la revendication 1, dans lequel le taux d'expression est déterminé par immunohistochimie.

6. Procédé selon la revendication 1, dans lequel le taux d'expression est déterminé en mesurant la quantité d'ARNm de EPH-B4 dans ledit échantillon, en particulier où le taux d'expression est déterminé par une technique choisie dans le groupe constitué de la PCR, la QPCR, la R-PCR, l'analyse par micropuce de l'expression génique, l'analyse Northern blot, la transcription inverse et l'amplification, la zymographie, la réaction en chaîne de la ligase, la technique NASBA, le test de protection à la ribonucléase (RPA), l'électrophorèse capillaire avec fluorescence induite par laser (CE-LIF), et la RT-PCR.

7. Procédé selon la revendication 6, dans lequel le taux d'expression est déterminé par transcription inverse et amplification.

8. Procédé selon la revendication 1, dans lequel l'échantillon tissulaire est choisi parmi du tissu cancéreux ou des cellules circulantes dérivées de ce dernier, ou dans lequel l'échantillon tissulaire est choisi parmi le sang, la lymphe, l'urine ou le liquide céphalo-rachidien.

9. Procédé selon la revendication 1, comprenant en outre la détermination du taux d'expression dans ledit échantillon d'au moins l'un de l'éphrine A1 ou l'EPOR.

10. Procédé selon la revendication 1, dans lequel ladite réponse physiologique négative comprend une augmentation de la progression de la tumeur, une survie plus faible du patient ou une amélioration de la survie des cellules tumorales.
